(19) Europäisches Patentamt
European Patent Office
Office européen des brevets

(11) EP 2 470 547 B1

(12) # FASCICULE DE BREVET EUROPEEN

(45) Date de publication et mention de la délivrance du brevet:
**14.05.2014 Bulletin 2014/20**

(21) Numéro de dépôt: **10761055.2**

(22) Date de dépôt: **12.08.2010**

(51) Int Cl.:
***C07D 519/00*** $^{(2006.01)}$ ***A61K 31/551*** $^{(2006.01)}$
***A61P 35/00*** $^{(2006.01)}$

(86) Numéro de dépôt international:
**PCT/FR2010/051709**

(87) Numéro de publication internationale:
**WO 2011/023883 (03.03.2011 Gazette 2011/09)**

(54) **CONJUGUES DE DIMERES DE PYRROLO[1,4]BENZODIAZEPINE EN TANT QU'ANTICANCEREUX**

PYRROLO[1,4]BENZODIAZEPINDIMERKONJUGATE ALS ANTIKREBSMITTEL

CONJUGATES OF PYRROLO[1,4]BENZODIAZEPINE DIMERS AS ANTICANCER AGENTS

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **25.08.2009 FR 0904043**
**11.09.2009 FR 0904368**

(43) Date de publication de la demande:
**04.07.2012 Bulletin 2012/27**

(73) Titulaire: **SANOFI**
**75008 Paris (FR)**

(72) Inventeurs:
- **COMMERÇON, Alain**
**F-75013 Paris (FR)**
- **GAUZY-LAZO, Laurence**
**F-75013 Paris (FR)**

(74) Mandataire: **Gaslonde, Aude**
**Sanofi**
**Département Brevets**
**54, rue La Boétie**
**75008 Paris (FR)**

(56) Documents cités:
**WO-A1-2007/085930 WO-A2-2009/016516**

EP 2 470 547 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention se rapporte à des conjugués de dimères de pyrrolo[1,4]benzodiazépine (PBD), les compositions les contenant et leur application thérapeutique, notamment comme anticancéreux. L'invention se rapporte aussi au procédé de préparation des conjugués à leur application en tant qu'anticancéreux ainsi qu'aux dimères eux-mêmes.

## [Domaine technique]

Les dimères de pyrrolo[1,4]benzodiazépines sont des anticancéreux qui agissent en se liant de façon covalente à l'ADN des cellules. Ces dérivés ont été décrits dans les demandes WO 00/12508 **et** WO 2005/085260 ainsi que dans les publications suivantes : Eur.J.Med.Chem. 2005, 40, 641-654 ; Tetrahedron Letters 1988, 29(40), 5105-51108.

La chimie des conjugués est connue depuis de nombreuses années et a été appliquée à plusieurs familles de cytotoxiques comme par exemple les maytansinoïdes (WO 04103272), les taxanes (WO 06061258), les leptomycines (WO 07144709), le **CC-1065** et ses analogues (WO 2007102069); voir aussi à propos des conjugués, Monneret C., et al., Bulletin du Cancer 2000, 87(11), 829-38 ; Ricart A.D., et al., Nature Clinical Practice Oncology 2007, 4, 245-255 ; Singh R. et Rickson H.K., Therapeutic Antibodies : Methods and Protocols, 2009, 525, 445-467.

## [Art antérieur]

Des conjugués de dimères de pyrrolo[1,4]benzodiazépine ont déjà été décrits dans les demandes WO 07085930 ou WO 2009/016516**.** Les dimères utilisés ont pour plus particulièrement pour formules :

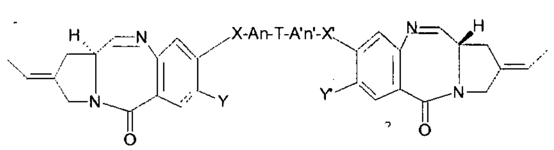
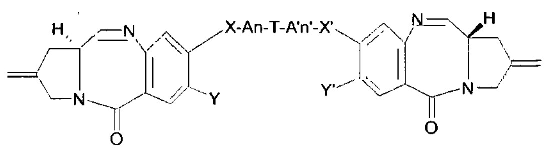

dans lesquelles T peut représenter un groupe aryle ou hétéroaryle substitué par $-G-D-(Z)_p-SZ_a$ ou $-G-D-(Z)_p-C(=O)Z_bR_b$. G représente une liaison simple ou double ou bien -O-, -S- ou -NR-. D représente une liaison simple ou bien l'un des groupes suivants : -E-, -E-NR-, -E-NR-F-, -E-O-, - E-O-F-, -E-NR-CO-, -E-NR-CO-F-, -E-CO-, -CO-E-, -E-CO-F, -E-S-, -E-S-F-, -E-NR-CS-, -E-NR-CS-F- pour lesquels E et F sont choisis parmi $-(OCH_2CH_2)_i$alkyl$(OCH_2CH_2)_j$-, -alkyl$(OCH_2CH_2)_i$-alkyl-, $-(OCH_2CH_2)_i$-, $-(OCH_2CH_2)_i$cycloalkyl$(OCH_2CH_2)_j$-, $-(OCH_2CH_2)_i$hétérocyclyl$(OCH_2CH_2)_j$-,$-(OCH_2CH_2)_i$aryl$(OCH_2CH_2)_j$-, $-(OCH_2CH_2)_i$hétéroaryl$(OCH_2CH_2)_j$-, -alkyl-$(OCH_2CH_2)_i$alkyl$(OCH_2CH_2)_j$-, -alkyl-$(OCH_2CH_2)_i$-, -alkyl-$(OCH_2CH_2)_i$cycloalkyl$(OCH_2CH_2)_j$,-alkyl$(OCH_2CH_2)_i$hétérocyclyl$(OCH_2CH_2)_j$-, -alkyl-$(OCH_2CH_2)_i$aryl$(OCH_2CH_2)_j$-,-alkyl$(OCH_2CH_2)_i$hétéroaryl$(OCH_2CH_2)_j$-,- cycloalkyl-alkyl-, -alkyl-cycloalkyl-, -hétérocyclyl-alkyl-,-alkyl-hétérocyclyl-, -alkyl-aryl-, -aryl-alkyl-, -alkyl-hétéroaryl- , -hétéroaryl-alkyl-. I et j représentent des nombres entiers allant de 0 à 2000. Z représente un groupe alkyle et p est un nombre entier valant 0 ou 1.

Le groupe $L_2$= $-CH_2C(=O)NR_3-(CH_2CH_2O)_i$-ALK- qui caractérise certains des composés de la présente invention comprend le motif amide ($-CONR_3-$) et ne peut correspondre dans WO 07085930 ou dans WO 2009/016516 qu'au motif -E-CONR-F- avec E= alkyl et F= $-(CH_2CH_2O)_i$-alkyl-. Cependant, le groupe $L_1$ qui est rattaché au cycle phényle ou pyridinyle et qui est rattaché à $L_2$ n'est pas décrit ni suggéré par ces deux demandes. En effet, il ne pourrait correspondre qu'au motif G. Or, G ne peut être qu'une liaison (simple, double, triple) ou bien -O-, -S- ou -NR-. S'agissant des autres composés de la présente invention qui se caractérisent par le linker $-O-ALK-NR_3-ALK-S-(CH_2CH_2O)_i$-ALK-, aucun des motifs D de WO 07085930 ou de WO 2009/016516 ne prévoit la combinaison d'un groupe amine $NR_3$ et d'une liaison -S-. Les dimères suivants sont décrits dans WO 2009/016516 :

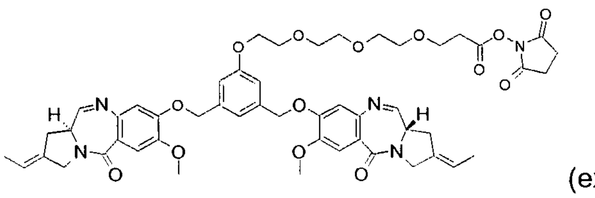
(ex.3)

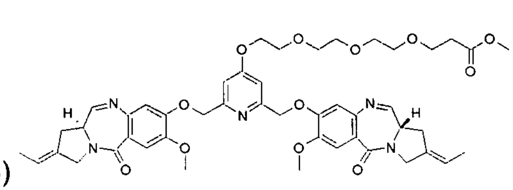
(ex.5)

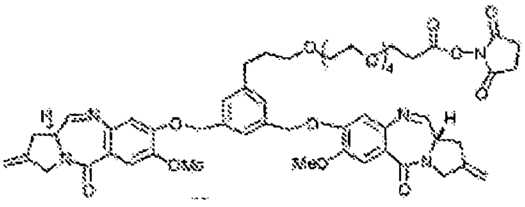 (ex.10) 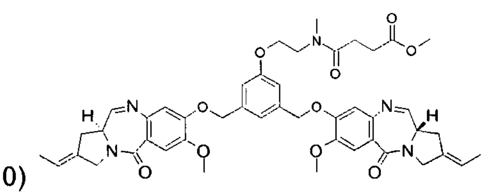 (ex.7)

mais aucun de ces dimères ne comprend un linker similaire à ceux décrits dans la présente invention (en particulier, pas de motif -ALK-S-).

Ainsi, les deux demandes WO 07085930 et WO 2009/016516 ne décrivent ni ne suggèrent les composés de la présente invention.

**[Problème technique]**

Le problème technique qu'entend résoudre la présente invention est de proposer de nouveaux conjugués de dimères de pyrrolo[1,4]benzodiazépine.

**[Définitions]**

On entend par :

- conjugué : un agent de ciblage cellulaire auquel est attaché de façon covalente au moins une molécule d'un composé cytotoxique ;
- agent de ciblage cellulaire (ou « cell binding agent » en anglais) : une molécule ayant une affinité pour une cible biologique : il peut s'agir par exemple d'un ligand, d'une protéine, d'un anticorps, plus particulièrement monoclonal, d'un fragment de protéine ou d'anticorps, d'un peptide, d'un oligonucléotide, d'un oligosaccharide. L'agent de ciblage a pour fonction de diriger le composé biologiquement actif comme un cytotoxique vers la cible biologique ;
- cible biologique : un antigène (ou groupe d'antigènes) localisé préférentiellement à la surface des cellules cancéreuses ou cellules stromales associées à cette tumeur ; ces antigènes pouvant être par exemple, un récepteur de facteur de croissance, un produit d'oncogène ou de gène « suppresseur de tumeur » muté, une molécule liée à l'angiogénèse, une molécule d'adhésion ;
- groupe alkyle : un groupe hydrocarboné aliphatique saturé obtenu en enlevant un atome d'hydrogène d'un alcane. Le groupe alkyle peut être linéaire ou ramifié. A titre d'exemples, on peut citer les groupes méthyle, éthyle, propyle, isopropyle, butyle, iso-butyle, tertio-butyle, pentyle, 2,2-diméthylpropyle, hexyle ;
- groupe cycloalkyle : un groupe alkyle cyclique comprenant entre 3 et 8 atomes de carbone engagés dans la structure cyclique. A titre d'exemples, on peut citer les groupes cyclopropyle, cyclobutyle, cyclopentyle, cyclohexyle ;
- groupe aryle : un groupe aromatique ne contenant pas d'hétéroatome, mono- ou bicyclique. Il s'agit plus particulièrement des groupes phényle et naphtyle ;
- groupe hétéroaryle : un groupe aromatique comprenant au moins un hétéroatome (O, S, N) engagé dans le cycle et relié aux atomes de carbone formant le cycle, mono- ou bicylique. Il s'agit plus particulièrement des groupes pyridinyle, pyrrolyle, thienyle, furanyle, pyrimidinyle, triazolyle ;
- groupe hétérocycloalkyle : un groupe cycloalkyle comprenant au moins un hétéroatome (O, S, N) engagé dans le cycle et relié aux atomes de carbone formant le cycle ;
- groupe alcoxy : un groupe -O-alkyle, où le groupe alkyle est tel que défini ci-dessus ;
- groupe alcanoyloxy : un groupe -O-CO-alkyle, où le groupe alkyle est tel que défini ci-dessus ;
- groupe alkylène : un groupe divalent saturé de formule brute $-C_mH_{2m}-$, obtenu en enlevant deux atomes d'hydrogène d'un alcane. L'alcane peut être linéaire ou ramifié. A titre d'exemples, on peut citer les groupes méthylène ($-CH_2-$), éthylène ($-CH_2CH_2-$), propylène ($-CH_2CH_2CH_2-$), butylène ($-CH_2CH_2CH_2CH_2-$), isobutylène (

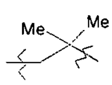

), hexylène ($-CH_2CH_2CH_2CH_2CH_2CH_2-$). Un groupe alkylène linéaire peut être plus particulièrement de formule $-(CH_2)_m-$, m représentant un nombre entier ;
- dans les plages de valeurs, les bornes sont incluses (par ex. une plage du type « i allant de 1 à 6 » inclut les bornes

1 et 6.

## Abréviations utilisées

AcOEt : acétate d'éthyle ; ALK : groupe ($C_1$-$C_{12}$)alkylène, plus particulièrement ($C_1$-$C_6$)alkylène ; CCM : chromatographie sur couche mince (TLC en Anglais) ; DAR : Drug Antibody Ratio ; DBU : 1,8-diazabicyclo[5.4.0]undec-7-ène ; DCC : N,N'-dicyclohexylcarbodiimide ; DCM : dichlorométhane ; DEAD : diéthylazodicarboxylate ; DIC : N,N'-diisopropylcarbodiimide ; DIPEA : N,N-diisopropyléthylamine ; DMA : diméthylacétamide ; DMAP : 4-diméthylaminopyridine ; DME : diméthoxyéthane ; DMF: diméthylformamide ; DMSO: diméthylsulfoxyde ; ε : coefficient d'extinction molaire; EEDQ : 2-éthoxy-1-éthoxycarbonyl-1,2-dihydroquinoline; EDCI : N-(3-diméthylaminopropyl)-N'-éthylcarbodiimide ; EDTA : acide éthylène-diamine-tétraacétique ; Fmoc : fluorénylméthoxycarbonyl ; GP : groupe protecteur ; Hal : atome d'halogène; HOBt : 1-hydroxybenzotriazole ; HEPES : acide 4-(2-hydroxyéthyl)-1-pipérazineéthanesulfonique ; LG : groupe partant ; NHS : N-hydroxysuccinimide ; NMP : N-méthylpyrrolidinone ; PR : pression réduite ; Rf : facteur de rétention ; SEC : chromatographie d'exclusion stérique ; TA : température ambiante ; TBDMS : *tert*-butyldiméthylsilyl ; TEA : triéthylamine ; TFA : acide trifluoroacétique ; TIPS : triisopropylsilyl ; THF : tétrahydrofurane ; $t_R$ : temps de rétention.

**[Figures]**

**Fig.1** : spectre de masse haute résolution du conjugué de l'ex.1 après déglycosylation ;
**Fig.2** : spectre de masse haute résolution du conjugué de l'ex.2 après déglycosylation ;
**Fig.3** : spectre de masse haute résolution du conjugué de l'ex.3 après déglycosylation ;
**Fig.4** : spectre de masse haute résolution du conjugué de l'ex.4 après déglycosylation ;
**Fig.5** : spectre de masse haute résolution du conjugué non déglycosylé de l'ex.6.

Ces figures montrent pour chaque conjugué après déconvolution, la distribution des espèces portant de 0 à 8 dimères de tomaymycine ($D_0$ : aucun dimère ; $D_x$ : x dimères).

**[Description de l'invention]**

L'invention est relative à des composés de formule :

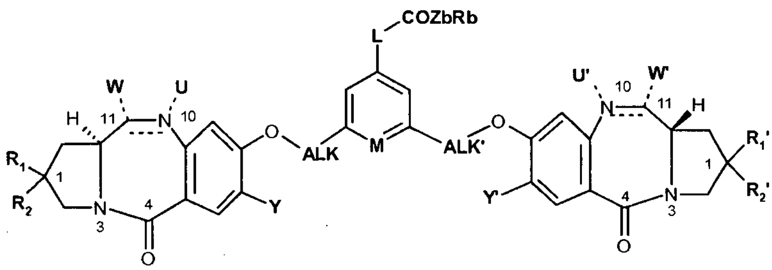

(I)

dans laquelle :

- ---- représente une liaison simple ou une liaison double avec la condition que si ---- représente une liaison simple alors :
  - ❖ ---- représente une liaison simple ;
  - ❖ **U** et/ou **U'** identique ou différent, représente(nt) indépendamment l'un de l'autre H ;
  - ❖ **W** et/ou **W'** identique ou différent, représente(nt) indépendamment l'un de l'autre : OH, -OR, -OCOR, -COOR, -OCOOR, -OCONRR', un carbamate cyclique tel que N10 et C11 soient inclus dans un cycle, -NRCONRR', -OCSNHR, un thiocarbamate cyclique tel que N10 et C11 soient inclus dans un cycle, -SH, -SR, -SOR, -SOOR, $-SO_3^-$, -NRSOOR', -NRR', une amine cyclique telle que N10 et C11 soient inclus dans un cycle, -NROR', -NRCOR', $-N_3$, -CN, Hal, un groupe trialkylphosphonium ou triarylphosphonium ;

- $R_1$, $R_2$, $R_1'$, $R_2'$ identiques ou différents, représentent, indépendamment l'un de l'autre : H, Hal ou un groupe

($C_1$-$C_6$)alkyle éventuellement substitué par un ou plusieurs substituant(s) choisi(s) parmi : Hal, CN, NRR', $CF_3$, OR, un groupe aryle ou hétéroaryle, $S(O)_qR$ avec q =0,1 ou 2 ;
ou bien

- **$R_1$** et **$R_2$** et/ou **$R_1$'** et **$R_2$'** forment ensemble respectivement une double liaison $=CH_2$ ou $=CH-CH_3$ ;
- **Y** et **Y',** identiques ou différents, représentent indépendamment l'un de l'autre H ou OR ;
- **M** représente CH ou N ;
- **ALK** et **ALK'** identiques ou différents, représentent indépendamment l'un de l'autre un groupe ($C_1$-$C_6$)alkylène ;
- **R** et **R'** représentent indépendamment l'un de l'autre, H ou un groupe ($C_1$-$C_6$)alkyle ou aryle éventuellement substitué par un ou plusieurs substituant(s) choisi(s) parmi : Hal, CN, NRR', $CF_3$, OR, un groupe aryle ou hétéroaryle ;
- L représente:

  ❖ le groupe $-L_1-L_2-$ dans lequel $L_1$ est rattaché au cycle aromatique comprenant **M** par le groupe ALK ou OALK et représente l'un des groupes suivants :

  - ALK-S-

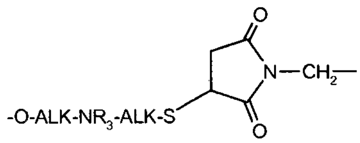

-O-ALK-$NR_3$-ALK-S-

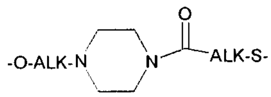

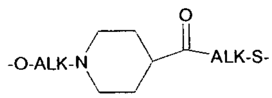

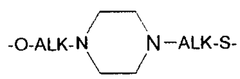

et **$L_2$** représente le groupe $-CH_2C(=O)-NR_3-(CH_2CH_2O)_i-ALK-$ rattaché à **$L_1$** par $-CH_2C(=O)-$
ou bien

  ❖ le groupe $-O-ALK-NR_3-ALK-S-(CH_2CH_2O)_i-ALK-$ rattaché au cycle aromatique comprenant **M** par le groupe OALK

- **$R_3$** représente H ou un groupe ($C_1$-$C_6$)alkyle ;
- **i** représente un nombre entier allant de 1 à 40, plutôt de 1 à 20, de préférence de 1 à 10 ;
- **$Z_b$** représente une liaison simple, -O- ou -NH- et **$R_b$** représentant H ou un groupe ($C_1$-$C_6$)alkyle, ($C_3$-$C_7$)cycloalkyle, aryle, hétéroaryle ou ($C_3$-$C_7$)hétérocycloalkyle ou bien **$Z_b$** représente une liaison simple et **$R_b$** représente Hal.

Les composés de formule (I), y compris ceux exemplifiés, peuvent exister à l'état de bases ou de sels d'addition à des acides pharmaceutiquement acceptables ou encore d'hydrates ou solvates de ces bases ou de ces sels.
Plus particulièrement, les deux groupes ALK et ALK' attachés au noyau phényle ou pyridinyle désignent tous deux un groupe méthylène :

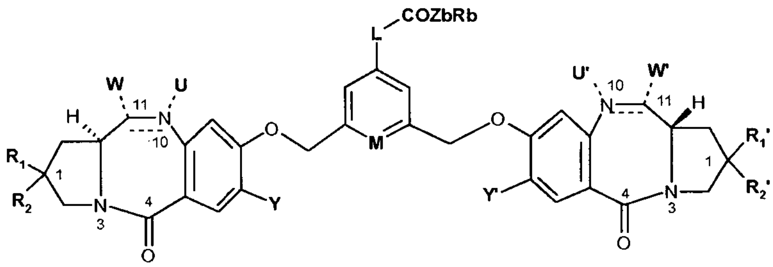

Plus particulièrement, parmi les composés de formule (I), on distingue ceux de formule **(IA)** ou **(IB) :**

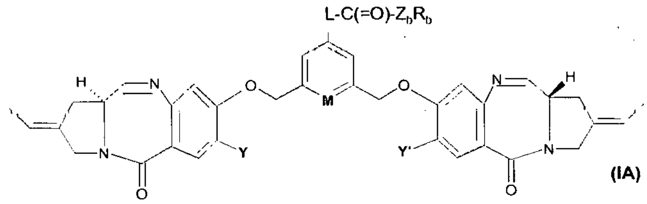

(IA)

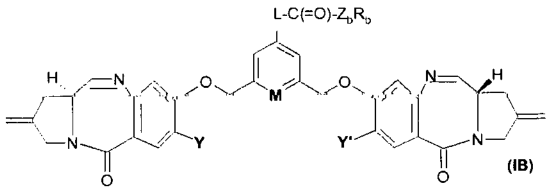

(IB)

**Y** et **Y'** représentent plus particulièrement un groupe ($C_1$-$C_4$)alcoxy, notamment le groupe méthoxy. **R** et **R'** peuvent représenter plus particulièrement indépendamment l'un de l'autre, H ou un groupe ($C_1$-$C_6$)alkyle. Selon un mode particulier, U=U' et/ou W=W' et/ou $R_1$=$R_1$' et/ou $R_2$=$R_2$' et/ou Y=Y' et/ou les deux groupes ALK et ALK' attachés au noyau phényle ou pyridinyle sont identiques.

**L** peut être plus particulièrement choisi parmi l'un des suivants :

- ALK-S-$CH_2C(=O)$-NH-$(CH_2CH_2O)_i$-$CH_2CH_2$-

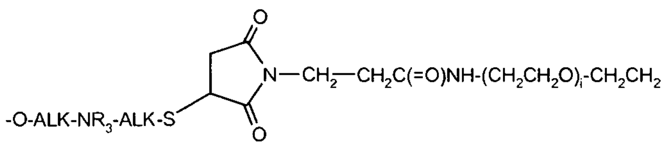

- O-ALK-$NR_3$-ALK-S-$CH_2C(=O)$-NH-$(CH_2CH_2O)_i$-$CH_2CH_2$

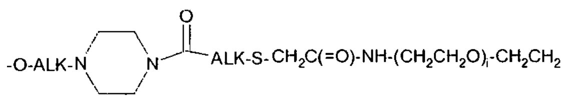

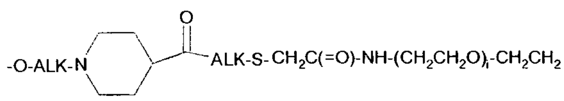

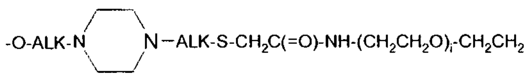

- O-ALK-$NR_3$-ALK-S-$(CH_2CH_2O)_i$-$CH_2CH_2$-

Parmi ceux-ci, ALK représente plus particulièrement un groupe ($C_1$-$C_4$)alkylène. En particulier, ALK peut être l'un des suivants : -$CH_2CH_2$-, -$CH_2CMe_2$-, -$CH_2CH_2CMe_2$-. **L** peut être aussi l'un de ceux décrits dans le **Tableau** I ci-dessous ou dans le **Tableau II.**

i représente un nombre entier allant de 1 à 40, plutôt de 1 à 20, de préférence de 1 à 10. i peut prendre chacune des valeurs allant de 1 à 40, en particulier i peut valoir 3, 4, 5, 6, 7, 8, 9 ou 10.

Le **Tableau I** décrit des exemples représentatifs de composés selon la formula **(IA).** Chaque composé de ce

tableau peut exister sous la forme avec **M=CH** (benzène) ou **M=N** (pyridine). Les composés avec **M=N** sont plus solubles dans l'eau.

**Tableau I**

| Composé de formule (IA) |
|---|
| 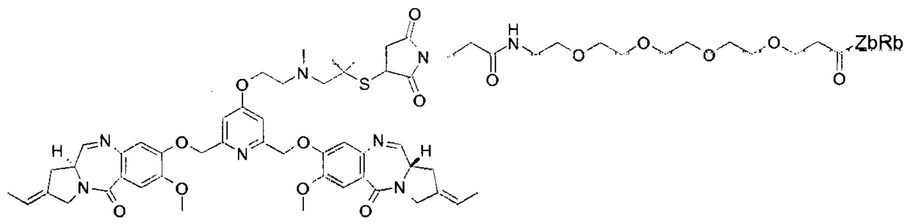 |
| 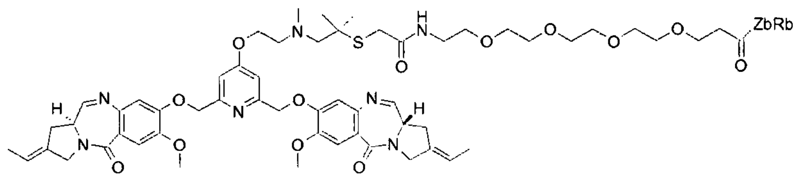 |
| 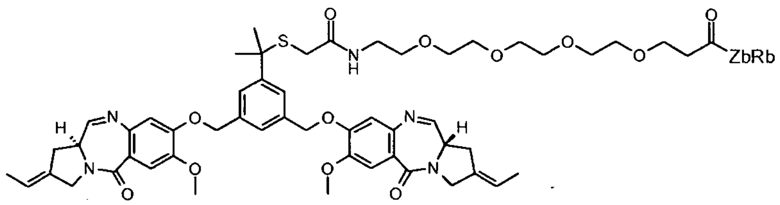 |
| 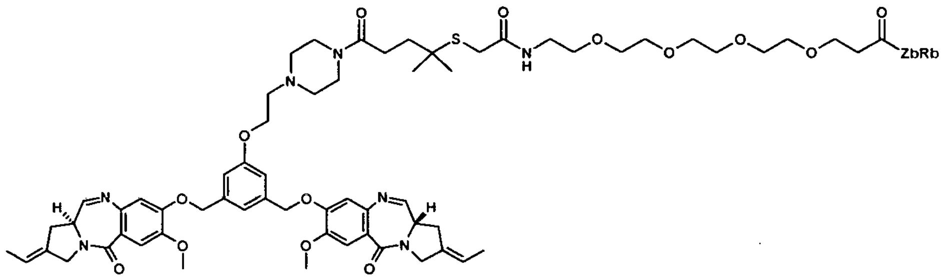 |
| 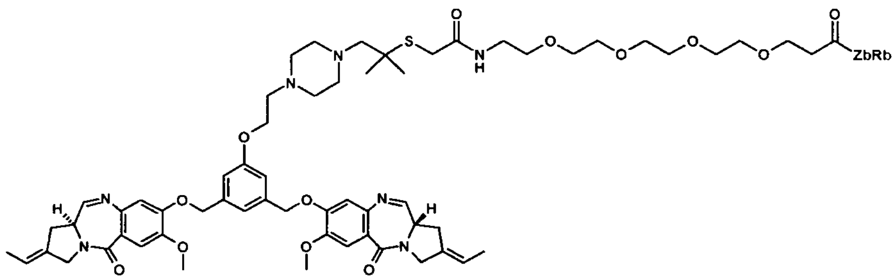 |
| 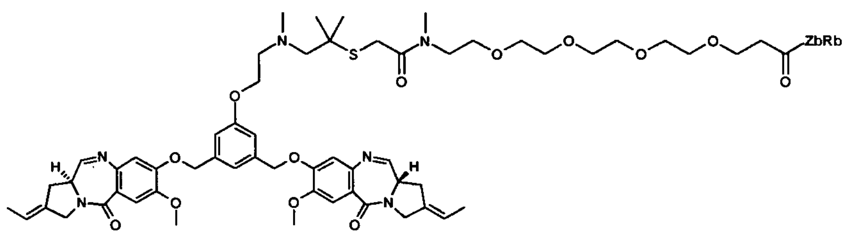 |

(suite)

| **Composé de formule (IA)** |
| --- |
| 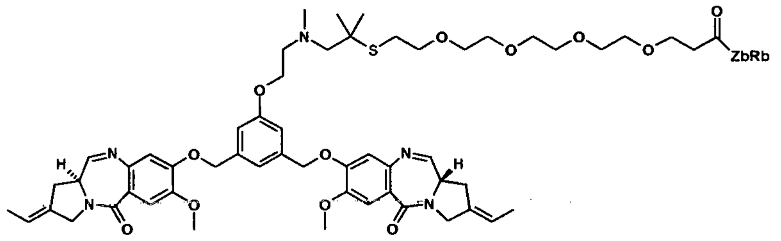 |

Les composés selon l'invention comprennent le groupe chimique $-C(=O)Z_bR_b$ (GCR1) qui est réactif vis-à-vis d'un groupe chimique réactif (GCR2) présent sur l'agent de ciblage. La réaction entre GCR1 et GCR2 assure l'attachement du composé sur l'agent de ciblage par formation d'une liaison covalente. Ainsi, le composé est apte à être conjugué à un agent de ciblage. Plus particulièrement, $Z_b$ représente O ; dans ce cas, GCR1 représente une fonction acide ($R_b$=H) ou ester. Plus particulièrement, $-C(=O)Z_bR_b$ représente -COOH, -COO($C_1$-$C_6$)alkyle, notamment-$COOCH_3$, ou -$COOCH_2CH=CH_2$. Parmi les fonctions esters, on préfère les esters dits « activés » qui présentent une bonne réactivité vis-à-vis des groupes GCR2, notamment vis-à-vis des groupes amino présents sur les anticorps. Des exemples d'esters activés sont les suivants :

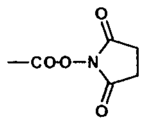 , 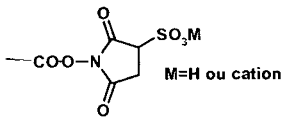 , 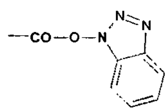

ou le groupe

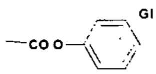

dans lequel **GI** représente au moins un groupe inductif tel que -$NO_2$ ou -Hal, notamment -F. Il peut s'agir par exemple des groupes suivants :

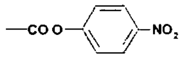 ou de 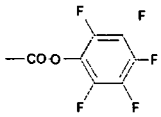 .

Un autre type de groupe-$C(=O)Z_bR_b$ est le suivant :

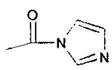

A titre d'exemple de GCR2, on peut citer les groupes ε-amino des lysines portés par les chaînes latérales des résidus lysine qui sont présents à la surface d'un anticorps, les groupes saccharides de la région charnière ou les thiols de cystéines par réduction de liaisons disulfures intra-chaîne (Garnett M.C., et al., Advanced Drug Delivery Reviews 2001, 53, 171-216). Plus récemment d'autres approches ont été considérées comme l'introduction de cystéines par mutation (Junutula J.R., et al., Nature Biotechnology 2008, 26, 925-932; WO 09026274) ou l'introduction d'acides aminés non-naturels permettant d'autres types de chimie (de Graaf A.J., et al., Bioconjugate Chem. 2009, February 3, 2009 (Review); **DOI:** 10.1021/bc800294a ; WO 2006/069246 et selon Chin J.W., et al., JACS 2002, 124, 9026-9027 (technologie ReCode®)). Ces modes d'attachement utilisés avec les anticorps sont applicables à l'ensemble des agents de ciblage connus en fonction de leur structure.

Les composés selon l'invention peuvent donc être utilisés pour la préparation d'un agent de ciblage auquel est

attaché de façon covalente en position para de **M** le dimère de formule :

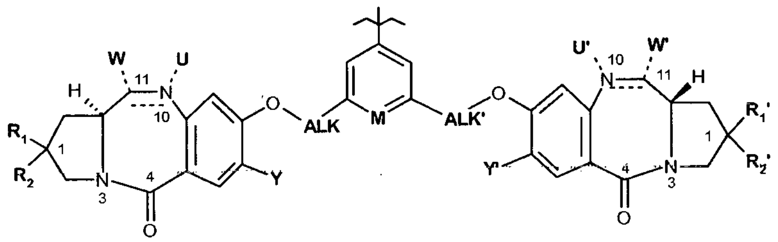

Plus particulièrement, l'agent de ciblage est un anticorps. Plus particulièrement, le dimère a pour formule :

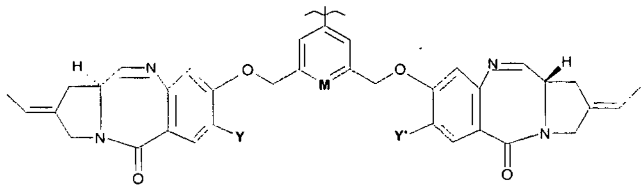

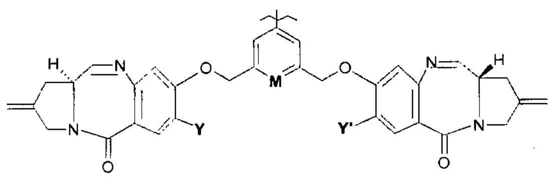

**Procédé de préparation des composés de formule (I)**

Les composés de formule (I) peuvent être préparés selon le **Schéma 1 :**

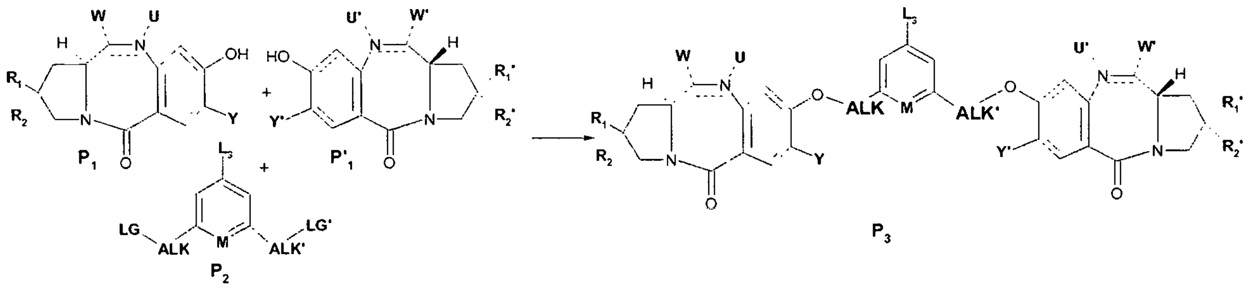

**Schéma 1**

On fait réagir ensemble les composés $\mathbf{P_1}$, $\mathbf{P'_1}$ et $\mathbf{P_2}$ pour conduire à $\mathbf{P_3}$. **LG** et **LG'** désignent un groupe partant. $L_3$ peut représenter le groupe -L-C(=O)$Z_bR_b$, dans ce cas, $P_3$ représente donc un composé de formule (I). Dans le cas où $\mathbf{P_3}$ ne représente pas le groupe -L-C(=O)$Z_bR_b$, il est nécessaire de transformer $L_3$ en groupe -L-C(=O)$Z_bR_b$ à l'aide d'une ou de plusieurs réactions.

En particulier, dans le cas où

–C(=O)$Z_bR_b$= 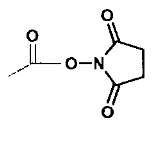

on peut introduire le groupe $L_3$ terminé par le groupe -C(=O)$Z_bR_b$= -C(=O)O-($C_1$-$C_4$)alkyle ou -C(=O)O-allyle, lequel est transformé ensuite en groupe -C(=O)OH lequel réagit finalement avec le carbonate de N-N'-disuccinimidyle ou le NHS. La transformation -COOalkyle/allyle en -COOH peut être réalisée par un traitement à la lithine. Il est particulièrement avantageux d'utiliser en particulier un ester méthylique. La réaction avec le carbonate de N,N'-disuccinimidyle est conduite en présence d'une base, par exemple la DIPEA ; celle avec le NHS en présence d'un agent de couplage, par exemple le DCC.

De même, dans le cas où

$-C(=O)Z_bR_b=$ 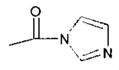 ,

on peut introduire un groupe $-C(=O)Z_bR_b=-COOH$ qui réagit ensuite avec le N,N'-carbonyldiimidazole (JACS 1958, 80, 4423 ; JACS 1960, 82, 4596).

Les composés **$P_1$** et **$P'_1$** sont décrits dans les demandes de brevets WO 00/12508, WO 00/12507, WO 2005/040170, WO 2005/085260, WO 07085930 ou WO 2009/016516 ou accessibles par synthèse totale (Mori M. et al., Tetrahedron, 1986, 42, 3793-3806). Dans le cas où **$P_1$** et/ou **$P'_1$** représente(nt) la tomaymycine de formule :

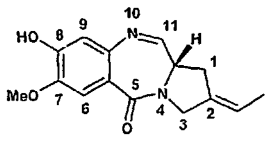

**Tomaymycin**

,

cette dernière peut être préparée à l'aide de la souche *Streptomyces croceus* en suivant l'enseignement de FR 1516743 ou bien par synthèse totale (voir J. Antibiotics 1983, XXXVI(3), 276-282 Z.Tozuka "Studies on tomaymycin. Total syntheses of the antitumor antibiotics E- and Z- tomaymycins"). Il existe également des composes **$P_1$/$P'_1$** commerciaux. Pour l'introduction des groupes W/W', la fonction imine (---- = liaison double) est susceptible d'additionner divers composés HW/HW' (par ex. $H_2O$, alcool ROH).

à propos des composés de formule $P_2$

Ceux-ci ont pour formule :

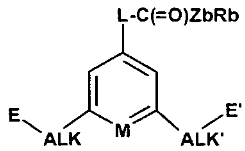 **$P_2$**

dans laquelle :

- L, M, ALK, ALK', $Z_b$ et $R_b$ sont tels que définis précédemment ;
- E et E' représentent indépendamment l'un de l'autre un groupe -OH ou un groupe partant.

L peut représenter plus particulièrement l'un de ceux décrits sur les Schémas 2, 2', 3, 3', 3", 4, 5, 5', 6, 6', 6", 7.

On distingue aussi les intermédiaires de formule :

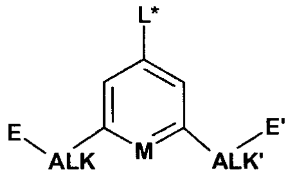

dans laquelle L* est choisi parmi : -ALK-SH ; $-O-ALK-NR_3-ALK-SH$ ;

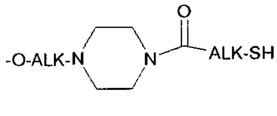

;

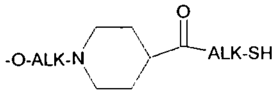 ; 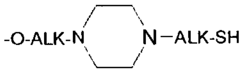

Dans cette invention, on désigne par « groupe partant » un atome ou un groupe d'atomes qui, dans la réaction hétérolytique entre $\mathbf{P_2}$ et $\mathbf{P_1}$ ou $\mathbf{P'_1}$, part en emportant le doublet d'électrons de la liaison covalente reliant ALK et LG ou LG'. Le groupe partant est choisi plus particulièrement parmi un atome d'halogène, notamment chlore ou brome, un groupe mésylate, tosylate, nosylate ou $-OPPh_3^+$.

Pour les autres Schémas ci-dessous, on utilise pour simplifier les abréviations suivantes :

Tom1 = 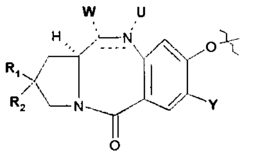 Tom'1 = 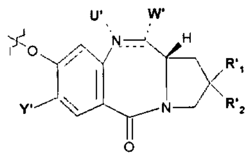

| $\mathbf{L_1}$= -ALK-S- et $\mathbf{L_2}$=-$CH_2C(=O)-NH-(CH_2CH_2O)_i$-ALK- |
|---|

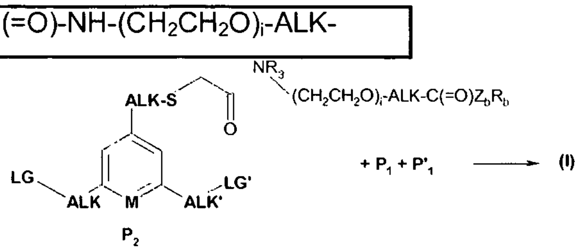


**Schéma 2**

<u>**Préparation de $\mathbf{P_2}$**</u> $\mathbf{P_2}$ est obtenu à partir du diol halogéné correspondant de formule

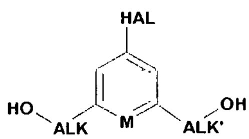

selon l'enseignement de l'ex.3 (Schéma 2') :

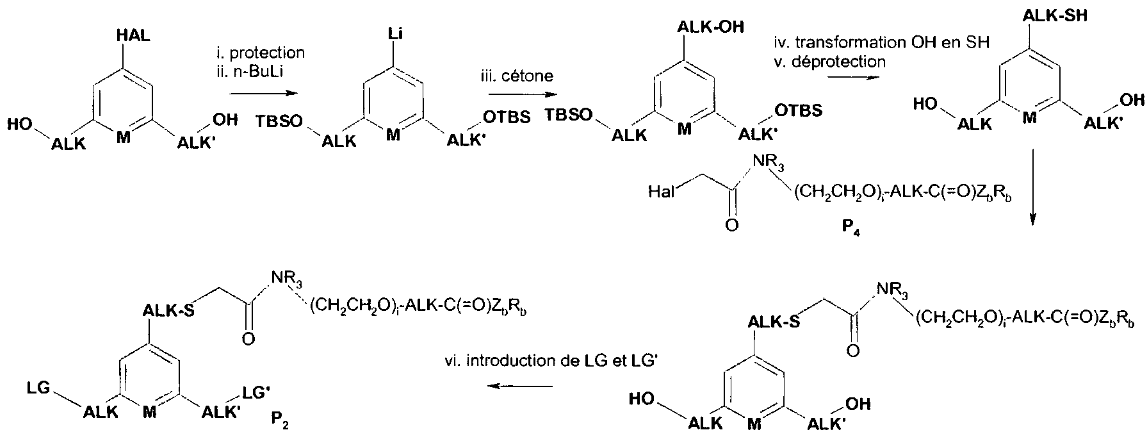


**Schéma 2'**

i. protection des deux fonctions alcool à l'aide d'un groupement protecteur tel que par ex. le tert-butyl-diméthyl-silyloxy (TBS) ;

ii. préparation de l'organolithien ou organomagnésien correspondant à l'aide respectivement de n-BuLi ou de

magnésium ;
iii. addition nucléophile sur une cétone pour former une fonction alcool ;
iv. préparation du thiol via la formation du thioacétate correspondant (voir ex.3.7 et 3.8) ;
v. déprotection ;
vi. introduction de **LG** et **LG'.** Dans le cas d'un groupe mésylate, on utilise le chlorure de méthanesulfonyle en présence d'une base telle qu'une amine tertiaire (par ex. TEA ou DIPEA) ; voir ex.1.4.

Un exemple de diol halogéné et du diol protégé correspondant est celui décrit sur le schéma 1 en page 48 de WO 2009/016516 (composés 2 et 3 du schéma 1). Deux exemples de diols protégés sont ceux de N° CAS 181225-40-1 et 181225-41-2.

Le diol halogéné peut être obtenu par réduction du composé diacide ou diester correspondant, par exemple celui de N° CAS 193010-40-1. Voir également dans le cas d'une pyridine (M=N) : Liebigs Annalen der Chemie 1991, 10, 987-988 ou Tetrahedron 2005, 61(7), 1755-1763 (composé 3 du Schéma 1).

**Préparation de $P_4$**

cas où $ALK=CH_2CH_2$

cas où $R_3=H$

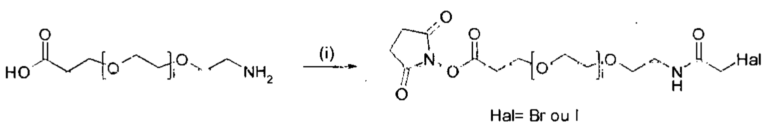


Etape (i) : formation de l'amide et activation de l'acide ; les deux étapes sont réalisées successivement dans un solvant polaire aprotique comme le DCM : réaction entre la fonction amine et l'halogénoacétate de N-hydroxysuccinimidyle puis addition *in situ* d'un agent de couplage comme le DIC.

cas où $R_3 \neq H$

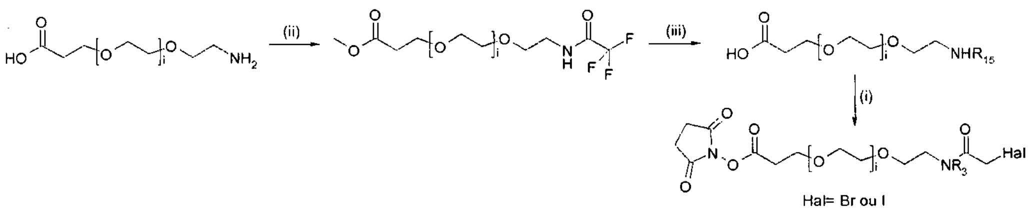


Etape (ii) : protection de l'acide carboxylique sous forme d'ester méthylique et de l'amine sous forme de trifluoroacétamide ; la réaction est réalisée en deux étapes successives dans un solvant polaire aprotique comme le DCM : protection de l'acide par traitement avec le triméthylsilyldiazométhane en présence de méthanol puis protection de l'amine par addition d'anhydride trifluoroacétique et d'une base comme la TEA ;

Etape (iii) : alkylation de l'amine et saponification de l'ester ; la réaction est réalisée en deux étapes successives dans un solvant polaire aprotique anhydre comme le THF : alkylation de l'amine par traitement avec une base comme NaH en présence d'un réactif porteur d'un groupement nucléofuge comme un halogénure d'alkyle $R_3Hal$ puis addition de lithine et d'eau ;

Etape (i) : suite à l'étape (iii), on reprend les réactions de l'étape (i) pour le cas $R_3=H$.

cas où $ALK \neq CH_2CH_2$

cas où $R_3=H$

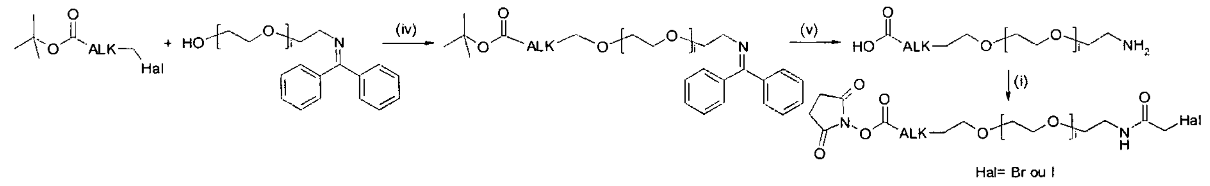


cas où $R_3 \neq H$

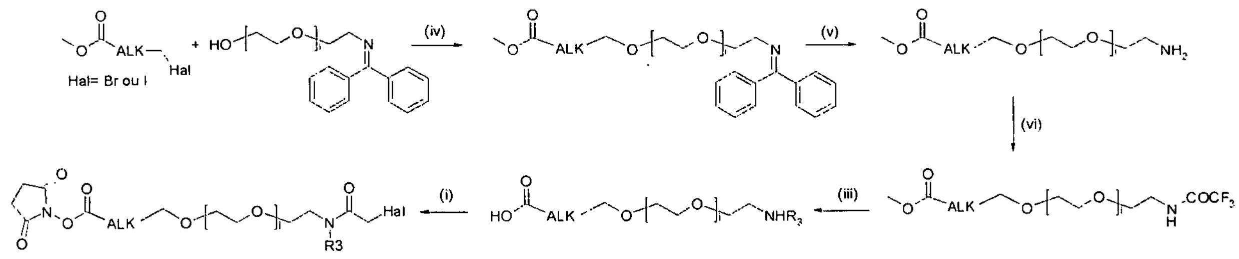


Etape (iv) : élongation de la chaîne PEG ; la réaction est réalisée dans un solvant polaire aprotique anhydre comme le THF ou le DMF par traitement d'un ester halogéné avec l'alcoolate d'un benzophénone-imine-PEG-alcool généré par l'action de NaH ou du naphtalénure de potassium comme décrit dans WO 2007/127440 ;

Etape (v) : clivage sélectif de l'imine par hydrogénation en présence de palladium sur charbon selon Wessjohann, L. et al., Synthesis 1989, 5, 359-63 ;

Etape (vi) : protection de l'amine par addition d'anhydride trifluoroacétique et d'une base comme la TEA.

Les amino-PEG-alcools sont commercialement disponibles pour par exemple i=3,4,7,8 ou peuvent être préparés à partir des diols PEG, commercialement disponibles pour i=3 à 12, selon la procédure décrite dans US 7230101. La protection de la fonction amine par la benzophénone peut être réalisée par deshydratation azéotropique en présence d'un acide de Lewis comme l'éthérate de $BF_3$.

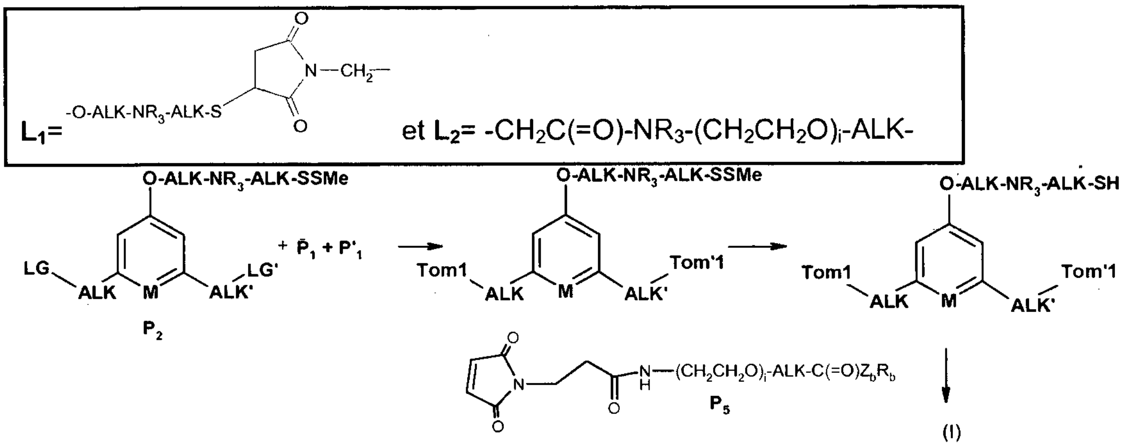


**Schéma 3**

**Préparation de** $P_2$

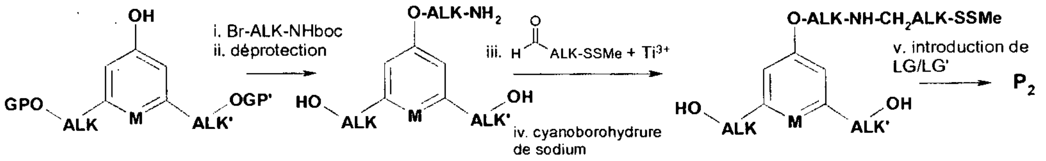


**Schéma 3'**

i. réaction nucléophile entre l'une des fonctions -OH (les deux autres étant protégées par GP et GP' qui désignent des groupes protecteurs) et une bromo-amine protégée par boc de formule Br-ALK-NHboc en présence d'une base telle que par ex. $K_2CO_3$ dans un solvant polaire tel que le DMF, le THF ou le MeCN (voir par ex. les conditions en page 63 de WO 07085930).

Selon une variante, on peut réaliser la substitution nucléophile de la bromo-amine par l'hydroxy-diester de formule :

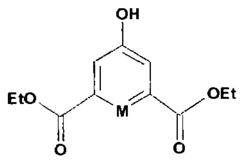

puis réduire par la suite la fonction ester en fonction-$CH_2OH$, par exemple avec le borohydrure de sodium ; on peut appliquer pour cela les conditions de la substitution nucléophile et de la réduction données en pages 62-63 de WO 2007/085930 ;

ii. déprotection de groupes protecteurs ;

iii. amination réductrice avec l'aldéhyde de formule HC(=O)-ALK-SSMe en présence d'isopropoxyde de titane ; la réaction est réalisée à température ambiante dans un solvant polaire aprotique anhydre comme le THF ;

iv. il se forme un complexe intermédiaire qui est réduit *in situ* avec un agent réducteur comme par exemple le cyanoborohydrure de sodium ;

v. introduction de LG et LG'. Dans le cas d'un groupe mésylate, on utilise le chlorure de méthanesulfonyle en présence d'une base telle qu'une amine tertiaire (par ex. TEA) ; voir ex.1.4.

Une variante représentée sur le **Schéma 3"** consiste à introduire le groupe -ALK-SSMe sur le groupe NHboc en s'inspirant en particulier de la méthode décrite par Kitagawa T. et al. JACS, 2006, 128(45), 14448-14449 utilisée pour introduire des chaines acétylthioalkyles sur une amine secondaire :

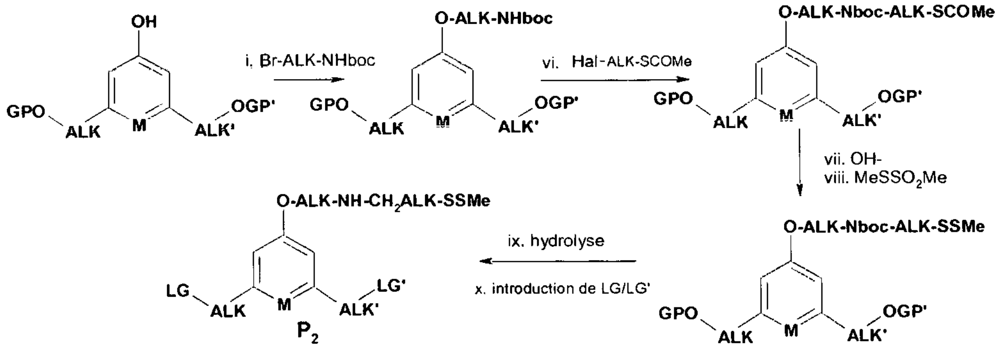


**Schéma 3"**

Selon cette variante, on réalise l'alkylation à l'aide d'un intermédiaire Hal-ALK-SCOMe (Hal=I par exemple) puis on libère le thiol par un traitement en milieu basique :

vi. alkylation par un halogénure d'alkyle porteur d'un groupe thioacétyle en présence de carbonate de césium dans un solvant aprotique polaire comme le DMF ;

vii. clivage sélectif du groupe acétyle en milieu basique faible ;

viii. formation du groupe -SSMe par réaction du thiol intermédiaire avec $MeSSO_2Me$ ;

ix. clivage des groupements protecteurs GP et GP' ;

x. transformation des groupes hydroxyles en groupes nucléofuges LG/LG', de préférence des groupes mésylates.

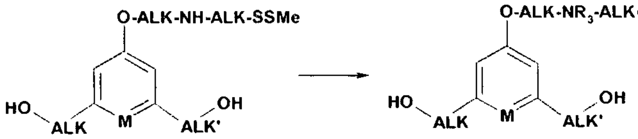

**Schéma 3'''**

L'introduction d'un groupe $R_3$=($C_1$-$C_4$)alkyle sur un groupe NH est possible à différents stades de la synthèse (un exemple est donné sur le **Schéma 3'''**). Elle est réalisée par exemple en employant la réaction de Wallach qui utilise un aldéhyde (voir l'ex.1.5 où l'alkylation NH→NMe utilise le formaldéhyde).

**<u>à propos de $P_5$</u>**

Les sociétés Thermo Fisher Scientific, Rockford, IL. 61105, USA, Jenkem Technology USA Inc., 2033, W.McDermott Dr., Allen Tx 75013-4675, USA et Quanta BioDesign, Ltd.195, West Olentangy Street, Suite O, Powell, Ohio 43065-8720, USA commercialisent des composés de formule générale

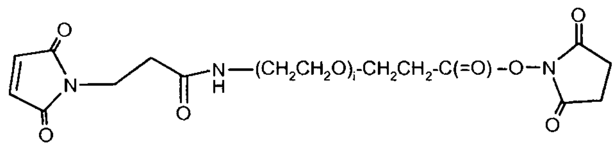

désignés par NHS-PEG-maleimide. Il peut s'agir plus particulièrement du composé de N° CAS 756525-99-2.

L'addition du thiol sur le motif maléimide est décrite en page 721 de « Bioconjugate Techniques », Greg T. Hermanson, 2ème Ed, Elsevier Inc. (isbn-13: 978-0-12-370501-3; isbn-10:0-12-370501-0).

**$L_1$= -O-ALK-$NR_3$-ALK-S- et $L_2$= -$CH_2C$(=O)-$NR_3$-($CH_2CH_2O$)$_i$-ALK-**

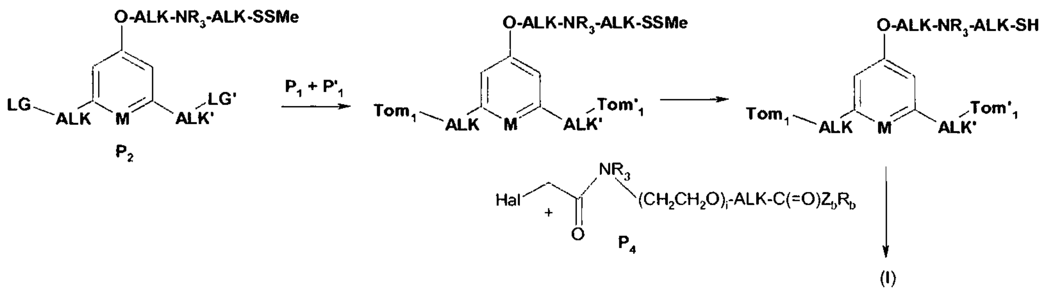


**Schéma 4**

La préparation est semblable à celle décrite sur le **Schéma 3** en remplaçant **$P_5$** par **$P_4$**.

Une variante décrite sur le **Schéma 4'** correspond à une préparation semblable à celle décrite sur les **Schéma 2 et 2' :**

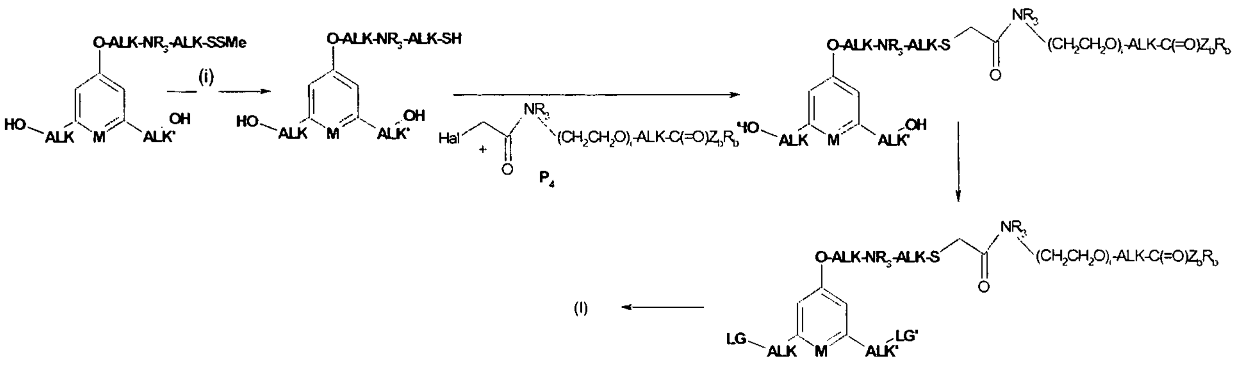


**Schéma 4'**

i. déprotection par réduction du disulfure

$L_1$= 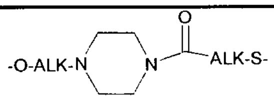 et $L_2$= $-CH_2C(=O)-NR_3-(CH_2CH_2O)_i-ALK-$

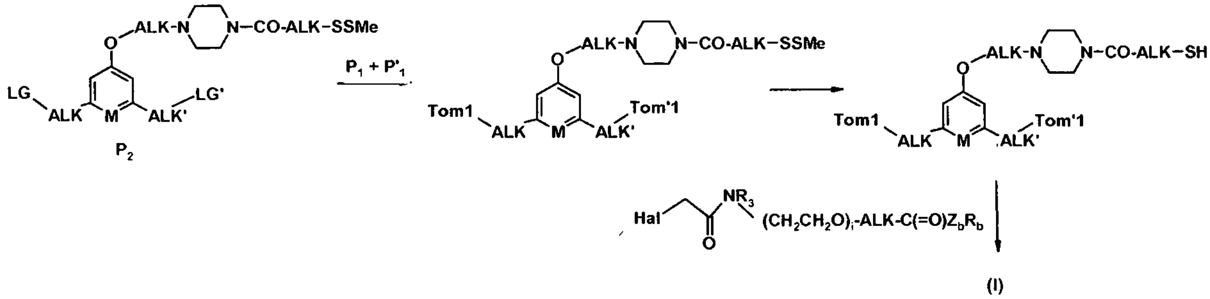


**Schéma 5**

<u>**Préparation** de $P_2$</u>

On utilise le composé $P_2$ de formule :

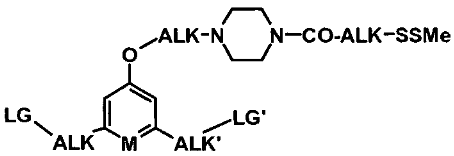

qui est obtenu selon le **Schéma 5**' ci-dessous :

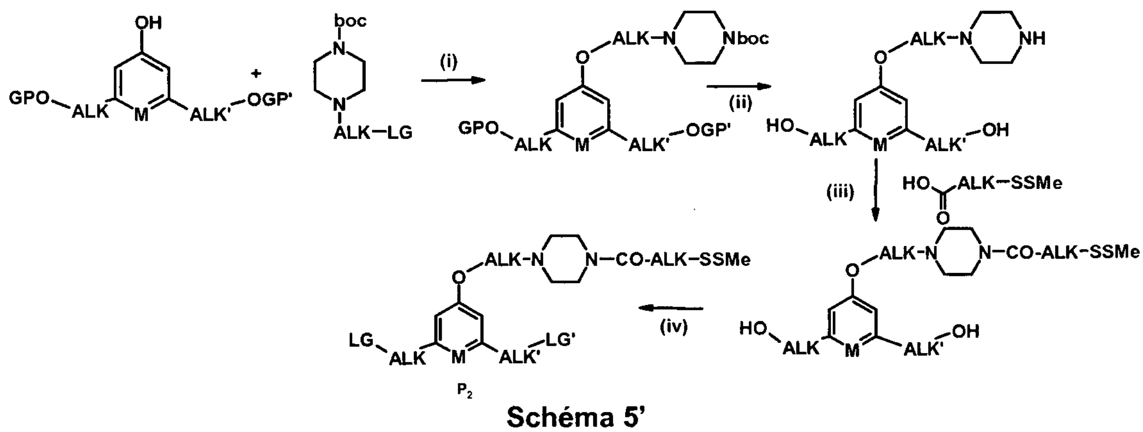


**Schéma 5'**

i. alkylation de l'hydroxyle du cycle aromatique par une pipérazine mono protégée en position 1 et porteuse en 4 d'une chaine alkyle fonctionnalisée en position terminale par un groupe LG nucléofuge. De préférence le groupe nucléofuge est un groupe mésylate et la réaction de Williamson s'opère en présence d'un hydrure dans un solvant polaire aprotique anhydre comme le THF ou le DMF ;

ii. déprotection des groupes boc, GP et GP', de préférence en milieu acide, par ex. en présence d'acide chlorhydrique ou de TFA quand les groupes GP et GP' sont le TBDMS.

Selon une variante des étapes i et ii, on peut réaliser la substitution nucléophile de la bromo-amine par l'hydroxy-diester de formule :

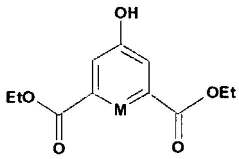

puis réduire par la suite la fonction ester en fonction $-CH_2OH$, par exemple avec le borohydrure de sodium ; on peut appliquer pour cela les conditions de la substitution nucléophile et de la réduction données en pages 62-63 de WO 2007/085930 ;

iii. couplage réalisé après activation initiale de l'acide en ester de NHS ;

iv. introduction des groupes partants **LG** et **LG'.** Dans le cas d'un groupe mésylate, on utilise le chlorure de méthanesulfonyle en présence d'une base telle qu'une amine tertiaire (par ex. TEA).

$L_1$= 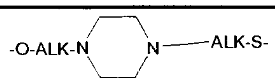 et $L_2$= $-CH_2C(=O)-NR_3-(CH_2CH_2O)_i-ALK-$

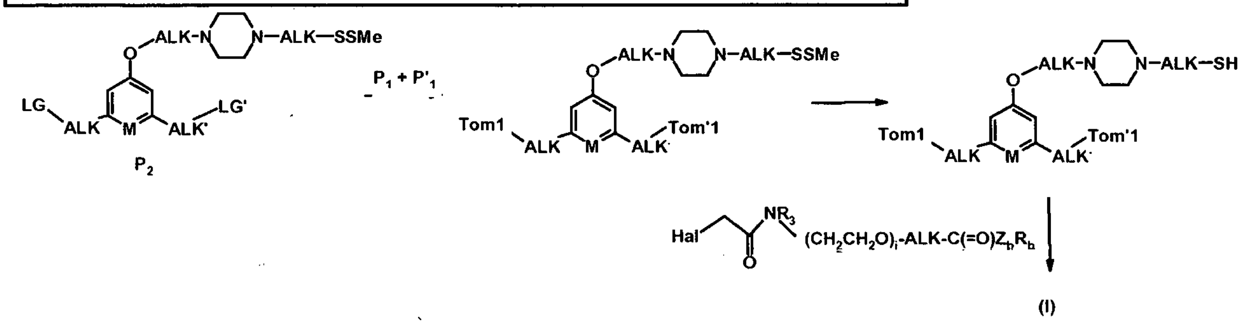


**Schéma 6**

## Préparation de $P_2$

On utilise le composé $\mathbf{P_2}$ de formule :

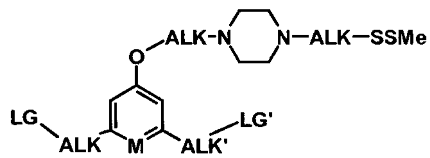

qui est obtenu par amination réductrice selon le **Schéma 6'** ci-dessous.

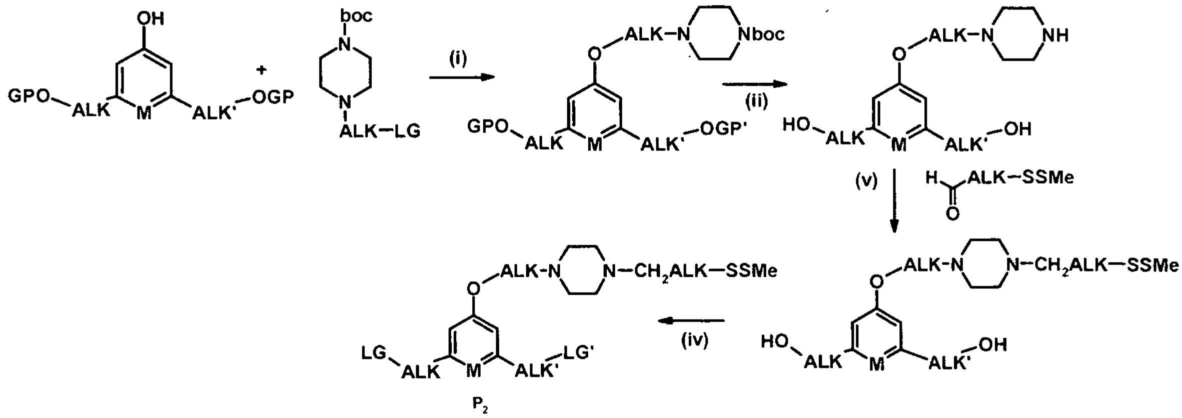


**Schéma 6'**

v. introduction du groupe -ALK-SSMe par amination réductrice effectuée par exemple en présence de cyanoborohydrure et d'isopropoxyde de titane ;

Une variante similaire à celle représentée sur le **Schéma 3**" et utilisant la méthode d'alkylation décrite par Kitagawa T. et al. JACS, 2006, 128(45), 14448-14449 est donnée sur le **Schéma 6**" :

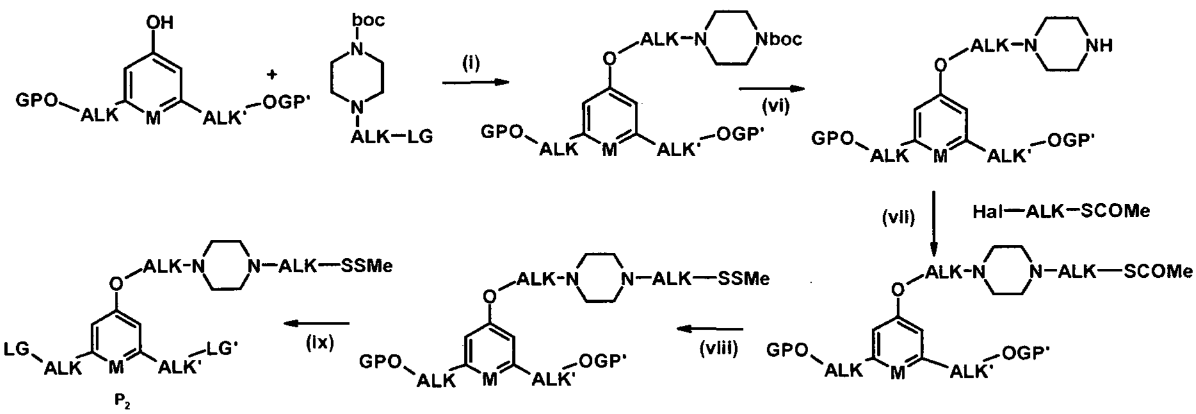


**Schéma 6"**

i. alkylation par pipérazine mono protégée (cf. étape i. de **Schéma 5**') ;
vi. déprotection sélective du groupe Boc en milieu acide
vii. alkylation par un halogénure d'alkyle porteur d'un groupe thioacétyle en présence de carbonate de césium dans un solvant aprotique polaire comme le DMF ;
viii. clivage sélectif du groupe acétyle en milieu basique faible et formation du groupe -SSMe par réaction du thiol intermédiaire avec $MeSSO_2Me$ en présence d'une base telle que la TEA ;
ix. clivage des groupements protecteurs GP et GP' et transformation des groupes hydroxyles de préférence en mésylates avec le chlorure de méthanesulfonyle.

L = –O-ALK-$NR_3$-ALK-S-$(CH_2CH_2O)_i$-ALK-

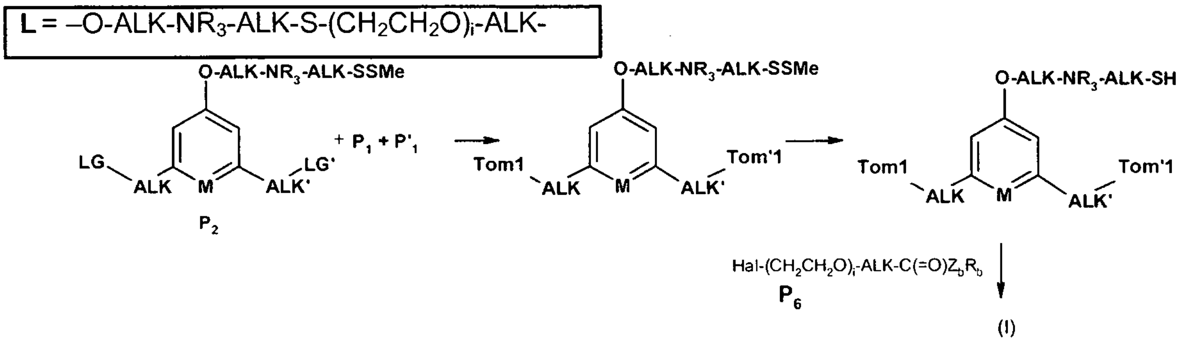


**Schéma 7**

Ce Schéma s'inspire du **Schéma 3** précédent. Le composé $\mathbf{P_6}$ peut être par exemple le composé de N° CAS 564476-32-0 qui est préparé selon WO 03068144 (cf. composé 10a de la figure 7) ou bien le composé de N° CAS 309916-91-4. On peut préparer des composés analogues de longueurs de chaîne i différentes selon le même principe de la figure 7 de WO 03068144 à partir du composé PEG correspondant.

**à propos de $P_6$**

RbZb-CO-ALK-$(OCH_2CH_2)_i$-Br ou

peut être préparé selon les schémas ci-dessous :

cas où ALK=$CH_2CH_2$

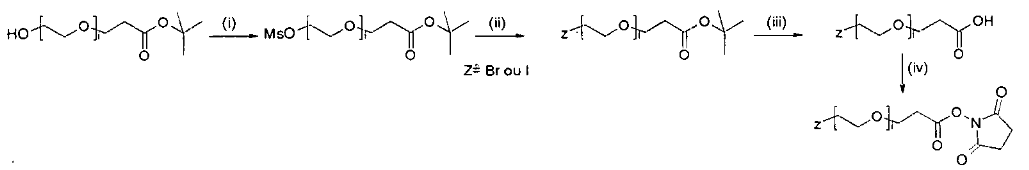


cas où ALK≠$CH_2CH_2$

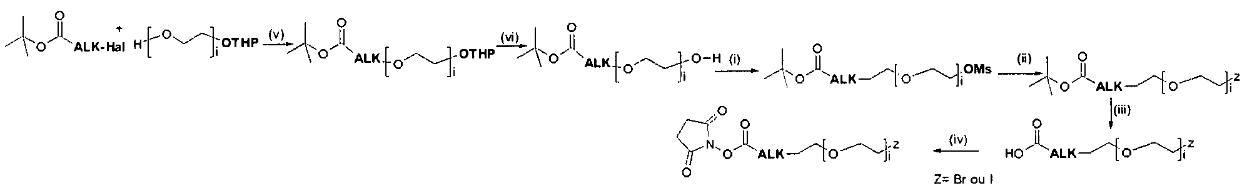


Etape (i) : activation de l'alcool sous forme de mésylate ; la réaction est réalisée dans un solvant polaire aprotique anhydre comme le DCM par traitement avec le chlorure de mésyle en présence d'une base comme la TEA.

Etape (ii) : échange mésylate / halogène ; la réaction est réalisée au reflux d'un solvant polaire aprotique comme l'acétone avec un halogénure de sodium comme l'iodure de sodium.

Etape (iii) : déprotection à l'aide d'une solution d'acide chlorhydrique (par ex. solution dans le dioxane) ou d'acide trifluoroacétique.

Etape (iv) : activation de l'acide ; la réaction est réalisée à température ambiante dans un solvant polaire

aprotique comme le DCM par traitement avec le NHS en présence d'un agent de couplage comme le DCC.

Etape (v) : élongation de la chaine PEG ; la réaction est réalisée dans un solvant polaire aprotique anhydre comme le THF ou le DMF par traitement d'un ester halogéné avec l'alcoolate d'un diol PEG monoprotégé sous forme d'éther de tétrahydropyrane (THP). La préparation de ce type de diol PEG monoprotégé est bien décrite dans la littérature, voir par ex. Richard A. et al. Chem. Eur. J. 2005, 11, 7315-7321 ou Sakellariou E.G., et al. Tetrahedron 2003, 59, 9083-9090. Etape (vi) : déprotection à l'aide d'une solution d'acide chlorhydrique 0,1N (par ex. solution dans le dioxane ou l'éthanol).

L'homme du métier pourra s'inspirer des conditions opératoires des exemples décrits ci-après.

**procédé de préparation du conjugué**

Le conjugué est obtenu par le procédé consistant à :

(i) mettre en contact et laisser réagir une solution aqueuse de l'agent de ciblage éventuellement tamponnée et une solution d'un composé de formule (I) ;
(ii) puis à éventuellement séparer le conjugué formé à l'étape (i) du composé de formule (I) et/ou de l'agent de ciblage n'ayant pas réagi et/ou des agrégats qui se seraient formés.

Selon une variante, on sépare à l'étape (ii) le conjugué qui s'est formé à l'étape (i) de l'agent de ciblage n'ayant pas réagi et des agrégats éventuellement présents dans la solution. Selon une autre variante, on ne sépare à l'étape (ii) le conjugué de l'étape (i) que du composé de formule (I) n'ayant pas réagi et des agrégats qui se seraient formés et on laisse dans la solution l'agent de ciblage qui n'aurait éventuellement pas réagi.

Le composé de formule (I) comprend de préférence une fonction activée, $-C(=O)Z_bR_b$, réactive vis-à-vis des groupes GCR2, notamment vis-à-vis des groupes amino présents sur les anticorps. Un groupe chimique peu réactif ou insuffisamment réactif peut être transformé aisément en un groupe plus réactif à l'aide d'une ou plusieurs réactions chimique connues de l'homme du métier ; par ex. -COOH + N-hydroxysuccinimide →

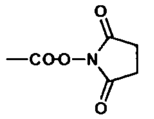

ou bien $-COO(C_1-C_6)$alkyle → -COOH → -COOH + N-hydroxysuccinimide →

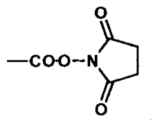

Un exemple de procédé applicable dans le cas d'un anticorps et d'un composé de formule (I) est celui donné à l'exemple 1.

La solution aqueuse de l'agent de ciblage peut être tamponnée à l'aide par exemple de tampons tels que par exemple le phosphate de potassium ou l'acide N-2-hydroxyéthylpipérazine-N'-2-éthanesulfonique (tampon HEPES). Le tampon dépend de la nature de l'agent de ciblage. Le composé de formule (I) est mis en solution dans un solvant organique polaire, par exemple le DMSO ou la DMA.

La réaction a lieu à une température comprise généralement entre 20 et 40°C. La durée de la réaction peut varier entre 1 à 24 h. La réaction entre l'agent de ciblage et composé de formule (I) peut être suivie par SEC avec un détecteur réfractométrique et/ou ultraviolet afin d'en déterminer l'état d'avancement. Si le taux de greffage est insuffisant, on peut laisser réagir plus longtemps et/ou rajouter du composé de formule (I). On pourra se reporter à la méthode générale donnée dans la partie exemples pour plus de détails sur des conditions particulières utilisables pour la conjugaison.

L'homme du métier dispose de différentes techniques chromatographiques pour la séparation de l'étape (ii) : le conjugué peut être purifié par exemple par chromatographie d'exclusion stérique (SEC), par chromatographie d'adsorption (comme l'échangeuse d'ions, IEC), par chromatographie d'interaction hydrophobe (HIC), par chromatographie d'affinité, par chromatographie sur des supports mixtes comme l'hydroxyapatite céramique ou par HPLC. La purification par dialyse ou diafiltration peut également être utilisée.

On entend par « agrégats » les associations qui peuvent se former entre deux agents de ciblage ou plus, les

agents de ciblage ayant été modifiés ou non par conjugaison. Les agrégats sont susceptibles de se former sous l'influence d'un grand nombre de paramètres tels qu'une concentration élevée en agent de ciblage dans la solution, le pH de la solution, des forces de cisaillement élevées, le nombre de dimères greffés et leur caractère hydrophobe, la température (voir les références citées dans l'introduction de J.Membrane Sci. 2008, 318, 311-316), l'influence de certains d'entre eux n'étant parfois pas éclaircie avec précision. Dans le cas des protéines ou des anticorps, on pourra se reporter à AAPS Journal, « Protein Aggregation and Bioprocessing » 2006, 8(3), E572-E579. La teneur en agrégats peut être déterminée à l'aide de techniques connues telles que la SEC (voir à ce propos, Analytical Biochemistry 1993, 212(2), 469-480).

Après l'étape (i) ou (ii), la solution du conjugué peut subir une étape (iii) d'ultrafiltration et/ou de diafiltration. On obtient donc à l'issue de ces étapes le conjugué en solution aqueuse.

**<u>anticorps</u>**

L'anticorps (voir à ce propos, Janeway et al. « Immunobiology », 5ème édition, 2001, Garland Publishing, New York) pourra être choisi parmi ceux décrits notamment dans les demandes WO 04043344, WO 08010101, WO 08047242, WO 05009369 (anti-CA6). L'anticorps peut être notamment monoclonal, polyclonal ou multispécifique. Il peut s'agir aussi d'un fragment d'anticorps. Il peut s'agir aussi d'un anticorps murin, humain, humanisé ou chimérique.

**<u>conjugué</u>**

Un conjugué comprend généralement de l'ordre de 1 à 10 dimère(s) de pyrrolo[1,4]benzodiazépine attaché(s) à l'agent de ciblage (il s'agit du taux de greffage ou "drug-to-antibody ratio" ou "DAR" en Anglais). Ce nombre varie en fonction de la nature de l'agent de ciblage et du dimère ainsi que des conditions opératoires utilisées pour la conjugaison (par exemple le nombre d'équivalents de dimère par rapport à l'agent de ciblage, le temps de réaction, la nature du solvant et de l'éventuel cosolvant). La mise en contact de l'agent de ciblage et du dimère conduit à un mélange comprenant : plusieurs conjugués se distinguant individuellement les uns des autres par des DAR différents ; éventuellement l'agent de ciblage n'ayant pas réagi (dans le cas d'une réaction incomplète) ; éventuellement des agrégats. Le DAR qui est déterminé sur la solution finale par exemple par spectroscopie UV correspond donc à un DAR moyen.

Dans le cas où l'agent de ciblage est un anticorps, la spectroscopie UV peut être une méthode utilisée pour déterminer le DAR. Cette méthode s'inspire de celle présentée dans Antony S. Dimitrov (ed), LLC, 2009, « Therapeutic Antibodies and Protocols », vol. 525, 445, Springer Science. Elle consiste à mesurer l'absorbance d'une solution de conjugué après l'étape de séparation (ii) à deux longueurs d'onde notées LO1 et LO2. On utilise les coefficients d'extinction molaires suivants de l'anticorps nu et du dimère de pyrrolo[1,4]benzodiazépine mesurés préalablement à la conjugaison.

Les absorbances de la solution de conjugué à LO1 et LO2, ($A_{LO1}$) et ($A_{LO2}$) sont mesurées soit sur le pic correspondant du spectre SEC (ceci permet de calculer un "DAR(SEC)") ou en utilisant un spectrophotomètre UV classique (ceci permet de calculer un "DAR(UV)"). Les absorbances peuvent être exprimées sous la forme :

$$A_{LO1} = (c_D \times e_{D\,LO1}) + (c_A \times e_{A\,LO\,1})$$

$$A_{LO2} = (c_D \times e_{D\,LO\,2}) + (c_A \times e_{A\,LO\,2})$$

équations pour lesquelles :

- $c_D$ et $c_A$ désignent respectivement les concentrations dans la solution de la partie du conjugué relative au dimère de pyrrolo[1,4]benzodiazépine et la partie du conjugué relative à l'anticorps ;
- $e_{DLO1}$ et $e_{DLO2}$ désignent respectivement les coefficients d'absorption molaires du dimère de pyrrolo[1,4]benzodiazépine avant conjugaison aux deux longueurs d'onde LO1 et LO2 ;
- $e_{ALO1}$ et $e_{ALO2}$ désignent respectivement les coefficients d'absorption molaires de l'anticorps nu aux deux longueurs d'onde LO1 et LO2.

On entend par anticorps nu, l'anticorps auquel n'est attaché aucun dimère de pyrrolo[1,4]benzodiazépine, c'est-à-dire l'anticorps avant l'étape de conjugaison.

La résolution de ces deux équations conduit à :

$$c_D = [(e_{A\,LO1} \times A_{LO\,2}) - (e_{A\,LO\,2} \times A_{LO\,1})] / [(e_{D\,LO\,2} \times e_{A\,LO\,1}) - (e_{A\,LO\,2} \times e_{D\,LO\,1})]$$

$$c_A = [A_{LO1} - (c_D \times e_{D\,LO\,1})] / e_{A\,LO\,1}$$

Le DAR moyen correspond alors à $c_D/c_A$. Dans le cas des dimère de pyrrolo[1,4]benzodiazépine, on considère les deux longueurs d'onde : LO1= 280 nm et LO2= 320 nm. Le DAR moyen mesuré sur spectre SEC est de préférence compris entre 1 et 10, de préférence entre 1,5 et 7.

Le conjugué peut être utilisé en tant qu'anticancéreux. De par la présence de l'agent de ciblage, le conjugué est rendu très sélectif vis-à-vis des cellules tumorales plutôt que des cellules saines. Ceci permet de diriger le composé de formule (I) qui a une activité anticancéreuse dans un environnement proche de celles-ci ou directement à l'intérieur de celles-ci (à ce propos, voir les publications suivantes qui décrivent l'utilisation de conjugués d'anticorps monoclonaux dans le traitement de cancers : « Antibody-drug conjugates for cancer therapy » Carter P.J., et al., Cancer J. 2008, 14, 154-169 ; « Targeted cancer therapy: conferring specificity to cytotoxic drugs » Chari R., Acc. Chem. Res. 2008, 41, 98-107). Il est possible de traiter des cancers solides ou liquides.

Le conjugué est formulé sous forme d'une solution aqueuse tamponnée à une concentration généralement comprise entre 1 et 10 mg/ml. Cette solution peut être injectée sous forme de perfusion telle qu'elle ou bien être rediluée pour former une solution de perfusion.

**[Exemples]**

Les déplacements chimiques (δ en ppm) sont exprimés en ppm.

Méthode A : chromatographie Liquide Haute pression - spectrométrie de masse (LCMS)

Les spectres ont été obtenus sur un appareil Waters UPLC-SQD en mode électrospray positif et/ou négatif (ES+/-). Conditions chromatographiques : colonne : ACQUITY BEH C18 1,7 μm-2,1x50 mm ; solvants : A : $H_2O$ (0,1% acide formique) B : $CH_3CN$ (0,1% acide formique); température de colonne : 50°C ; débit : 1 ml/min ; gradient (2 min) : de 5 à 50 % de B en 0,8 min ; 1,2 min : 100 % de B ; 1,85 min : 100 % de B ; 1,95 : 5 % de B.

Méthode B : chromatographie Liquide Haute pression - spectrométrie de masse (LCMS)

Les spectres ont été obtenus sur un appareil Waters ZQ en mode électrospray positif et/ou négatif (ES+/-). Conditions chromatographiques : colonne : XBridge C18 2,5 μm 3x50 mm ; solvants : A : $H_2O$ (0,1 % acide formique) B : $CH_3CN$ (0,1 % acide formique ; température de colonne : 70°C ; débit : 0,9 ml/min ; gradient (7 min) : de 5 à 100 % de B en 5,3 min ; 5,5 min : 100 % de B ; 6,3 min: 5 % de B.

Méthode C : spectrométrie de masse (MS)

Les spectres ont été réalisés par ionisation chimique (gaz réactant : ammoniac) sur appareil WATERS GCT of (introduction directe sans LC).

Méthode D : chromatographie Liquide Haute pression - spectrométrie de masse (LCMS)

Les spectres ont été obtenus sur un appareil Waters UPLC-SQD en mode électrospray positif et/ou négatif (ES+/-). Conditions chromatographiques : colonne : ACQUITY BEH C18 1,7 μm-2,1x50 mm ; solvants : A : $H_2O$ (0,1% acide formique) B : $CH_3CN$ (0,1% acide formique); température de colonne : 70°C ; débit : 1 ml/min ; gradient (2 min) : de 5 à 50% de B en 1 min ; 1,3 min : 100% de B ; 1,45 min : 100% de B ; 1,75 : 5% de B.

Méthode E : chromatographie Liquide Haute pression - spectrométrie de masse (LCMS)

Les spectres ont été obtenus sur un appareil Waters UPLC-SQD en mode ionisation : électrospray en mode positif et/ou négatif (ES+/-). Conditions chromatographiques : colonne : ACQUITY BEH C18 1,7 μm - 2,1x50 mm ; solvants : A : $H_2O$ (0,1 % acide formique) B : $CH_3CN$ (0,1% acide formique) ; température de colonne : 70°C ; débit : 1 ml/min ; gradient (4 min) : de 5 à 100% de B en 3,15 min ; 3,75 : 5% de B.

Méthode F : chromatographie Liquide Haute pression - spectrométrie de masse (LCMS)

Les spectres ont été obtenus sur un appareil Waters UPLC-SQD en mode ionisation électrospray en mode positif et/ou négatif (ES+/-). Conditions chromatographiques : colonne : ACQUITY BEH C18, 1,7 μm - 2,1x30 mm ; solvants : A : $H_2O$ (0,1% acide formique) B : $CH_3CN$ (0,1% acide formique) ; température de colonne : 45°C ; débit : 0,6 ml/min ; gradient (2 min) : de 5 à 50% de B en 1 min ; 1,3 min : 100% de B ; 1,45 min : 100% de B ; 1,75 min : 5% de B.

Méthode G : déglycosylation et spectrométrie de masse (HRMS) d'un conjugué

La déglycosylation est une technique de digestion enzymatique à l'aide de glycosidase. Elle est faite à partir de 500 μl de conjugué + 100 μl de tampon Tris HCl 50 mM + 10 μl d'enzyme glycanase-F (100 unités d'enzyme lyophilisée/100 μl d'eau). Le mélange est passé au vortex et maintenu une nuit à 37°C. L'échantillon déglycosylé est alors prêt à être analysé en HRMS. Selon le cas, l'analyse HRMS de l'échantillon peut aussi être effectuée sans déglycosylation préalable. Dans les deux cas, les spectres de masse ont été obtenus sur un appareil Waters Q-Tof-2 en mode électrospray positif (ES+). Conditions chromatographiques : colonne : 4 μm BioSuite 250 URH SEC 4,6x300 mm (Waters) ; solvants : A : formiate d'ammonium 25 mM +1% d'acide formique : B : $CH_3CN$ ; température de colonne 30°C : débit 0,4 ml/min ; isocratique 70%A+30%B (15 min).

Tous les composés intermédiaires décrits dans cette demande sont revendiqués pour leur utilisation pour la préparation d'un composé de formule (I). Plus particulièrement pour chaque exemple, tous les composés intermédiaires décrits sont revendiqués pour leur utilisation pour la préparation du composé de formule (I) respectif.

**Exemple 1**

**1.1. Préparation du conjugué**

On prépare un conjugué en faisant réagir le hu2H11 (dénommé aussi hu53 2H11 en page 15 de WO 2008010101 **;** il s'agit d'un anticorps comprenant une Vh ayant la séquence d'aminoacides SED ID N°24)) et le 3-(2-{2-[2-(2-{3-[3-(2-{[2-(2,6-bis-[(S)-2-éth-(E)-ylidène-7-diméthoxy-1,2,3,11 a-tétrahydro-pyrrolo[2,1c][1,4]benzodiazépin-5-one-8-yloxyméthyl]-pyridin-4-yloxy)-éthyl]-méthyl-amino}-1,1-diméthyl-éthylsulfanyl)-2,5-dioxo-pyrrolidin-1-yl]-propanoylamino}-éthoxy)-éthoxy]-éthoxy}-éthoxy)-propanoate de N-hydroxysuccinimidyle.

A 8,19 mg de hu2H11 dans 2,16 mL d'un tampon aqueux de concentration 0,05 M en acide N-(2-hydroxyéthyl)-pipérazine-N'-2-éthanesulfonique (HEPES), 0,05 M en NaCl et 2 mM en acide éthylène-diamine-tetra-acétique (EDTA) de pH=8, sont additionnés 516 μg de 3-(2-{2-[2-(2-{3-[3-(2-{[2-(2,6-bis-[(S)-2-éth-(E)-ylidène-7-diméthoxy-1,2,3,11a-tétrahydro-pyrrolo[2,1 c][1,4]benzodiazépin-5-one-8-yloxyméthyl]-pyridin-4-yloxy)-éthyl]-méthyl-amino}-1,1-diméthyl-éthylsulfanyl)-2,5-dioxo-pyrrolidin-1-yl]-propanoylamino}-éthoxy)-éthoxy]-éthoxy}-éthoxy)-propanoate de N-hydroxysuccinimidyle en solution dans 540 μL de DMA. Après 3 h d'agitation à TA, le mélange est filtré sur Millex$^R$-SV 0,45 μM (PVDF Durapore Millipore) et purifié sur une colonne Superdex™ 200 prep grade (Colonne Hiload™ 26/60 GE) préalablement équilibrée dans un tampon phosphate salin amené à pH=6,5 par addition de HCl. Les fractions d'intérêt sont réunies et concentrées sur Amicon Ultra-15 (Ultracel 50k Millipore) puis filtrées sur Sephadex G-25 (Colonnes NAP-5 et NAP-10 GE) préalablement équilibrée dans un tampon aqueux de concentration 10 mM en histidine contenant 10% de sucrose et 5% de NMP.

Le conjugué obtenu (2,5 mL) est dosé par spectrophotométrie en utilisant les coefficients d'extinction de la 4-{2-[méthyl-(2-méthyl-2-mercapto-propyl)-amino]-éthoxy}-2,6-bis-[(S)-2-éth-(E)-ylidène-7-diméthoxy-1,2,3,11a-tetrahydropyrrolo[2,1c][1,4]benzodiazépin-5-one-8-yloxyméthyl]-pyridine ($\varepsilon_{319\ nm}$= 8848 $M^{-1}$ $cm^{-1}$ and $\varepsilon_{280\ nm}$= 8634 $M^{-1}$ $cm^{-1}$) et de hu2H11 ($\varepsilon_{280\ nm}$= 208380 $M^{-1}cm^{-1}$): une moyenne de 3,8 dimères de tomaymycine par molécule d'anticorps à la concentration de 1,52 mg/mL a été déterminée.

**1.2. 3-(2-{2-[2-(2-{3-[3-(2-{[2-(2,6-bis-[(S)-2-éth-(E)-ylidène-7-diméthoxy-1,2,3,11a-tetrahydropyrrolo[2,1c][1,4]benzodiazépin-5-one-8-yloxyméthyl]-pyridin-4-yloxy)-éthyl]-méthylamino}-1,1-diméthyl-éthylsulfanyl)-2,5-dioxo-pyrrolidin-1-yl]-propanoylamino}-éthoxy)-éthoxy]-éthoxy}-éthoxy)-propanoate de N-hydroxysuccinimidyle**

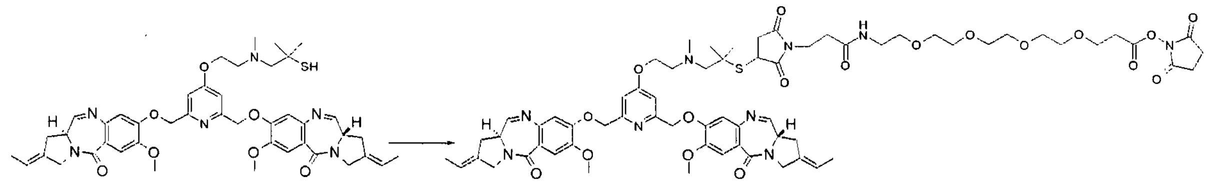

A 3,3 mg de diisopropyléthylamine supportée sur résine (3,72 mmol/g), on ajoute une solution de 9,82 mg de 4-{2-[méthyl-(2-méthyl-2-mercapto-propyl)-amino]-éthoxy}-2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridine dans 50 μL de DMA ainsi qu'une solution de 6,8 mg de 3-{2-[2-(2-{2-[3-(2,5-dioxo-2,5-dihydro-pyrrol-1-yl)-propanoylamino]-éthoxy}-éthoxy)-éthoxy]-éthoxy}-propanoate de N-hydroxysuccinimidyle dans 50 μL de DMA. Le mélange obtenu est agité pendant 24 h à TA puis filtré sur silice (Interchrom Puriflash Silice 15/35U 2G) en utilisant un gradient de 0 à 10% de méthanol dans le DCM. Les fractions contenant le produit recherché sont réunies, concentrées sous PR puis purifiées par chromatographie flash sur silice (Interchrom Puriflash Silice 15/35U 2G) en utilisant un gradient de 0 à 10% de MeOH dans le DCM. Les fractions contenant le produit recherché sont réunies et concentrées sous PR. On obtient ainsi 1,16 mg de 3-(2-{2-[2-(2-{3-[3-(2-{[2-(2,6-bis-[(S)-2-éth-(E)-ylidène-7-diméthoxy-1,2,3,11a-tétrahydro-pyrrolo[2,1c][1,4]benzodiazépin-5-one-8-yloxyméthyl]-pyridin-4-yloxy)-éthyl]-méthyl-amino}-1,1-diméthyl-éthylsulfanyl)-2,5-dioxo-pyrrolidin-1-yl]-propanoylamino}-éthoxy)-éthoxy]-éthoxy}-éthoxy)-propanoate de N-hydroxysuccinimidyle. LC/MS (E) : tr= 1,38 min ; [M+H]+ : m/z 1322.

**1.3. 4-{2-[méthyl-(2-méthyl-2-mercapto-propyl)-amino]-éthoxy}-2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridine**

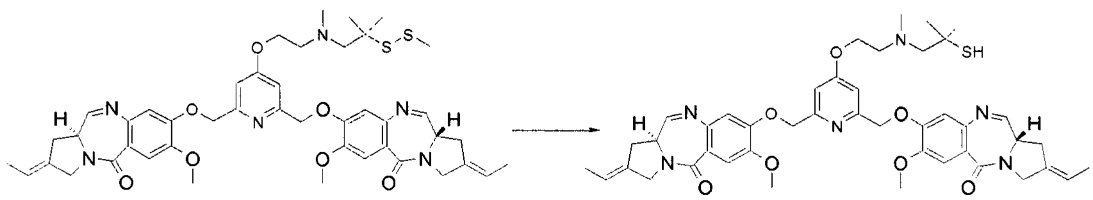

A 40 mg de 4-{2-[méthyl-(2-méthyl-2-méthyldisulfanyl-propyl)-amino]-éthoxy}-2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridine en solution dans 2,1 mL de méthanol et 930 μL de DMF, est additionnée une solution de 40 mg de chlorhydrate de tris(2-carboxyéthyl)phosphine et de 36,6 mg de $NaHCO_3$ dans 680 μL d'eau. Le mélange est agité 45 min à TA puis concentré sous PR et purifié par chromatographie flash sur silice (Merck SuperVarioFlash 15 g column, Si60 15-40 μm), en utilisant un gradient de 0 à 10% de MeOH dans un mélange DCM/acétonitrile 9:1. Les fractions contenant le produit recherché sont réunies et concentrées sous PR. On obtient ainsi 21 mg de 4-{2-[méthyl-(2-méthyl-2-mercapto-propyl)-amino]-éthoxy}-2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridine. LC/MS (E) : tr= 1,28 min ; [M+H]+ : m/z 809 ; [M+$H_2O$+H]+ : m/z 827.

**1.4. 4-{2-[méthyl-(2-méthyl-2-méthyldisulfanyl-propyl)-amino]-éthoxy}-2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridine**

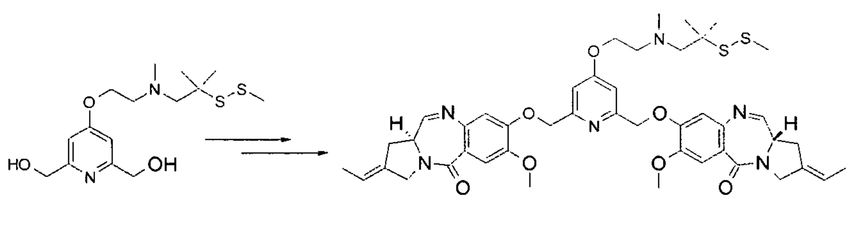

A une solution de 22 mg de 4-{2-[méthyl-(2-méthyl-2-méthyldisulfanyl-propyl)-amino]-éthoxy}-2,6bis-(hydroxyméthyl)-pyridine et de 53 µL de TEA dans 0,5 mL de DCM refroidie à -25°C, est additionnée 19,6 µL de chlorure de méthanesulfonyle. Après 30 min d'agitation, le mélange est hydrolysé et la phase organique lavée à l'eau puis séchée sur $MgSO_4$ et concentrée sous PR. Le résidu ainsi obtenu (22 mg) est additionné à une solution de 20 mg de tomaymycine dans 0,7 mL de DMF, ainsi que 30 mg de $K_2CO_3$ et 12 mg de KI. Le mélange est agité 2 h à 30°C puis hydrolysé par 4 mL d'eau. Le précipité résultant est lavé à l'eau, séché sous vide puis dissout dans du DCM, concentré sous PR et purifié par chromatographie flash sur silice (Merck SuperVarioFlash 15 g column, Si60 15-40 µm), en utilisant un gradient de 0 à 5% de MeOH dans le DCM. Les fractions contenant le produit recherché sont réunies et concentrées sous PR. On obtient ainsi 8 mg de 4-{2-[méthyl-(2-méthyl-2-méthyldisulfanyl-propyl)-amino]-éthoxy}-2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridine. RMN $^1$H (500 MHz, CHLOROFORM-d) : 1,28 (s, 6 H) ; 1,76 (d, J=6,4 Hz, 6 H) ; 2,39 (s, 3 H) ; 2,43 (s, 3 H) ; 2,60 (s, 2 H) ; 2,91 (s, 2 H) ; 2,97 (s, 4 H) ; 3,91 (d, J=4,2 Hz, 2 H) ; 4,00 (s, 6 H) ; 4,09 (s, 2 H) ; 4,27 (s, 4 H) ; 5,27 (s, 4 H) ; 5,61 (q, J=6,4 Hz, 2 H) ; 6,85 (s, 2 H) ; 7,00 (s, 2 H) ; 7,56 (s, 2 H) ; 7,65 (d, J=4,2 Hz, 2 H). LC/MS (A) : tr= 0,74 min ; [M+H]+ : m/z 855; [M-H]- : m/z 853.

**1.5. 4-{2-[méthyl-(2-méthyl-2-méthyldisulfanyl-propyl)-amino]-éthoxy}-2,6-bis-(hydroxyméthyl)-pyridine**

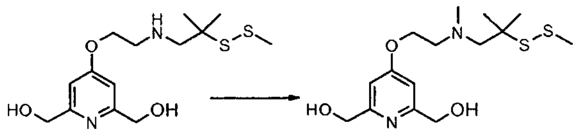

A une suspension de 322 mg de 4-[2-(2-méthyl-2-méthyldisulfanyl-propylamino)-éthoxy]-2,6-bis-(hydroxyméthyl)-pyridine dans 262 µL de formaldéhyde refroidie à 0°C, est additionné 365 µL d'acide formique. Le mélange est chauffé 1 h et quart à 100°C. Après retour à l'ambiante, le mélange est hydrolysé puis additionné d'une solution aqueuse de soude 5N jusqu'à l'obtention d'un pH=12. La phase aqueuse est extraite 3x avec de AcOEt et les phases organiques rassemblées sont séchées sur $MgSO_4$ et concentrées sous PR. On obtient ainsi 310 mg de 4-{2-[méthyl-(2-méthyl-2-méthyldisulfanyl-propyl)-amino]-éthoxy}-2,6-bis-(hydroxyméthyl)-pyridine. RMN $^1$H (300 MHz, DMSO-d6) : 1,26 (s, 6 H) ; 2,39 (s, 3 H) ; 2,40 (s, 3 H) ; 2,60 (s, 2H) ; 2,88 (t, J=5,7 Hz, 2 H) ; 4,13 (t, J=5,7 Hz, 2 H) ; 4,45 (d, J=6,0 Hz, 4 H) ; 5,31 (t, J=6,0 Hz, 2 H) ; 6,85 (s, 2 H). LC/MS (A) : tr= 0,22 min ; [M+H]+ : m/z 333 ; [M+HCO2H-H]- : m/z 377.

**1.6. 4-[2-(2-méthyl-2-méthyldisulfanyl-propylamino)-éthoxy]-2,6-bis-(hydroxyméthyl)-pyridine**

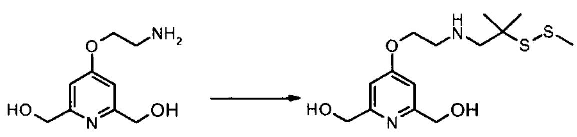

A une suspension de 390 mg de 4-[2-amino-éthoxy]-2,6-bis-(hydroxyméthyl)-pyridine (préparé après déprotection du groupe boc de 4-(2-tert-butoxycarbonylamino-éthoxy)-2,6-bis-(hydroxyméthyl)-pyridine décrit en page 101 de WO 07085930**)** dans 2 mL de THF sont additionnés 270 µL de 2-(méthyldithio)-isobutyraldéhyde et 730 µL d'isopropoxyde de titane. Après 20 min, 270 µL de 2-(méthyldithio)-isobutyraldéhyde et 730 µL d'isopropoxyde de titane supplémentaires sont additionnés et le mélange est agité à TA pendant 2 h. Le mélange est alors additionné de 6 mL d'éthanol, agité pendant 20 min à TA puis additionné de 124 mg de cyanoborohydrure de sodium. Après 45 min d'agitation, 124 mg de cyanoborohydrure de sodium supplémentaires sont additionnés et après 1 h d'agitation, le mélange est concentré sous PR, dilué dans AcOEt et de l'eau. Le précipité résultant est filtré, dissout dans une solution aqueuse HCl 1M. La phase aqueuse obtenue est amenée à pH basique avec une solution aqueuse de soude 5M, extraite trois fois avec du DCM et les phases organiques rassemblées sont concentrées sous PR. On obtient ainsi 322 mg de 4-[2-(2-méthyl-2-méthyldisulfanyl-propylamino)-éthoxy]-2,6-bis-(hydroxyméthyl)-pyridine. RMN $^1$H (400 MHz, DMSO-d6) : 1,26 (s, 6 H) ; 1,81 (m large, 1 H) ; 2,39 (s, 3 H) ;2,67 (s large, 2 H) ; 2,94 (t large, J=5,7 Hz, 2 H) ; 4,11 (t, J=5,7 Hz, 2 H) ; 4,45 (d, J=5,5 Hz, 4 H) ; 5,32 (t,J=5,5 Hz, 2 H) ; 6,85 (s, 2 H). LC/MS (A) : tr= 0,24 min ; [M+H]+ : m/z 347.

**Exemple** 2

**2.1. Préparation du conjugué**

On prépare comme pour l'ex.1 un conjugué en faisant réagir le hu2H11 et le 3-{2-[2-(2-{2-[2-(2-{[2-(2,6-bis-[(S)-2-éth-(E)-ylidène-7-diméthoxy-1,2,3,11a-tetrahydropyrrolo[2,1 c][1,4]benzodiazépin-5-one-8-yloxyméthyl]-pyridin-4-yloxy)-éthyl]-méthylamino}-1,1-dimëthyléthylsulfanyl)-acétylamino]-éthoxy}-éthoxy)-éthoxy]-éthoxy}-propanoate de N-hydroxysuccinimidyle. Le conjugué obtenu est dosé par spectrophotométrie en utilisant les coefficients d'extinction de la 4-{2-[méthyl-(2-méthyl-2-mercapto-propyl)-amino]-éthoxy}-2,6-bis-[(S)-2-éth-(E)-ylidène-7-diméthoxy-1,2,3,11a-tetrahydropyrrolo[2,1c][1,4]benzodiazepine-5-one-8-yloxyméthyl]-pyridine ($\varepsilon_{319\,nm}$= 8848 $M^{-1}$ $cm^{-1}$, $\varepsilon_{280\,nm}$= 8634 $M^{-1}$ $cm^{-1}$) et de hu2H11 ($\varepsilon_{280}$ nm= 208380 $M^{-1}cm^{-1}$) : une moyenne de 5,6 dimères de tomaymycine par molécule d'anticorps a été déterminée.

**2.1. 3-{2-[2-(2-{2-[2-(2-{[2-(2,6-bis-[(S)-2-éth-(E)-ylidène-7-diméthoxy-1,2,3,11a-tetrahydropyrrolo[2,1c][1,4]benzodiazépin-5-one-8-yloxyméthyl]-pyridin-4-yloxy)-éthyl]-méthylamino}-1,1-diméthyl-éthylsulfanyl)-acétylamino]-éthoxy}-éthoxy)-éthoxy]-éthoxy}-pronanoate de N-hydroxysuccinimidyle**

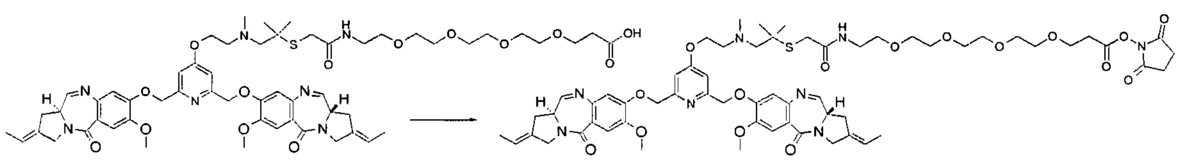

A 12 mg d'acide 3-{2-[2-(2-{2-[2-(2-{[2-(2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridin-4-yloxy)-éthyl]-méthyl-amino}-1,1-diméthyl-éthylsulfanyl)-acétylamino]-éthoxy}-éthoxy)-éthoxy]-éthoxy}-propanoïque en solution dans 1 mL de THF et 1 mL de DCM sont additionnés 5,5 mg de carbonate de N,N'-disuccinimidyle et 15 μL de DIPEA. Après 3 h à TA, 4 mL de DCM sont additionnés et la phase organique résultante est lavée deux fois avec de l'eau, séchée sur $MgSO_4$, concentrée sous PR et purifiée par chromatographie flash sur silice (Interchrom Puriflash Silice 15/35U 2G), en utilisant un gradient de 3 à 8% de méthanol dans le DCM. On obtient ainsi 8 mg de 3-{2-[2-(2-{2-[2-(2-{[2-(2,6-bis-[(S)-2-éth-(E)-ylidène-7-diméthoxy-1,2,3,11a-tétra hydro-pyrrolo[2,1c][1,4]benzodiazépin-5-one-8-yloxyméthyl]-pyridin-4-yloxy)-éthyl]-méthyl-amino}-1,1-diméthyl-éthylsulfanyl)-acétylamino]-éthoxy}-éthoxy)-éthoxy]-éthoxy}-propanoate de N-hydroxysuccinimidyle. LC/MS (E) : tr=1,36 min ; [M+2 $H_2O$+Na]+ : m/z 1270 ; [M+$H_2O$+Na]+ : m/z 1252 ; [M+$H_2O$+H]+ : m/z 1229 ; [M+H]+ : m/z 1211.

**2.2. acide 3-{2-[2-(2-{2-[2-(2-{[2-(2,6-bis-[(S)-2-éth-(E)-ylidène-7-diméthoxy-1,2,3,11a-tetra hydro-pyrrolo[2,1c][1,4]benzodiazépin-5-one-8-yloxyméthyl]-pyridin-4-yloxy)-éthyl]-méthyl-amino}-1,1-diméthyl-éthylsulfanyl)-acétylamino]-éthoxy}-éthoxy)-éthoxy]-éthoxy}-propanoïque**

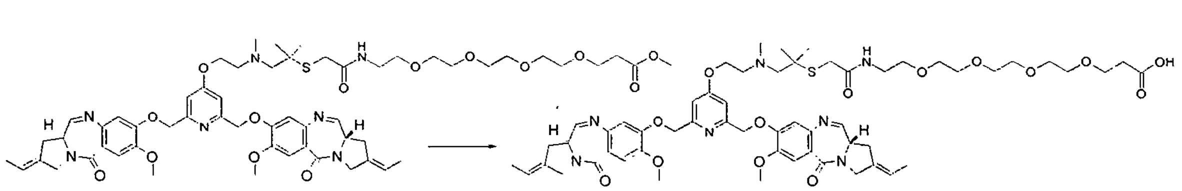

A 18 mg de 3-{2-[2-(2-{2-[2-(2-{[2-(2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridin-4-yloxy)-éthyl]-méthyl-amino}-1,1-diméthyl-éthylsulfanyl)-acétylamino]-éthoxy}-éthoxy)-éthoxy]-éthoxy}-propanoate de méthyle en solution dans 270 μL de THF, sont additionnés 17,5 μL d'une solution aqueuse de lithine et 100 μL d'eau. Après 2 h, le mélange est dilué dans du DCM et additionné d'un tampon phosphate à pH=3. La phase aqueuse résultante est extraite 3x avec du DCM et les phases organiques rassemblées sont séchées sur $MgSO_4$, concentrées sous PR et purifiées par chromatographie flash sur silice (Interchrom Puriflash Silice 15/35U 2G), en utilisant un gradient de 3 à 15% de méthanol dans le DCM. On obtient ainsi 11,5 mg d'acide 3-{2-[2-(2-{2-[2-(2-{[2-(2,6-bis-[(S)-2-éth-(E)-ylidène-7-diméthoxy-1,2,3,11a-tetrahydropyrrolo[2,1c][1,4]benzodiazépin-5-one-8-yloxyméthyl]-pyridin-4-yloxy)-éthyl]-méthyl-amino}-1,1-diméthyl-éthylsulfanyl)-acétylamino]-éthoxy}-éthoxy)-éthoxy]-éthoxy}-propanoïque. LC/MS (D) : tr=0,88 min ; [M+$H_2O$+Na]+ : m/z 1154 ; [M+$H_2O$+H]+ : m/z 1133 ; [M+H]+ : m/z 1115 ;

**2.3. 3-{2-[2-(2-{2-[2-(2-{[2-(2,6-bis-[(S)-2-éth-(E)-ylidène-7-diméthoxy-1,2,3,11a-tetrahydropyrrolo[2,1c][1,4]benzodiazépin-5-one-8-yloxyméthyl]-pyridîn-4-yloxy)-éthyl]-méthyl-amino}-1,1-diméthyl-éthylsulfanyl)-acétylamino]-éthoxy}-éthoxy)-éthoxy]-éthoxy}-propanoate** de **méthyle**

Préparé selon ex.1 à partir du 3-{2-[2-(2-{2-[2-(2-{[2-(2,6-bis-hydroxyméthyl-pyridin-4-yloxy)-éthyl]-méthyl-amino}-1,1-diméthyl-éthylsulfanyl)-acétylamino]-éthoxy}-éthoxy)-éthoxy]-éthoxy}-propanoate de méthyle :

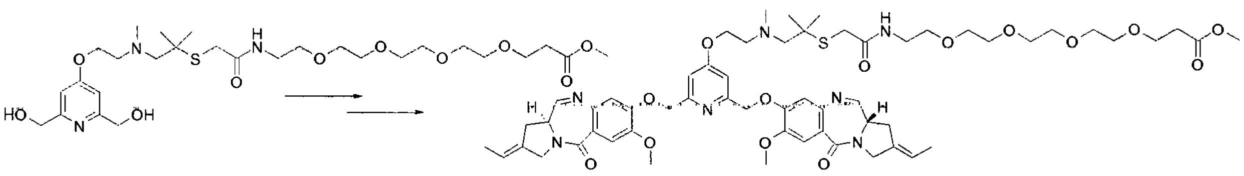

LC/MS (D) : tr= 0,93 min ; [M+$H_2O$+H]+ : m/z 1146 ; [M+H]+ : m/z 1128

**2.4. 3-{2-[2-(2-{2-[2-(2-{[2-(2,6-bis-hydroxyméthyl-pyridin-4-yloxy)-éthyl]-méthyl-amino}-1,1-diméthyl-éthylsulfanyl)-acétylamino]-éthoxy}-éthoxy)-éthoxy]-éthoxy}-propanoate de méthyle**

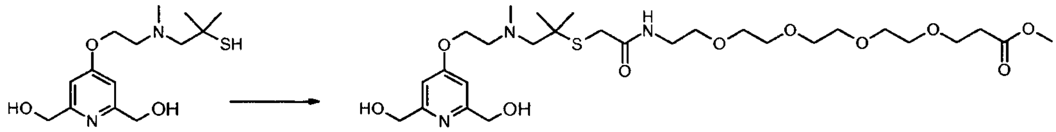

A 45 mg de 4-{2-[(2-mercapto-2-méthyl-propyl)-méthyl-amino]-éthoxy}-2,6-bis-(hydroxyméthyl)-pyridine en solution dans 1 mL de DMF, sont additionnés 73,7 mg de 3-[2-(2-{2-[2-(2-iodo-acétylamino)-éthoxy]-éthoxy}-éthoxy)-éthoxy]-propanoate de méthyle en solution dans 1 mL de DMF et 39 µL de DIPEA. Après 24 h à TA, le mélange est concentré sous PR et purifié par chromatographie flash sur silice (Analogix Super Flash $SiO_2$ SF25-8g), en utilisant un gradient de 0 à 10% de méthanol dans le DCM. On obtient ainsi 53 mg de 3-{2-[2-(2-{2-[2-(2-{[2-(2,6-bis-hydroxyméthyl-pyridin-4-yloxy)-éthyl]-méthyl-amino}-1,1-diméthyl-éthylsulfanyl)-acétylamino]-éthoxy}-éthoxy)-éthoxy]-éthoxy}-propanoate de méthyle. RMN $^1$H (400 MHz, DMSO-d6) : 1,21 (s, 6 H) ; 2,40 (s, 3 H) ; 2,50 à 2,56 (m, 4 H) ;2,87 (t, J=5,8 Hz, 2 H) ; 3,15 à 3,23 (m, 4 H) ; 3,40 (t, J=5,8 Hz, 2 H) ; 3,47 à 3,52 (m, 12 H) ; 3,59 (s, 3 H) ; 3,62 (t, J=6,2 Hz, 2 H) ; 4,13 (t, J=5,8 Hz, 2 H) ; 4,45 (d, J=5,8 Hz, 4 H) ; 5,31 (t, J=5,8 Hz, 2 H) ; 6,85 (s, 2 H) ; 7,98 (t, J=5,8 Hz, 1 H). LC/MS (A) : tr= 0,35 min ; [M+H]+ : m/z 620 ; [M+2H]2+: m/z 310,5 (pic de base) ; [M-H+$HCO_2$H]-: m/z 664.

**2.5. 3-[2-(2-{2-[2-(2-iodo-acétylamino)-éthoxy]-éthoxy}-éthoxy)-éthoxy]-propanoate** de **méthyle**

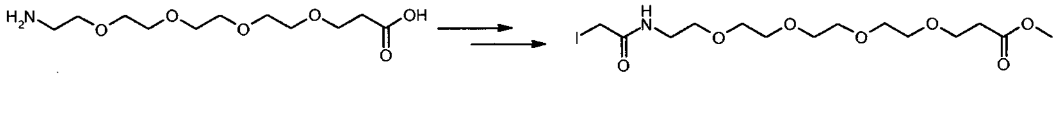

A 100 mg d'acide 3-(2-{2-[2-(2-amino-éthoxy)-éthoxy]-éthoxy}-éthoxy)-propanoïque est additionné 117,4 mg de iodoacétate de N-hydroxysuccinimidyle en solution dans 3 mL de DCM. Après 2 h à TA, 330 µL de MeOH est additionné et le mélange est refroidi à 0°C. On ajoute 360 µL d'une solution 2M de triméthylsilyldiazométhane dans l'hexane. Après 1 h, le mélange est neutralisé par addition de 50 µL d'acide acétique puis additionné d'une solution aqueuse saturée de $NaHCO_3$ jusqu'à l'obtention de pH=8. La phase organique est séchée sur $MgSO_4$, concentrée sous PR et purifié par chromatographie flash sur silice (Interchrom Puriflash Silice 15/35U 10G), en utilisant un gradient de 0 à 10% de méthanol dans le DCM. On obtient ainsi 132 mg de 3-[2-(2-{2-[2-(2-iodo-acétylamino)-éthoxy]-éthoxy}-éthoxy)-éthoxy]-propanoate de méthyle. RMN $^1$H (400 MHz, DMSO-d6) : 2,54 (t, J=6,4 Hz, 2 H) ; 3,20 (q, J=5,8 Hz, 2 H) ; 3,41 (t, J=5,8 Hz, 2 H) ; 3,48 à 3,53 (m, 12 H) ; 3,60 (s, 3 H) ; 3,63 (t, J=6,4 Hz, 2 H) ; 3,65 (s, 2 H) ; 8,27 (t large,

J=5,8 Hz, 1 H). LC/MS (A) : tr= 0,54 min ; [M+H]+ : m/z 448 ; [M+$HCO_2$H-H]- : m/z 492.

**2.6. 4-{2-[(2-mercapto-2-méthyl-propyl)-méthyl-amino]-éthoxy}-2,6-bis-(hydroxyméthyl)-pyridine**

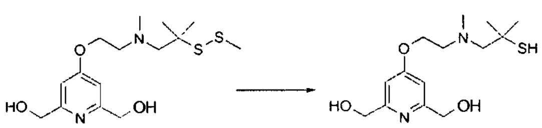

A 80 mg de 4-{2-[méthyl-(2-méthyl-2-méthyldisulfanyl-propyl)-amino]-éthoxy)-2,6-bis-(hydroxyméthyl)-pyridine en solution dans 1,95 mL de méthanol, est additionné une solution de 198 mg de chlorhydrate de tris(2-carboxyéthyl)phosphine dans 730 µL d'eau. Après 2 h à TA, le mélange est concentré sous PR et repris dans 10 mL d'eau. La phase aqueuse est amenée à pH=8 par addition d'une solution aqueuse de soude puis extraite 2x avec de AcOEt. Les phases organiques rassemblées sont lavées avec une solution aqueuse saturée en NaCl et concentrées sous PR. On obtient 68 mg de 4-{2-[(2-mercapto-2-méthyl-propyl)-méthyl-amino]-éthoxy}-2,6-bis-(hydroxyméthyl)-pyridine. RMN $^1$H (400 MHz, DMSO-d6) : 1,28 (s, 6 H) ; 2,43 (s, 3 H) ; 2,54 (s, 2 H) ; 2,62 (s, 1H) ; 2,91 (t, J=5,7 Hz, 2 H) ; 4,15 (t, J=5,7 Hz, 2 H) ; 4,45 (d, J=5,8 Hz, 4 H) ; 5,30 (t, J=5,8 Hz, 2 H) ; 6,85 (s, 2 H). LC/MS (A) : tr= 0,11 min ; [M+H]+ : m/z 301.

**Exemple 3**

**3.1. préparation du conjugué**

On prépare un conjugué comme pour l'ex. 1 en faisant réagir le hu2H11 et le 3-(2-{2-[2-(2-{2-[1-(3,5-bis-[(S)-2-éth-(E)-ylidène-7-diméthoxy-1,2,3,11a-tetrahydro-pyrrolo[2,1c][1,4]benzodiazépin-5-one-8-yloxyméthyl]-1-méthyl-éthylsulfanyl]-acétylamino}-éthoxy)-éthoxy]-éthoxy}-éthoxy)-propanoate de N-hydroxysuccinimidyle.

Le conjugué obtenu est dosé par spectrophotométrie en utilisant les coefficients d'extinction du 1-(1-méthyl-1-méthyldisulfanyl-éthyl)-3,5-bis-[(S)-2-éth-(E)-ylidène-7-diméthoxy-1,2,3,11a-tetrahydro-pyrrolo[2,1c][1,4]benzodiazépin-5-one-8-yloxyméthyl] -benzène ($\varepsilon_{319\ nm}$= 8460 $M^{-1}$ $cm^{-1}$ and $\varepsilon_{280\ nm}$= 10531 $M^{-1}$ $cm^{-1}$) et de hu2H11 ($\varepsilon_{280\ nm}$= 208380 $M^{-1}cm^{-1}$): une moyenne de 4,2 dérivés de tomaymycine par molécule d'anticorps a été déterminée.

**3.2. 3-(2-{2-[2-(2-{2-[1-(3.5-bis-[(S)-2-éth-(E)-ylidène-7-diméthoxy-1,2,3,11a-tetrahydro - pyrrolo[2,1c][1,4]benzodiazépin-5-one-8-yloxyméthyl]-1-méthyl-éthylsulfanyl]-acétylamino}-éthoxy)-éthoxy]-éthoxy}-éthoxy)-propanoate de N-hydroxysuccinimidyle**

Préparé comme pour l'ex.2, à partir de l'acide 3-(2-{2-[2-(2-{2-[1-(3,5-bis-((S)-2-éth-(E)-ylidène-7-diméthoxy-1,2,3,11a-tetrahydro-pyrrolo[2,1c][1,4]benzodiazépin-5-one-8-yloxyméthyl]-1-méthyl-éthylsulfanyl]-acétylamino}-éthoxy)-éthoxy]-éthoxy}-éthoxy)-propanoïque :

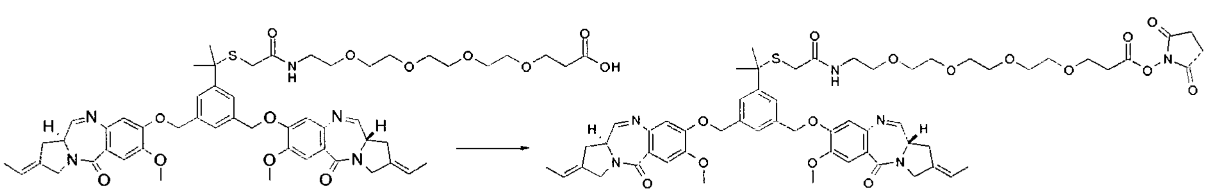

LC/MS (F) : tr= 1,27 min; [M+H]+ : m/z 1123

**3.3. acide 3-(2-{2-[2-(2-{2-[1-(3,5-bis-[(S)-2-éth-(E)-ylidène-7-diméthoxy-1,2,3,11a-tetrahydro-pyrrolo[2,1c][1,4]benzodiazépin-5-one-8-yloxyméthyl]-1-méthyl-éthylsulfanyl]-acétylamino}-éthoxy)-éthoxy]-éthoxy}-éthoxy)-propanoïque**

Préparé comme pour l'ex. 2, à partir du 3-(2-{2-[2-(2-{2-[1-(3,5-bis-[(S)-2-éth-(E)-ylidène-7-diméthoxy-1,2,3,11a-tetrahydro-pyrrolo[2,1c][1,4]benzodiazépin-5-one-8-yloxyméthyl]-1-méthyl-éthylsulfanyl]-acétylamino}-éthoxy)-éthoxy]-éthoxy}-éthoxy)-propanoate de méthyle :

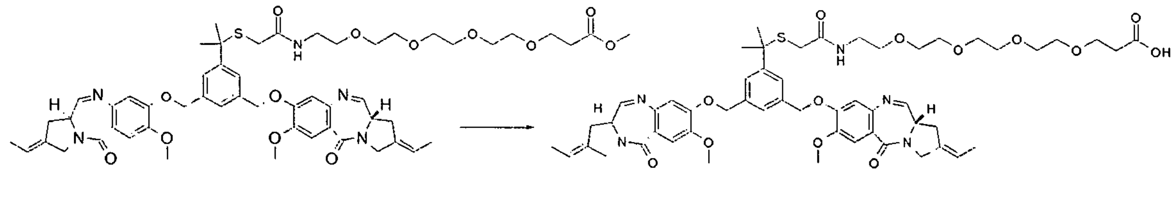

LC/MS (F) : tr= 1,19 min ; [M+H]+ : m/z 1026.

**3.4. 3-(2-{2-[2-(2-{2-[1-(3,5-bis-[(S)-2-éth-(E)-ylidène-7-diméthoxy-1,2,3,11a-tetrahydro-pyrrolo[2,1c][1,4]benzodiazépin-5-one-8-yloxyméthyl]-1-méthyl-éthylsulfanyl]-acétyl amino}-éthoxy)-éthoxy]-éthoxy}-éthoxy)-propanoate de méthyle**

Préparé comme pour l'ex.2, à partir du 3-(2-{2-[2-(2-{2-[1-(3,5-bis-hydroxyméthyl-phenyl)-1-méthyl-éthylsulfanyl]-acétylamino}-éthoxy)-éthoxy]-éthoxy}-éthoxy)-propanoate de méthyle :

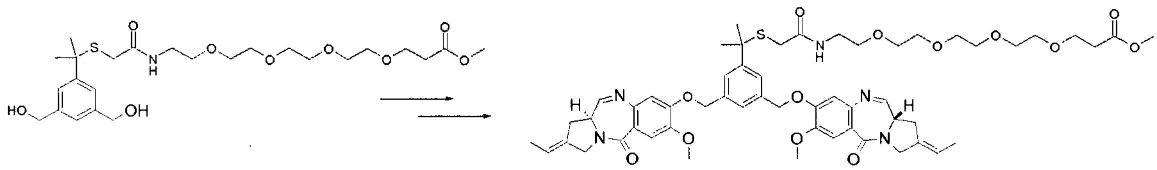

LC/MS (F) : tr=1,28 min ; [M+H]+ : m/z 1040

**3.5. 3-(2-{2-[2-(2-{2-[1-(3,5-bis-hydroxyméthyl-phenyl)-1-méthyl-éthylsulfanyl]-acétylamino}-éthoxy)-éthoxy]-éthoxy}-éthoxy)-propanoate de méthyle**

Préparé comme décrit pour l'ex.2, à partir de 3,5-bis-hydroxyméthyl-1-(1-mercapto-1-méthyl-éthyl)-benzène :

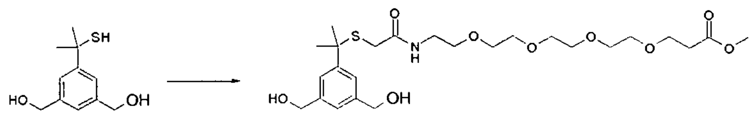

RMN $^1H$ (400 MHz, DMSO-d6) : 1,64 (s, 6 H) ; 2,53 (t, J=6,2 Hz, 2 H) ; 2,93 (s, 2 H); 3,15 (q, J=6,0 Hz, 2 H) ; 3,37 (t, J=6,0 Hz, 2 H) ; 3,49 (m, 12 H) ; 3,59 (s, 3 H) ; 3,62 (t, J=6,2 Hz, 2 H) ; 4,49 (d, J=5,6 Hz, 4 H) ; 5,13 (t, J=5,6 Hz, 2 H) ; 7,14 (s, 1 H) ; 7,32 (s, 2 H) ; 7,84 (t, J=6,0 Hz, 1 H). LC/MS (B) : tr= 2,98 min ; [M+H]+ : m/z 532 ; pic de base : m/z 354 ; $[M-H+HCO_2H]^-$ : m/z 576.

**3.8. 3,5-bis-hydroxyméthyl-1-(1-mercapto-1-méthyl-éthyl)-benzène**

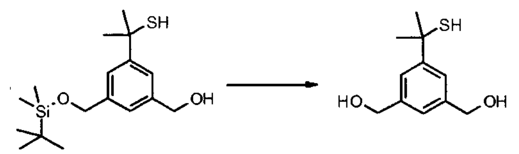

Une solution de 43 mg de 5-hydroxyméthyl-1-(1-mercapto-1-méthyl-éthyl)-3-(tert-butyl-diméthyl-silanyloxy méthyl)-benzène dans 1 mL d'un mélange acide acétique/THF/eau (3/1/1) est agité à TA pendant 4,5 h, concentré sous PR puis repris dans 3 mL d'eau. Le pH de la phase aqueuse est amené à 7 par addition d'une solution aqueuse 10% de $Na_2CO_3$ puis la phase aqueuse est extraite avec AcOEt. La phase organique est séchée sur $MgSO_4$ et concentrée sous PR. On obtient ainsi 18 mg de 3,5-bis-hydroxyméthyl-1-(1-mercapto-1-méthyl-éthyl)-benzène. RMN $^1H$ (400 MHz, DMSO-d6) : 1,76 (s, 6 H) ; 3,19 (s, 1 H) ; 4,48 (d, J=5,9 Hz, 4 H) ; 5,14 (t, J=5,9 Hz, 2 H) ; 7,13 (s, 1 H) ; 7,36 (s, 2 H). MS (C) : CI : $[M+NH_4]^+$ : m/z 230.

**3.9. 5-hydroxyméthyl-1-(1-mercapto-1-méthyl-éthyl)-3-(tert-butyl-diméthyl-silanyloxy méthyl)-benzène**

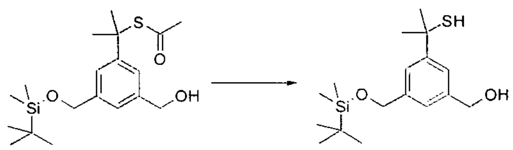

A 336 mg de thioacétate de 1-[3-(tert-butyl-diméthyl-silanyloxyméthyl)-5-hydroxyméthyl-phenyl]-1-méthyl-éthyle en solution dans 3,7 mL d'acétonitrile est additionnée 114 µL d'hydrate d'hydrazine. Après 3 h à TA, le mélange est concentré sous PR et purifié par chromatographie flash sur silice (Biotage 25+M), en utilisant un gradient de 0 à 55% AcOEt dans l'heptane. On obtient ainsi 230 mg de 5-hydroxyméthyl-1-(1-mercapto-1-méthyl-éthyl)-3-(tert-butyl-diméthyl-silanyloxyméthyl)-benzène. RMN $^1$H (400 MHz, DMSO-d6) : 0,08 (s, 6 H) ; 0,92 (s, 9 H) ; 1,76 (s, 6 H) ; 3,19 (s, 1H) ; 4,48 (d, J=5,7 Hz, 2 H) ; 4,71 (s, 2 H) ; 5,14 (t, J=5,7 Hz, 1 H) ; 7,10 (s, 1 H) ; 7,33 à 7,42 (m, 2 H). MS (C) : CI : [M+$NH_4$]+ : m/z 344.

**3.10. thioacétate de 1-[3-(tert-butyl-diméthyl-silanyloxyméthyl)-5-hydroxyméthyl-phenyl]-1-méthyl-éthyle**

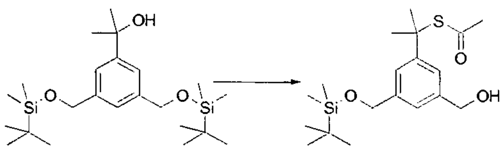

A 1 g de 1-(1-Hydroxy-1-méthyl-éthyl)-3,5-bis-(tert-butyl-diméthyl-silanyloxyméthyl)-benzène en solution dans 4,7 mL de 1,2-dichloroéthane sont additionnés 404 µL d'acide thioacétique et 376 mg d'iodure de zinc. Le mélange est chauffé à 50°C pendant 40 min. Après retour à TA, les sels sont éliminés par filtration et la phase organique est concentrée sous PR et purifié par chromatographie flash sur silice (Biotage 40+M), en utilisant un gradient de 0 à 30% AcOEt dans l'heptane. On obtient ainsi 248 mg de thioacétate de 1-[3-(tert-butyl-diméthyl-silanyloxyméthyl)-5-hydroxyméthyl-phenyl]-1-méthyl-éthyle. RMN $^1$H (400 MHz, DMSO-d6) : 0,08 (s, 6 H) ; 0,91 (s, 9 H) ; 1,79 (s, 6 H) ; 2,17 (s, 3H) ; 4,48 (d, J=5,6 Hz, 2 H) ; 4,70 (s, 2 H) ; 5,16 (t, J=5,6 Hz, 1 H) ; 7,11 (s, 1 H) ; 7,33 (s, 1 H) ; 7,35 (s, 1 H). LC/MS (A) : tr= 1,23 min ; [M+Na]+ : m/z 391.

**3.11. 1-(1-Hydroxy-1-méthyl-éthyl)-3,5-bis-(tert-butyl-diméthyl-silanyloxyméthyl)-benzène**

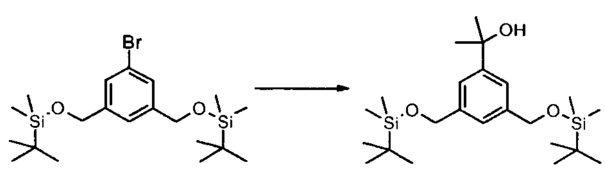

A 4,32 g de 1-bromo-3,5-bis-(tert-butyl-diméthyl-silanyloxyméthyl)-benzène (G.T.Crisp, P.D.Turner Tetrahedron 2000, 56 (42), 8335) en solution dans 150 mL de THF refroidie à -71°C est additionné goutte à goutte 10,5 mL de n-BuLi en solution 1,6 M dans l'hexane. Après 1 h30, 4,27 mL d'acétone est additionné goutte à goutte. Le mélange est laissé remonter à TA puis hydrolysé par une solution aqueuse saturée $NH_4Cl$. La phase aqueuse est extraite avec 100 mL AcOEt et les phases organiques rassemblées sont séchées sur $MgSO_4$, concentrées sous PR et purifiées par chromatographie flash sur silice (Biotage 65+M), en utilisant un gradient de 0 à 20% AcOEt dans l'heptane. On obtient ainsi 2,48 g de 1-(1-hydroxy-1-méthyl-éthyl)-3,5-bis-(tert-butyl-diméthyl-silanyloxyméthyl)-benzène. RMN $^1$H (400 MHz, CHLOROFORM-d) : 0,11 (s, 12 H) ; 0,96 (s, 18 H) ; 1,59 (s, 6H) ; 1,69 (s, 1 H) ; 4,76 (s, 4 H) ; 7,21 (s, 1 H) ; 7,33 (s, 2 H). LC/MS (B) : tr=6,43 min ; [M+Na]+ : m/z 447 ; pic de base : m/z 407.

**Exemple 4**

**4.1. Préparation du conjugué.**

On prépare comme pour l'ex.1 un conjugué en faisant réagir le hu2H11 et le 3-{2-[2-(2-{2-[2-(4-{4-[2-(2,6-bis-[(S)-2-éth-(E)-ylidène-7-diméthoxy-1,2,3,11a-tetrahydro-pyrrolo[2,1 c][1,4]benzodiazépin-5-one-8-yloxyméthyl]-pyridin-4-yloxy)-éthyl]-pipérazin-1-yl}-1,1-diméthyl-4-oxo-butylsulfanyl)-acétylamino]-éthoxy}-éthoxy)-éthoxy]-éthoxy}-propanoate de N-hydroxysuccinimidyle. Le conjugué obtenu est dosé par spectrophotométrie en utilisant les coefficients d'extinction $\varepsilon_{320nm}$= 7876 $M^{-1}cm^{-1}$ et $\varepsilon_{280nm}$= 4334 $M^{-1}cm^{-1}$ pour le dimère de tomaymycine et $\varepsilon_{280nm}$= 208,380 $M^{-1}cm^{-1}$ pour hu2H11 : une moyenne de 3,13 dimères de tomaymycine par molécule d'anticorps a été déterminée.

**4.2. 3-{2-[2-(2-{2-[2-(4-{4-[2-(2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridin-4-yloxy)-éthyl]-pipérazin-1-yl}-1,1-diméthyl-4-oxo-butylsulfanyl)-acétylamino]-éthoxy}-éthoxy)-éthoxy]-éthoxy}-propanoate de N-hydroxysuccinimidyle** :

Préparé selon l'exemple 2, à partir de l'acide 3-{2-[2-(2-{2-[2-(4-{4-[2-(2,6-bis-[(S)-2-éth-(E)-ylidène-7-diméthoxy-1,2,3,11a-tetrahydro-pyrrolo[2,1c][1,4]benzo diazépin-5-one-8-yloxyméthyl]-pyridin-4-yloxy)-éthyl]-pipérazin-1-yl}-1,1-diméthyl-4-oxo-butylsulfanyl)-acétylamino]-éthoxy}-éthoxy)-éthoxy]-éthoxy}-propanoïque

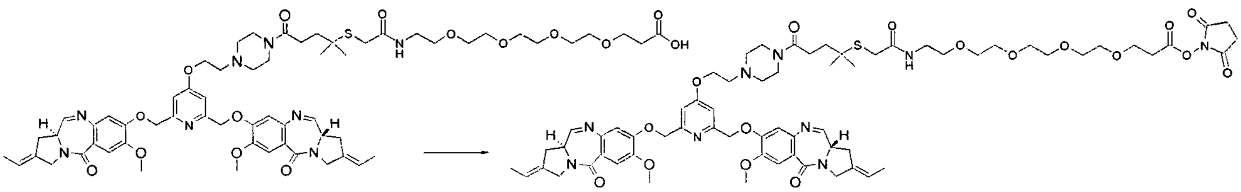

LC/MS (B) : tr= 3,27 min ; $[M+H]^+$ : m/z 1309 ; $[M+2H]^{2+}$ : m/z 655.

**4.3. acide 3-{2-[2-(2-{2-[2-(4-{4-[2-(2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridin-4-yloxy)-éthyl]-pipérazin-1-yl}-1,1-diméthyl-4-oxo-butylsulfanyl)-acétylamino]-éthoxy}-éthoxy)-éthoxy]-éthoxy}-propanoïque**

Préparé selon l'exemple 2, à partir de 3-{2-[2-(2-{2-[2-(4-{4-[2-(2,6-bis-[(S)-2-éth-(E)-ylidène-7-diméthoxy-1,2,3,11a-tetra hydro-pyrrolo[2,1c][1,4]benzodiazépin-5-one-8-yloxyméthyl]-pyridin-4-yloxy)-éthyl]-pipérazin-1-yl}-1,1-diméthyl-4-oxo-butyl sulfanyl)-acétylamino]-éthoxy}-éthoxy)-éthoxy]-éthoxy}-propanoate de méthyle

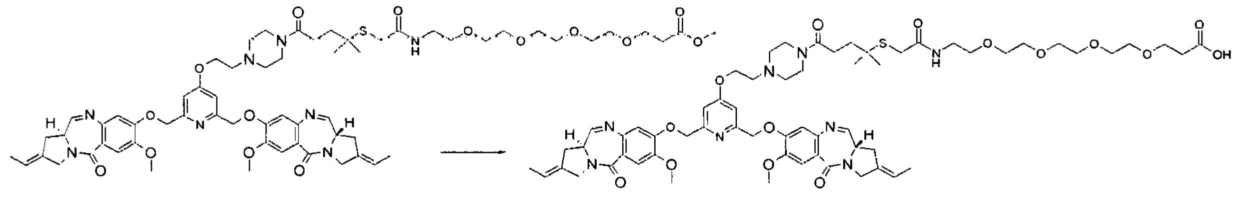

LC/MS (B) : tr= 3,16 min ; $[M+H]^+$ : m/z 1212 ; $[M+2H]^{2+}$ : m/z 606.

**4.4. 3-{2-[2-(2-{2-[2-(4-{4-[2-(2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridin-4-yloxy)-éthyl]-pipérazin-1-yl}-1,1-diméthyl-4-oxo-butylsulfanyl)-acétylamino]-éthoxy}-éthoxy)-éthoxy]-éthoxy}-propanoate** de **méthyle**

Préparé selon l'exemple 2, à partir de 3-{2-[2-(2-{2-[2-(4-{4-[2-(2,6-bis-hydroxyméthyl-pyridin-4-yloxy)-éthyl]-pipérazin-1-yl}-1,1-diméthyl-4-oxo-butyl sulfanyl)-acétylamino]-éthoxy}-éthoxy)-éthoxy]-éthoxy}-propanoate de méthyle

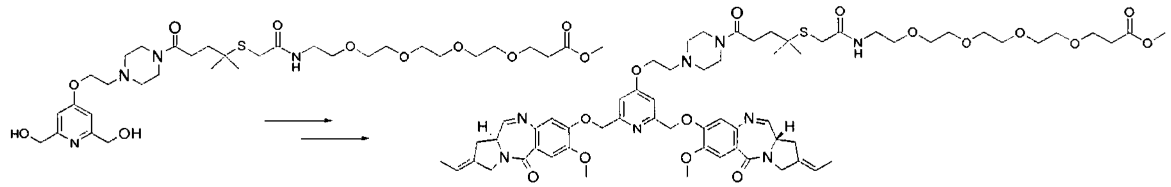

RMN $^1H$ (500 MHz, DMSO-d6) : 1,22 (s, 6 H) ; 1,69 (m, 8 H) ; 2,37 (m, 2 H) ; 2,40 (m, 2 H) ; 2,48 (m, 2 H) ; 2,53 (t, *J*=6,1 Hz, 2 H) ; 2,72 (m, 2 H) ; 2,92 (m, 2 H) ; 3,04 (m, 2 H) ; 3,11 (s, 2 H) ; 3,19 (q, *J*=5,4 Hz, 2 H) ; 3,39 (t, *J*=5,4 Hz, 2 H) ; 3,42 (m, 4 H) ; 3,49 (m, 12 H) ; 3,59 (s, 3 H) ; 3,62 (t, *J*=6,1 Hz, 2 H) ; 3,86 (s, 6 H) ; 3,88 (m, 2 H) ; 4,10 (m, 4 H) ; 4,22 (m, 2 H) ; 5,17 (d, *J*=13,0 Hz, 2 H) ; 5,22 (d, J=13,0 Hz, 2 H) ; 5,55 (m, 2 H) ; 6,94 (s, 2 H) ; 7,09 (s, 2 H) ; 7,38 (s, 2 H) ; 7,77 (d, *J*=3,9 Hz, 2 H) ; 8,01 (t, *J*=5,4 Hz, 1 H). LC/MS (B) : tr = 3,30 min ; $[M+H]^+$ : m/z 1225 ; $[M+2H]^{2+}$ : m/z 613 (pic de base).

**4.5. 3-{2-[2-(2-{2-[2-(4-{4-[2-(2,6-bis-hydroxyméthyl-pyridin-4-yloxy)-éthyl]-pipérazin-1-yl}-1,1-diméthyl-4-oxo-butylsulfanyl)-acétylamino]-éthoxy}-éthoxy)-éthoxy]-éthoxy}-propanoate** de **méthyle**

Préparé selon l'exemple 2, à partir de 4-(2-[4-(4-mercapto-4-méthyl)-pentanoyl)-pipérazin-1-yl]-éthoxy)-2,6-bis-(hydroxyméthyl)-pyridine

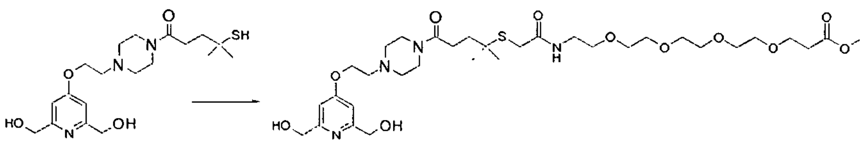

RMN $^1H$ (500 MHz, DMSO-d6): tous les signaux sont larges avec 1,22 (s, 6 H) ; 1,71 (m, 2 H) ; 2,20 à 2,58 (m partiellement masqué, 8 H) ; 2,75 (m, 2 H) ; 3,12 (m, 2 H) ; 3,19 (m, 2 H) ; 3,47 à 3,53 (m, 18 H) ; 3,60 (m, 5 H) ; 4,18 (m, 2 H) ; 4,46 (m, 4 H) ; 5,31 (m, 2 H) ; 6,86 (s, 2 H) ; 8,00 (m, 1 H). LC/MS (B) : tr= 2,20 min ; $[M+H]^+$: m/z 717 ; $[M-H+HCO_2H]^-$ : m/z 761.

**4.6. 4-(2-[4-(4-mercapto-4-méthyl)-pentanoyl)-pipérazin-1-yl]-éthoxy)-2,6-bis-(hydroxyméthyl)-pyridine**

Préparé selon l'exemple 2, à partir de 4-(2-[4-(4-méthyl-4-méthyldisulfanyl)-pentanoyl)-pipérazin-1-yl]-éthoxy)-2,6-bis-(hydroxyméthyl)-pyridine

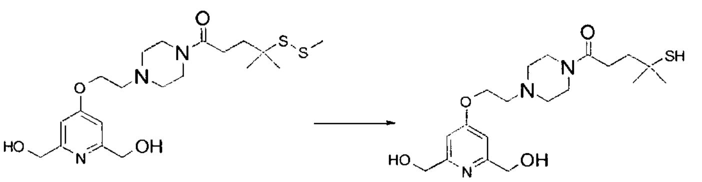

RMN $^1H$ (400 MHz, DMSO-d6) : 1,32 (s, 6 H) ; 1,76 (m, 2 H) ; 2,36 à 2,53 (m partiellement masqué, 6 H) ; 2,66 (s, 1 H) ; 2,75 (t, *J*=5,5 Hz, 2 H) ; 3,40 à 3,52 (m, 4 H) ; 4,18 (t, *J*=5,5 Hz, 2 H) ; 4,46 (s, 4 H) ; 5,30 (m très étalé, 2 H) ; 6,86 (s, 2 H). LC/MS (B) : tr = 0,76 min ; $[M+H]^+$ : m/z 398 ; $[M-H]^-$ : m/z 396.

**4.7. 4-(2-[4-(4-méthyl-4-méthyldisulfanyl)-pentanoyl)-pipérazin-1-yl]-éthoxy)-2,6-bis-(hydroxyméthyl)-pyridine**

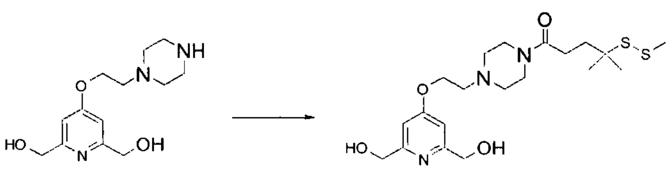

A 600 mg de 4-(2-pipérazin-1-yl-éthoxy)-2,6-bis-(hydroxyméthyl)-pyridine en solution dans 12 mL de DM F sont additionnés 344 μL de TEA puis, après 10 min d'agitation, 748 mg d'acide 4-méthyl-4-méthyldisulfanyl-pentanoïque, 417 μL de diisopropylcarbodiimide et 69 mg d'hydrate de 1-hydoxybenzotriazole. Après 15 h à TA, le mélange est concentré sous PR, additionné de 15 mL d'eau et extrait 2x avec AcOEt. Les phases organiques rassemblées sont séchées sur $MgSO_4$, concentrées sous PR et purifiées par chromatographie flash sur silice (Analogix Super Flash $SiO_2$ SF25-80g) en utilisant un gradient de 2 à 10% de MeOH dans DCM. On obtient ainsi 390 mg de 4-(2-[4-(4-méthyl-4-méthyldisulfanyl)-pentanoyl)-pipérazin-1-yl]-éthoxy)-2,6-bis-(hydroxyméthyl-pyridine. RMN $^1H$ (400 MHz, DMSO-d6) : 1,26 (s, 6 H) ; 1,79 (m, 2 H) ; 2,36 (m, 2 H) ; 2,39 (s, 3 H) ; 2,40 à 253 (m partiellement masqué, 4 H) ; 2,75 (t, J=5,6 Hz, 2 H) ; 3,45 (m, 4 H) ; 4,18 (t, J=5,6 Hz, 2 H) ; 4,46 (d, J=5,9 Hz, 4 H) ; 5,30 (t, J=5,9 Hz, 2 H) ; 6,86 (s, 2 H). LC/MS (A) : tr = 0,42 min ; $[M+H]^+$ : m/z 444 ; $[M-H+HCO_2H]^-$ : m/z 488.

**4.8. 4-(2-pipérazin-1-yl-éthoxy)-2,6-bis-(hydroxyméthyl)-pyridine**

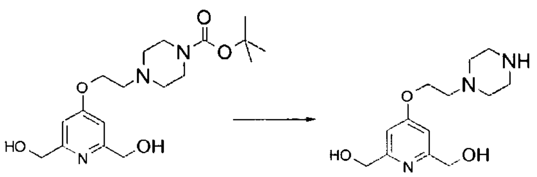

A 2,3 g de 4-(2-(4-tert-butoxycarbonyl-pipérazin-1-yl)-éthoxy)-2,6-bis-(hydroxyméthyl) -pyridine en solution dans 33 mL de dioxanne sont additionnés 19 mL d'une solution HCl 4M dans le dioxane. Après 12 h à TA, le précipité résultant est récupéré par filtration sur verre fritté, repris dans MeOH puis concentré sous PR et dilué dans 40 mL d'un mélange MeOH / eau 1/1. La solution résultante est déposée sur Mega BE-SCX, 25GM 150ML (Varian). Après lavage de la phase avec du MeOH, le produit d'intérêt est élué avec une solution d'ammoniac 2N dans le méthanol. La phase MeOH / $NH_3$ est concentrée sous PR. On obtient ainsi 1,6 g de 4-(2-pipérazin-1-yl-éthoxy)-2,6-bis-(hydroxyméthyl)-pyridine. RMN $^1H$ (400 MHz, DMSO-d6) : 2,40 (m, 4 H) ; 2,68 (m, 6 H) ; 4,15 (t, J=5,7 Hz, 2H) ; 4,44 (m, 4 H) ; 5,29 (m, 2 H) ; 6,85 (s, 2 H). LC/MS (A) : tr= 0,10 min ; [M+H]+ : m/z 268 ; [M+2H]2+ : m/z 134,5 ; pic de base : m/z 113.

**4.9. 4-(2-(4-tert-butoxycarbonyl-pipérazin-1-yl)-éthoxy)-2,6-bis-(hydroxyméthyl)-pyridine**

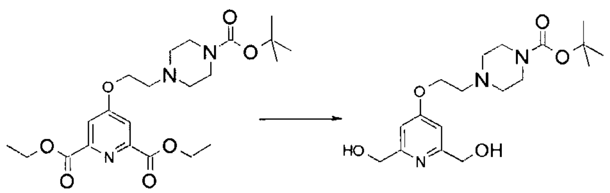

A 3,1 g de 4-[2-(4-tert-butoxycarbonyl-pipérazin-1-yl)-éthoxy]-pyridine-2,6-dicarboxylate de diéthyle en solution dans 105 mL d'EtOH, sont additionnés 779 mg de borohydrure de sodium et 2,29 g de $CaCl_2$. Après 3 h à TA, le mélange est hydrolysé et concentré sous PR. Le résidu obtenu est additionné d'eau et la phase aqueuse résultante est extraite 4x avec AcOEt. Les phases organiques rassemblées sont lavées avec une solution aqueuse saturée en NaCl puis concentrées sous PR. On obtient ainsi 2,4 g de 4-(2-(4-tert-butoxycarbonyl-pipérazin-1-yl)-éthoxy)-2,6-bis-(hydroxyméthyl)-pyridine. RMN $^1H$ (400 MHz, DMSO-d6) : 1,39 (s, 9 H) ; 2,43 (m, 4 H) ; 2,73 (t, J=5,6 Hz, 2 H); 3,30 (m partiellement masqué, 4 H) ; 4,17 (t, J=5,6 Hz, 2 H) ; 4,45 (d, J=5,9 Hz, 4 H) ; 5,30 (t, J=5,9 Hz, 2H) ; 6,86 (s, 2 H). LC/MS (A) : tr = 0,24 min ; $[M+H]^+$ : m/z 368.

**4.10. 4-[2-(4-tert-butoxycarbonyl-pipérazin-1-yl)-éthoxy]-pyridine-2,6-dicarboxylate** de **diéthyle**

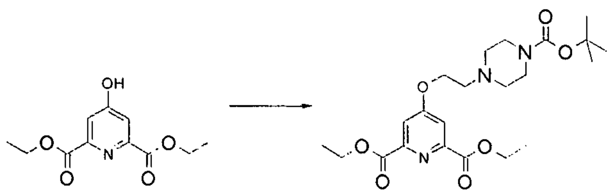

A une solution de 5 g de 1-tert-butoxycarbonyl-4-(2-hydroxyéthyl)-pipérazine dans 102 mL de DCM refroidie à 0°C sont additionnés 4,5 mL de TEA puis 2 mL de chlorure de méthanesulfonyle. Après 1 h, le mélange est ramené à TA. Après 1 h d'agitation supplémentaire, le mélange est hydrolysé et la phase organique lavée 2x à l'eau puis séchée sur $MgSO_4$ et concentrée sous PR. Le résidu ainsi obtenu (6,7 g) est additionné de 140 mL de DMF et porté à 60°C. 190 mg de diester d'éthyle de l'acide chélidamique (Scrimin, P. ; Tecilla, P.; Tonellato, U.; Vendrame, T. J. Org. Chem. 1989, 54, 5988) et 549 mg de $K_2CO_3$ sont alors ajoutés et le mélange est chauffé à 80°C pendant 20 h. Après concentration sous PR, le mélange est hydrolysé, extrait 3x avec AcOEt et les phases organiques réunies sont lavées avec une solution saturée en NaCl, concentrées sous PR et purifiées par chromatographie flash sur silice (Analogix Super Flash $SiO_2$ SF25-150g) en utilisant un gradient de 60 à 85% d'AcOEt dans l'heptane. On obtient ainsi 3,1 g de 4-[2-(4-tert-butoxycarbonyl-pipérazin-1-yl)-éthoxy]-pyridine-2,6-dicarboxylate de diéthyle. RMN $^1H$ (400 MHz, DMSO-d6) : 1,34 (t, J=7,2 Hz, 6 H) ; 1,39 (s, 9 H) ; 2,44 (m, 4 H) ; 2,76 (t, J=5,7 Hz, 2 H) ; 3,30 (m partiellement masqué, 4 H) ; 4,34 (t, J=5,7 Hz, 2 H) ; 4,38 (q, J=7,1 Hz, 4 H) ; 7,74 (s, 2 H). LC/MS (B) : tr= 2,81 min ; $[M+H]^+$ : m/z 452 ; $[MH+HCO_2H]^-$ : m/z 496.

**Exemple 5**

**5.1. 4-{2-[4-(2-méthyl-2-méthyldisulfanyl-propyl)-pipérazin-1-yl]-éthoxy}-2,6-bis-(hydroxyméthyl)-pyridine**

Préparé selon l'exemple 1, à partir de la 4-(2-pipérazin-1-yl-éthoxy)-2,6-bis-(hydroxyméthyl)-pyridine.

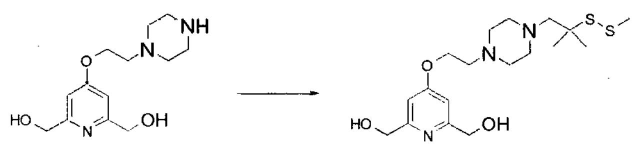

RMN $^1H$ (400 MHz, DMSO-d6) : 1,25 (s, 6 H) ; 2,39 (s, 3 H) ; 2,40 (s, 2 H) ; 2,44 à 2,57 (m partiellement masqué, 8 H) ; 2,69 (t, J=5,7 Hz, 2 H) ; 4,14 (t, J=5,7 Hz, 2 H) ; 4,45 (d, J=5,7 Hz, 4 H) ; 5,29 (t, J=5,7 Hz, 2 H) ; 6,85 (s, 2 H). LC/MS (B) : tr= 0,51 min ; [M+H]+ : m/z 402.

**Exemple 6**

**6.1. Préparation du conjugué.**

On prépare comme pour l'ex.1 un conjugué en faisant réagir le hu2H11 et le 3-{2-[2-(2-{2-[2-(2-{[2-(2,6-bis-[(S)-2-éth-(E)-ylidène-7-diméthoxy-1,2,3,11a-tetrahydro-pyrrolo[2,1 c][1,4]benzodiazépin-5-one-8-yloxyméthyl]-pyridin-4-yloxy)-éthyl]-méthyl-amino}-1,1-diméthyl-éthylsulfanyl)-acétyl-méthylamino]-éthoxy}-éthoxy)-éthoxy]-éthoxy}-propanoate de N-hydroxy succinimidyle. Le conjugué obtenu est dosé par spectrophotométrie en utilisant les coefficients d'extinction $\varepsilon_{319nm}$= 7789 $M^{-1}cm^{-1}$ et $\varepsilon_{280nm}$= 4362 $M^{-1}cm^{-1}$ pour le dimère de tomaymycine et $\varepsilon_{280nm}$= 208380 $M^{-1}cm^{-1}$ pour hu2H11 : une moyenne de 2,90 dimères de tomaymycine par molécule d'anticorps a été déterminée.

**6.2. 3-{2-[2-(2-{2-[2-(2-{[2-(2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridin-4-yloxy)-éthyl]-méthylamino}-1,1-diméthyl-éthylsulfanyl)-acétyl-méthylamino]-éthoxy}-éthoxy)-éthoxy]-éthoxy}-propanoate de N-hydroxysuccinimidyle**

Préparé selon l'exemple 2, à partir de l'acide 3-{2-[2-(2-{2-[2-(2-{[2-(2,6-bis-[(S)-2-éth-(E)-ylidène-7-diméthoxy-1,2,3,11a-tetrahydropyrrolo[2,1c][1,4]benzodiazépin-5-one-8-yloxyméthyl]-pyridin-4-yloxy)-éthyl]-méthyl-amino}-1,1-diméthyl-éthylsulfanyl)-acétyl-méthylamino]-éthoxy}-éthoxy)-éthoxy]-éthoxy}-propanoïque :

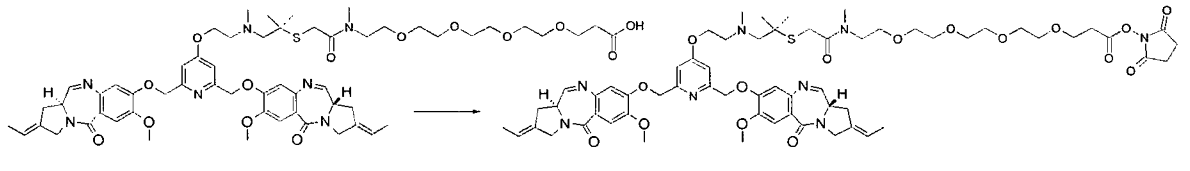

LC/MS (F) : tr= 1,02 min ; [M+H]+ : m/z 1225 ; [M+$H_2$O+H]+ : m/z 1243

**6.3. Acide 3-{2-[2-(2-{2-[2-(2-{[2-(2,6-bis-[(S)-2-éth-(E)-ylidène-7-diméthoxy-1,2,3,11a-tetrahydro-pyrrolo[2,1c][1,4]benzodiazépin-5-one-8-yloxyméthyl]-pyridin-4-yloxy)-éthyl]-méthyl-amino}-1,1-diméthyl-éthylsulfanyl)-acétyl-méthylamino]-éthoxy}-éthoxy)-éthoxy]-éthoxy}-propanoïque**

Préparé selon l'ex. 2 à partir de 3-{2-[2-(2-{2-[2-(2-{[2-(2,6-bis-[(S)-2-éth-(E)-ylidène-7-diméthoxy-1,2,3,11 a-tetrahydro-pyrrolo[2,1c][1,4]benzodiazépin-5-one-8-yloxyméthyl]-pyridin-4-yloxy)-éthyl]-méthyl-amino}-1,1-diméthyl-éthylsulfanyl)-acétyl-méthylamino]-éthoxy}-éthoxy)-éthoxy]-éthoxy}-propanoate de méthyle:

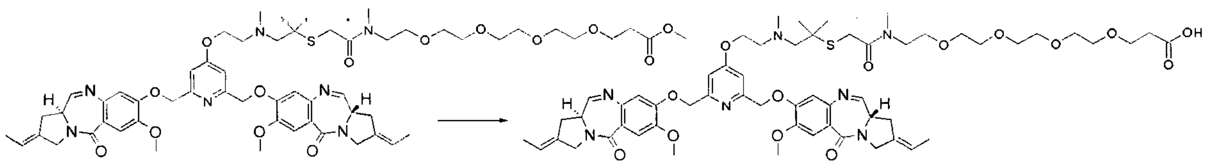

LC/MS (F) : tr= 0,97 min ; [M+H]+ : m/z 1129

**6.4. 3-{2-[2-(2-{2-[2-(2-{[2-(2,6-bis-[(S)-2-éth-(E)-ylidène-7-diméthoxy-1,2,3,11a-tetra hydropyrrolo[2,1c][1,4]benzodiazépin-5-one-8-yloxyméthyl]-pyridin-4-yloxy)-éthyl]-méthyl-amino}-1,1-diméthyl-éthylsulfanyl)-acétyl-méthylamino]-éthoxy}-éthoxy)-éthoxy]-éthoxy}-propanoate de méthyle**

Préparé selon l'ex. 2, à partir du 3-{2-[2-(2-{2-[2-(2-{[2-(2,6-bis-hydroxyméthyl-pyridin-4-yloxy)-éthyl]-méthyl-amino}-1,1-diméthyl-éthylsulfanyl)-acétyl-méthylamino]-éthoxy}-éthoxy)-éthoxy]-éthoxy}-propanoate de méthyle :

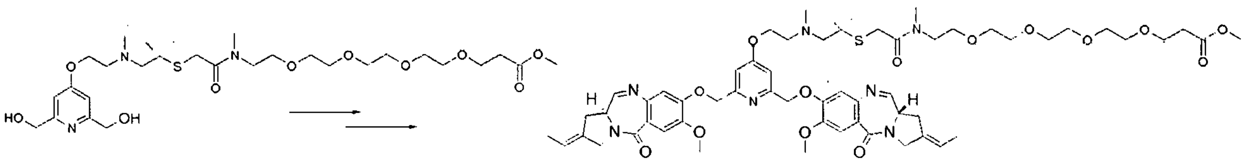

RMN $^1$H (500 MHz, DMSO-d6) : les absorptions sont larges avec 1,20 (s, 6 H) ; 1,58 à 1,73 (m, 6 H) ; 2,38 (s, 3 H) ; 2,52 à 3,07 (m, 13 H) ; 3,36 à 3,63 (m, 23 H) ; 3,67 à 3,91 (m, 8 H) ; 4,06 à 4,26 (m, 6 H) ; 5,02 à 5,26 (m, 4 H) ; 5,33 à 5,60 (m, 2 H) ; 6,39 à 7,42 (m, 6 H) ; 7,76 (m, 2 H). LC/MS (A) : tr = 0,77 min ; $[M+H]^+$ : m/z 1142 ; $[M+2H]^{2+}$ : m/z 571,5 (pic de base).

**6.5. 3-{2-[2-(2-{2-[2-(2-{[2-(2,6-bis-hydroxyméthyl-pyridin-4-yloxy)-éthyl]-méthyl-amino}-1,1-diméthyl-éthylsulfanyl)-acétyl-méthylamino]-éthoxy}-éthoxy)-éthoxy]-éthoxy}-propanoate de méthyle**

Préparé selon l'ex. 2, à partir du 3-{2-[2-(2-{2-[(2-iodo-acétyl)-méthyl amino]-éthoxy}-éthoxy)-éthoxy]-éthoxy}-propanoate de méthyle.

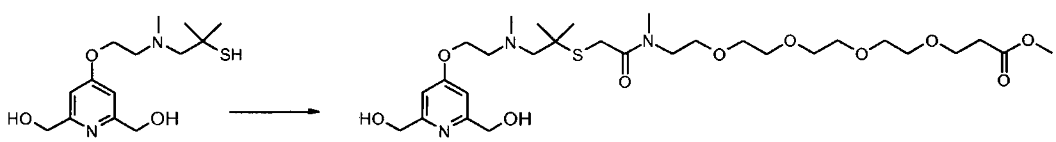

RMN $^1$H (400 MHz, DMSO-d6) : dédoublement 50-50 de rotamères avec : 1,22 (s, 3H) ; 1,24 (s, 3 H) ; 2,41 (s, 3 H) ; 2,53 (t, J=6,2 Hz, 2 H) ; 2,56 (m, 2 H) ; 2,80 (s, 1 H) ; 2,88 (t, J=5,9 Hz, 2 H) ; 3,05 (s, 2 H) ; 3,35 à 3,58 (m, 18 H) ; 3,59

(s, 3 H) ; 3,62 (t, J=6,2 Hz, 2 H) ; 4,13 (t, J=5,9 Hz, 2 H) ; 4,45 (d, J=5,9 Hz, 4 H) ; 5,29 (t, J=5,9 Hz, 2 H) ; 6,85 (s, 2 H). LC/MS (B) : tr= 2,18 min ; $[M+H]^+$ : m/z 634 ; $[M-H+HCO_2H]^-$ : m/z 678.

6.6. 3-{2-[2-(2-{2-[(2-iodo-acétyl)-méthyl-amino]-éthoxy}-éthoxy)-éthoxy]-éthoxy}-propanoate de méthyle

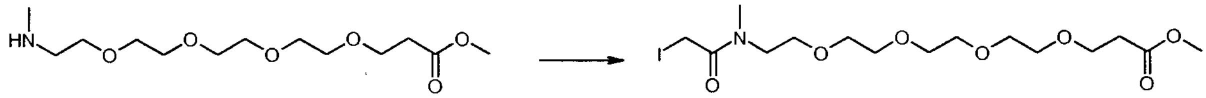

A 215 mg de 3-(2-{2-[2-(2-méthylamino-éthoxy)-éthoxy]-éthoxy}-éthoxy)-propanoate de méthyle est additionné 117,4 mg de iodoacétate de N-hyroxysuccinimidyle en solution dans 6,5 mL de DCM. Après 2 h à TA, le mélange est concentré sous PR et purifié par chromatographie flash sur silice (Analogix Super Flash $SiO_2$ SF25-24g), en utilisant un gradient de 0 à 6% de MeOH dans le DCM. On obtient ainsi 210 mg de 3-{2-[2-(2-{2-[(2-iodo-acétyl)-méthyl-amino]-éthoxy}-éthoxy)-éthoxy]-éthoxy}-propanoate de méthyle. RMN $^1H$ (400 MHz, DMSO-d6) : dédoublement 55-45 de rotamères avec 2,54 (t, J=6,2 Hz, 2 H) ; 2,83 (s, 1,35 H) ; 3,02 (s, 1,65 H) ; 3,36 à 3,55 (m, 16 H) ; 3,60 (s, 3 H) ; 3,63 (t, J=6,2 Hz, 2 H) ; 3,83 (s, 1,1 H) ; 3,88 (s, 0,9 H). LC/MS (A) : tr= 0,62 min ; [M+H]+ : m/z 462.

6.7. 3-(2-{2-[2-(2-méthylamino-éthoxy)-éthoxy]-éthoxy}-éthoxy)-propanoate de méthyle :

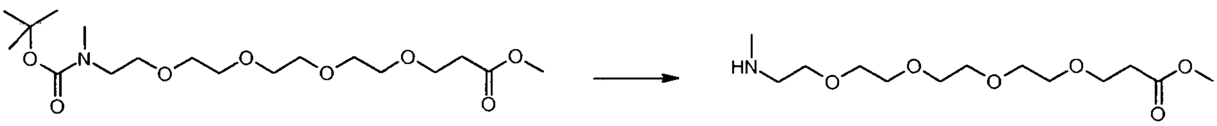

A 390 mg de 3-[2-(2-{2-[2-(tert-butoxycarbonyl-méthyl-amino)-éthoxy]-éthoxy}-éthoxy)-éthoxy]-propanoate de méthyle en solution dans 5,3 mL de dioxanne sont additionnés 3 mL d'une solution HCl 4M dans le dioxane. Après 12 h à TA, le mélange est concentré sous PR mis en solution dans un minimum de méthanol et déposé sur Mega BE-SCX, 10GM 60ML (Varian). Après lavage de la phase avec MeOH, le produit d'intérêt est élué avec une solution d'ammoniac 2N dans le méthanol. La phase méthanol / $NH_3$ est concentrée sous PR. On obtient ainsi 270 mg de 3-(2-{2-[2-(2-méthylamino-éthoxy)-éthoxy]-éthoxy}-éthoxy)-propanoate de méthyle. RMN $^1H$ (400 MHz, DMSO-d6) : 2,28 (s, 3 H) ; 2,54 (t, J=6,2 Hz, 2 H) ; 2,59 (t, J=5,9 Hz, 2 H) ; 3,44 (t, J=5,9 Hz, 2 H) ; 3,47 à 3,54 (m, 12 H) ; 3,60 (s, 3 H) ; 3,63 (t, J=6,2 Hz, 2 H). LC/MS (A) (ELSD) : tr= 0,30 min ; $[M+H]^+$ : m/z 294.

6.8. 3-[2-(2-{2-[2-(tert-butoxycarbonyl-méthyl-amino)-éthoxy]-éthoxy}-éthoxy)-éthoxy]-propanoate de méthyle

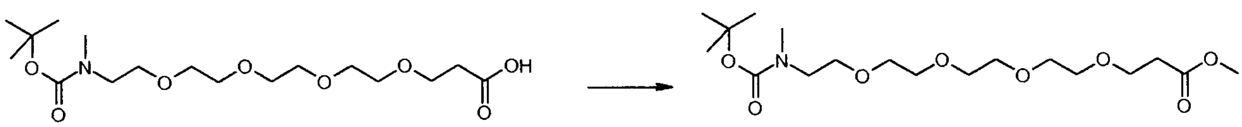

A une solution de 500 mg d'acide 3-[2-(2-{2-[2-(tert-butoxycarbonyl-méthyl-amino)-éthoxy]-éthoxy}-éthoxy)-éthoxy]-propanoïque dans 4,8 mL de MeOH refroidie à 0°C, sont additionnés 2 mL d'une solution 2M de triméthylsilyldiazométhane dans l'hexane. Après 2 h, le mélange est neutralisé par addition de 120 µL d'acide acétique puis concentré sous PR et purifié par chromatographie flash sur silice (Analogix Super Flash $SiO_2$ SF25-40g), en utilisant un gradient de 0 à 5% de méthanol dans le DCM. On obtient ainsi 400 mg de 3-[2-(2-{2-[2-(tert-butoxycarbonyl-méthyl-amino)-éthoxy]-éthoxy}-éthoxy)-éthoxy]-propanoate de méthyle. RMN $^1H$ (400 MHz, DMSO-d6) : 1,38 (s, 9 H) ; 2,54 (t, J=6,2 Hz, 2 H) ; 2,80 (s large,3 H) ; 3,29 (t partiellement marqué, *J*=5,9 Hz, 2 H) ; 3,45 à 3,55 (m, 14 H) ; 3,59 (s, 3 H) ; 3,63 (t, J=6,2 Hz, 2 H). LC/MS (A) : tr= 0,84 min ; $[M+Na]^+$ : m/z 416 (pic de base) ; LC/MS (A) : tr = 0,84 min ; $[M+Na]^+$ : m/z 416 (pic de base).

**6.9. Acide 3-[2-(2-{2-{2-(tert-butoxycarbonyl-méthyl-amino)-éthoxy]-éthoxy}-éthoxy)-éthoxy]-propanoïque**

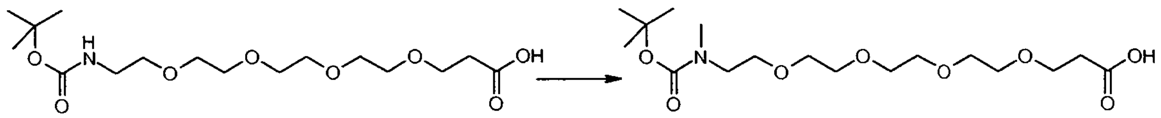

A une solution de 520 mg d' acide 3-[2-(2-{2-[2-(tert-butoxycarbonyl-amino)-éthoxy]-éthoxy}-éthoxy)-éthoxy]-propanoïque dans 14 mL de THF refroidie à 0°C, sont additionnés par portions 85,4 mg de NaH. Après 5 min d'agitation, sont additionnées 150 μL d'iodométhane. Le mélange est alors agité 2 h à TA puis additionné de 150 μL d'iodométhane supplémentaires. Après 12 h à TA, le mélange est hydrolysé puis amené à pH acide par ajout d'acide acétique aqueux à 0°C. La phase aqueuse est extraite 3x avec AcOEt, les phases organiques rassemblées sont séchées sur $MgSO_4$, concentrées sous PR et le brut obtenu est remis en réaction selon le même protocole avec 85 mg de NaH et 176 μL d'iodométhane pour 2 h supplémentaires. On obtient ainsi 500 mg d' acide 3-[2-(2-{2-[2-(tert-butoxycarbonyl-méthyl-amino)-éthoxy]-éthoxy}-éthoxy)-éthoxy]-propanoïque. LC/MS (F) : tr= 1,01 min ; [M+H]+ : m/z 380.

**Evaluation de l'inhibition de la prolifération des lignées cellulaires MDA-MB-231, MDA-A1 et HCT116 par les composés de formule (IA) avec $Z_bR_b$= -OMe** et Y=Y'= **-OMe**

Les cellules MDA-MB-231, MDA-A1 ou HCT116 dans leur phase de croissance exponentielle sont trypsinées et remises en suspension dans leur milieu de culture respectif (DMEM/F12 Gibco #21331, 10% SVF Gibco #10500-056, 2 nM Glutamine Gibco #25030 pour les cellules MDA; DMEM Gibco #11960, 10% SVF Gibco #10500-056, 2 mM Glutamine Gibco #25030 pour les cellules HCT116). La suspension cellulaire est ensemencée dans des plaques de culture 96 puits Cytostar (GE Healthcare Europe, #RPNQ0163) dans le milieu de culture complet contenant du sérum à une densité de 5000 cellules/puits (MDA-MB-231, MDA-A1, HCT116). Après 4 h d'incubation, des dilutions successives des dimères de tomaymycine sont ajoutées dans les puits en triplicate pour chaque concentration. Les cellules sont cultivées 3 jours à 37°C dans une atmosphère à 5% de $CO_2$ en présence des cytotoxiques. Le 4$^{e}$ jour, 10 μl d'une solution de thymidine $^{14}C$ (0,1 μCi/puits, Perkin Elmer #NEC56825000) sont ajoutés dans chaque puits. L'incorporation de thymidine $^{14}C$ est mesurée 96 h après le début de l'expérience avec un compteur radioactif microbétâ (Perkin Elmer). Les données sont exprimées sous la forme d'un % de survie en faisant le ratio entre le décompte obtenu avec les cellules traitées par le cytotoxique et celui obtenu avec les cellules des puits contrôlés (traitées par le milieu de culture seul).

**Tableau II**

| Inhibition de la prolifération (pulse $^{14}C$ thymidine à 96 h) | IC50 [nM] | | |
|---|---|---|---|
| Structure du composé de formule (IA) | HCT116 | MDA-A1 | MDA-MB231 |
| 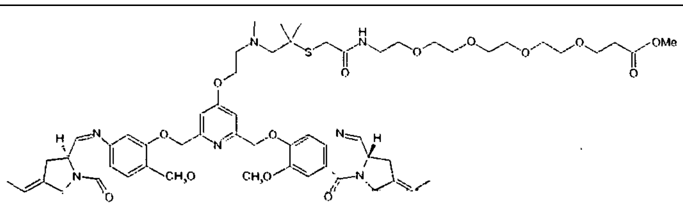 | 0,030 | 1,991 | 0,009 |
| 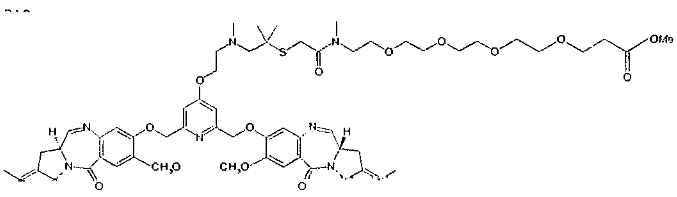 | 0,120 | 2,260 | 0,070 |

(suite)

| Inhibition de la prolifération (pulse $^{14}C$ thymidine à 96 h) | IC50 [nM] | | |
|---|---|---|---|
| Structure du composé de formule (IA) | HCT116 | MDA-A1 | MDA-MB231 |
| 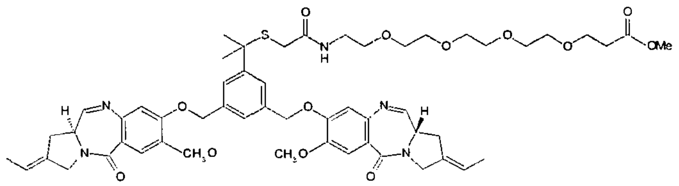 | 0,127 | >10 | 0,084 |
| 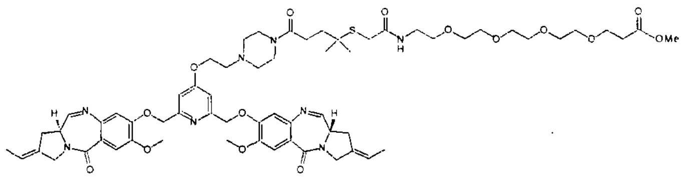 | 1,250 | >10 | 0,450 |

On constate que les composés testés pour lesquels $Z_bR_b$= -OMe ont une activité anticancéreuse puissante ; ceci laisse penser que les composés similaires se caractérisant par un autre groupe $Z_bR_b$ sont susceptibles de présenter une activité identique.

**<u>Evaluation de l'inhibition de la prolifération des lignées cellulaires MDA-MB-231 par les conjugués hu2H11-cytotoxique</u>**

Les cellules MDA-MB-231 dans leur phase de croissance exponentielle sont trypsinées et remises en suspension dans leur milieu de culture (DMEM/F12 Gibco #21331, 10% SVF Gibco #10500-056, 2 nM Glutamine Gibco #25030). La suspension cellulaire est ensemencée dans des plaques de culture 96 puits Cytostar (GE Healthcare Europe, #RPNQ0163) dans le milieu de culture complet contenant du sérum à une densité de 5000 cellules/puits. Après 4 h d'incubation, des dilutions successives des immunoconjugués anticorps-cytotoxique sont ajoutées dans les puits à des concentrations décroissantes de $10^{-7}$ à $10^{-12}$ M (en triplicate pour chaque concentration). Les cellules sont cultivées à 37°C dans une atmosphère à 5% de $CO_2$ en présence des immunoconjugués anticorps-cytotoxique pendant 3 jours. Le 4$^e$ jour, 10 µl d'une solution de thymidine $^{14}C$ (0,1 µCi/puits, Perkin Elmer #NEC56825000) sont ajoutés dans chaque puits. L'incorporation de thymidine $^{14}C$ est mesurée 96 h après le début de l'expérience avec un compteur radioactif microbêtâ (Perkin Elmer). Les données sont exprimées sous la forme d'un % de survie en faisant le ratio entre le décompte obtenu avec les cellules traitées par l'immunoconjugué et celui obtenu avec les cellules des puits contrôles (traitées par le milieu de culture seul). Dans certaines expériences*, l'anticorps nu hu2H11 a été ajouté dans les puits à une concentration de 1 µM au début de l'expérience et l'inhibition de la prolifération a été mesurée comme précédemment décrit.

**Tableau III**

| Inhibition de la prolifération (pulse $^{14}C$ thymidine à 96 h) | IC50 [pM] | | |
|---|---|---|---|
| Structure | IC50 moyen | IC50 moyen (+hu2H11 nu *) | rapport IC50 |
| 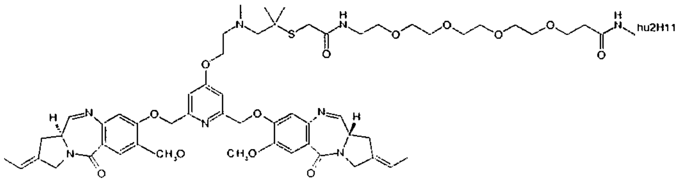 conjugué C2 | 31 | 540 | 17 |

(suite)

| Inhibition de la prolifération (pulse $^{14}C$ thymidine à 96 h) | IC50 [pM] | | |
|---|---|---|---|
| Structure | IC50 moyen | IC50 moyen (+hu2H11 nu *) | rapport IC50 |
| 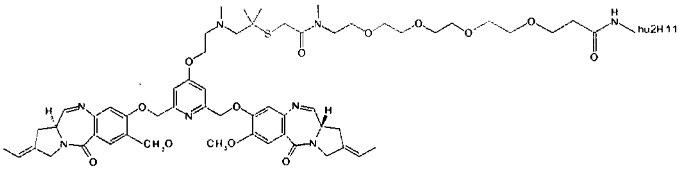 | 910 | 5095 | 6 |
| 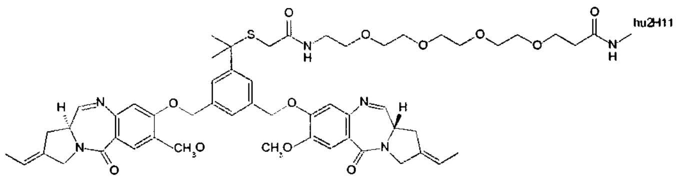 | 54 | 3981 | 74 |
| 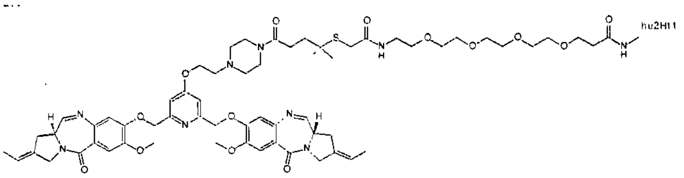 | 1255 | 9125 | 7 |
| 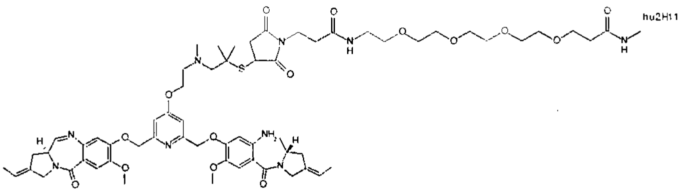 | 68,5 | 1119 | 16 |

**Stabilité monomérique des conjugués de dimères de tomaymycine non clivables**

Les conjugués hu2H11 non clivables préparés avec des dimères de tomaymycine décrits aux exemples 3 et 5 de la demande internationale WO 09016516 ont tendance à voir leur pureté monomérique diminuer dans le temps après plusieurs mois de stockage à 3-5°C. En effet, ces conjugués, formulés dans un tampon aqueux à pH 6,5 de concentration 10 mM en histidine contenant 10% de sucrose et 5% de NMP, présentant une moyenne de 3 à 3,5 dimères de tomaymycine par molécule d'anticorps, de pureté monomérique initiale de l'ordre de 97,5%, peuvent perdre de 6 à 15% de pureté monomérique en 6 à 8 mois. Ce phénomène n'a pas été observé avec le conjugué de l'exemple 2 qui, avec une moyenne de 3,5 dimères de tomaymycine par molécule d'anticorps, conserve une pureté monomérique supérieure à 99% en 4 mois dans les mêmes conditions de stockage, suggérant ainsi une meilleure stabilité de ce conjugué particulier. Il s'agit là d'un objectif général recherché dans le domaine des conjugués que de pouvoir disposer d'un conjugué conservant une pureté monomérique au cours du temps.

**Evaluation de l'activité antitumorale des conjugués hu2H11-cytotoxique sur souris SCID femelles porteuses d'un adénocarcinome humain avancé du sein, MDA-MB-231**

Deux conjugués du même anticorps hu2H11, notés C1 (préparé avec le dimère de tomaymycine décrit à l'exemple 5 de la demande internationale WO 09016516) et C2 (conjugué de l'exemple 2), ont été évalués à 4 niveaux de dose sur des tumeurs mammaires mesurables MDA-MB-231 implantées s.c. sur souris SCID femelles. Les groupes contrôle n'ont pas été traités. Les doses des deux conjugués sont données en μg de dimère de tomaymycine/kg. Elles ont été administrées à 80, 40, 20 et 10 μg/kg par injection de type bolus intraveineux (IV) au jour 13 pour C1 et au jour 24 pour C2.

En ce qui concerne l'évaluation de l'activité anti-tumorale des conjugués, les animaux ont été pesés quotidiennement et les tumeurs mesurées 2 fois par semaine à l'aide d'un pied à coulisse. L'évaluation de l'activité antitumorale

a été effectuée à la Dose Maximale Tolérée (DMT). Une dose produisant une perte de poids de 20% au nadir ou 10% (ou plus) de mortalité liée au conjugué est considérée comme étant toxique. Le poids corporel des animaux inclut le poids des tumeurs. Le poids des tumeurs est calculé selon la formule : masse (mg) = [longueur (mm) x largeur $(mm)^2$]/2. Les paramètres d'efficacité sont le ΔT/ΔC, le pourcentage moyen de régression, les régressions partielles et complètes (RP et RC) et le nombre de souris sans tumeur à la fin de l'étude (SST).

L'évolution du volume des tumeurs pour chaque souris traitée (T) et contrôle (C) est calculée pour chaque tumeur par soustraction du volume de la tumeur au jour de démarrage de l'étude au volume de la tumeur au jour d'observation spécifié. La moyenne ΔT est calculée pour le groupe traité et la moyenne ΔC est calculée pour le groupe contrôle. Le ratio ΔT/ΔC est alors calculé et exprimé en pourcentage: **ΔT/ΔC = (delta T/delta C) x 100**

Une dose est considérée comme étant thérapeutiquement active pour un **ΔT/ΔC** inférieur à 40% et très active pour un ATIAC inférieur à 10%. Lorsque le **ΔT/ΔC** est inférieur à 0, la dose est considérée hautement active et le pourcentage de régression est alors calculé (selon Plowman J, Dykes DJ, Hollingshead M, Simpson-Herren L and Alley MC. Human tumor xenograft models in NCI drug development : Feibig HH BA, editor. Basel: Karger.; 1999 p 101-125) :

Le **pourcentage de régression tumorale** est défini comme étant le pourcentage de diminution du volume tumoral dans le groupe traité au jour d'observation spécifié en comparaison de son volume au premier jour de traitement. A un temps t spécifique et pour chaque animal, le pourcentage de régression tumorale est calculé. Le pourcentage de régression moyen est alors calculé pour le groupe.

$$\text{Pourcentage de régression (au temps t)} = \frac{\text{volume}_{t0} - \text{volume}_{t}}{\text{volume}_{t0}} \times 100$$

**Régression partielle (RP) :** Les régressions sont définies comme étant partielles lorsque la diminution du volume tumoral atteint 50 % du volume de la tumeur en début de traitement.

**Régression complete (RC) :** La régression complète est obtenue lorsque le volume de la tumeur = 0 $mm^3$ (on considère que l'on est en présence d'une RC lorsque le volume de la tumeur ne peut être mesuré)

**SST :** Les souris sans tumeur sont définies comme étant des souris ne présentant pas de tumeur décelable à la fin de l'étude (au-delà de 100 jours de traitement).

**Tableau IV - Evaluation de l'activité antitumorale des conjugués C1 et C2 sur souris SCID femelles porteuses d'un adénocarcinome humain avancé du sein, MDA-MB-231.**

| Conjugué | Voie d'administration/ Dose en mL/kg par injection | Dose en µg/kg de dimère de tomaymycine (mg prot/kg) | Jour d'administration | Mortalité | Perte de poids moyenne en % au nadir (jour) | $\Delta T/\Delta C$ moyen en %(jour) | Pourcentage de régression moyen (jour) | Régressions | | | |
|---|---|---|---|---|---|---|---|---|---|---|---|
| | | | | | | | | Partielle | Complète | | |
| C2 | IV | 80 (2,98) | 24 | 4/6 | - | - | - | - | - | - | Toxique |
| DAR 3,5 | 10 mL/kg | 40 (1,49) | | 0/6 | -5,0(31) | <0 (46) | 96,2(46) | 6/6 | 1/6 | 1/6 | DMT.hautement actif |
| | | 20 (0,75) | | 0/6 | -4,4(31) | <0 (46) | 66,4 (46) | 4/6 | 0/6 | 0/6 | Hautement actif |
| | | 10 (0,37) | | 0/6 | -4,0 (31) | 14 (46) | - | 0/6 | 0/6 | 0/6 | Actif |

Abbréviations: DMT = Dose Maximale Tolérée, SST = Souris sans tumeur à la fin de l'étude

Selon un schéma d'administration de dose unique, la plus haute dose testée pour ces deux conjugués dans cette étude (80 µg/kg) s'est révélée toxique, induisant perte de poids et mortalité.

A la DMT (40 µg/kg), C1 est très actif et induit une perte de poids de 9,6% au nadir, un ΔT/ΔC de 7% et 3 RP pour 5 souris SCID. Une activité antitumorale a aussi été observée aux doses plus faibles de 20 et 10 µg/kg sans induire de RP.

A la DMT (40 µg/kg), C2 est hautement actif et induit une perte de poids de 5% au nadir, une régression tumorale de 96,2% avec 6 RP pour 6 souris dont 1 SST. Il est également très actif à la dose inférieure de 20 µg/kg avec une régression tumorale de 66,4% et 4 RP pour 6 souris. Une activité antitumorale a aussi été observée à la dose la plus basse de 10 µg/kg sans toutefois induire de régression ou de RP.

En conclusion, on constate que C2 présente une forte activité anticancéreuse et a montré une meilleure activité que C1 avec une régression tumorale, RP et SST à la DMT, non observées avec C1 aux mêmes doses.

## Revendications

**1.** Composé de formule (I) :

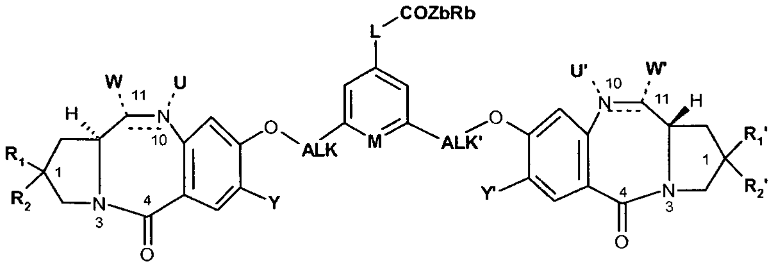

(I)

dans laquelle :

- --- représente une liaison simple ou une liaison double avec la condition que si

  --- représente une liaison simple alors :

  - ---- représente une liaison simple ;
  - U et/ou **U'** identique ou différent, représente(nt) indépendamment l'un de l'autre H ;
  - W et/ou **W'** identique ou différent, représente(nt) indépendamment l'un de l'autre : OH, -OR, -OCOR, -COOR, -OCOOR, -OCONRR', un carbamate cyclique tel que N10 et C11 soient inclus dans un cycle, -NRCONRR', -OCSNHR, un thiocarbamate cyclique tel que N10 et C11 soient inclus dans un cycle, -SH, -SR, -SOR, -SOOR, $-SO_3^-$, -NRSOOR', -NRR', une amine cyclique telle que N10 et C11 soient inclus dans un cycle, -NROR', -NRCOR', $-N_3$, -CN, Hal, un groupe trialkylphosphonium ou triarylphosphonium ;

- $\mathbf{R_1}$, $\mathbf{R_2}$, $\mathbf{R_1'}$, $\mathbf{R_2'}$ identiques ou différents, représentent, indépendamment l'un de l'autre : H, Hal ou un groupe $(C_1\text{-}C_6)$alkyle éventuellement substitué par un ou plusieurs substituant(s) choisi(s) parmi : Hal, CN, NRR', $CF_3$, OR, un groupe aryle ou hétéroaryle, $S(O)_qR$ avec q =0,1 ou 2 ;
  ou bien
- $\mathbf{R_1}$ et $\mathbf{R_2}$ et/ou $\mathbf{R_1'}$ et $\mathbf{R_2'}$ forment ensemble respectivement une double liaison $=CH_2$ ou $=CH\text{-}CH_3$ ;
- **Y** et **Y'**, identiques ou différents, représentent indépendamment l'un de l'autre H ou OR ;
- **M** représente CH ou N ;
- **ALK** et **ALK'** identiques ou différents, représentent indépendamment l'un de l'autre un groupe $(C_1\text{-}C_6)$alkylène ;
- **R** et **R'** représentent indépendamment l'un de l'autre, H ou un groupe $(C_1\text{-}C_6)$alkyle ou aryle éventuellement substitué par un ou plusieurs substituant(s) choisi(s) parmi : Hal, CN, NRR', $CF_3$, OR, un groupe aryle ou hétéroaryle ;
- L représente :

  - le groupe $-L_1\text{-}L_2-$ dans lequel $L_1$ est rattaché au cycle aromatique comprenant **M** par le groupe ALK ou OALK et représente l'un des groupes suivants :

- ALK-S-

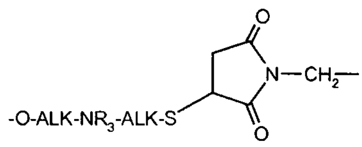

- O-ALK-$NR_3$-ALK-S-

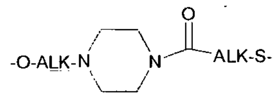

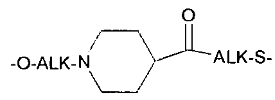

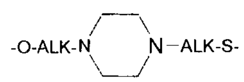

et $L_2$ représente le groupe $-CH_2C(=O)-NR_3-(CH_2CH_2O)_i-ALK-$ rattaché à $\mathbf{L_1}$ par $-CH_2C(=O)-$ ou bien

❖ le groupe $-O-ALK-NR_3-ALK-S-(CH_2CH_2O)_i-ALK-$ rattaché au cycle aromatique comprenant **M** par le groupe OALK ;

- $\mathbf{R_3}$ représente H ou un groupe $(C_1-C_6)$alkyle ;
- **i** représente un nombre entier allant de 1 à 40, plutôt de 1 à 20, de préférence de 1 à 10 ;
- $\mathbf{Z_b}$ représente une liaison simple, -O- ou -NH-, $\mathbf{R_b}$ représentant H ou un groupe $(C_1-C_6)$alkyle, $(C_3-C_7)$cycloalkyle, aryle, hétéroaryle ou $(C_3-C_7)$hétérocycloalkyle ou bien $\mathbf{Z_b}$ représente une liaison simple et $\mathbf{R_b}$ représente Hal.

**2.** Composé selon la revendication 1 de formule :

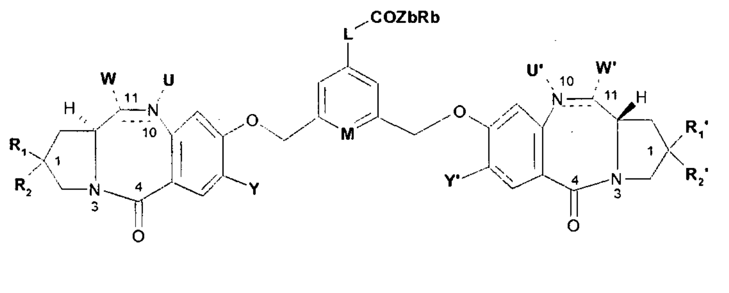

**3.** Composé selon la revendication 1 de formule **(IA)** ou **(IB)** :

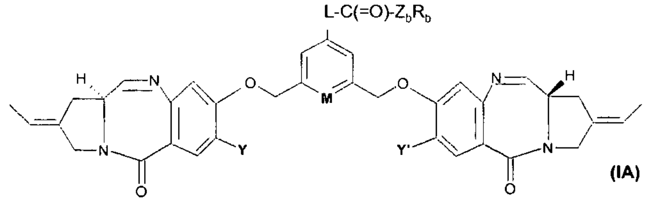
**(IA)**

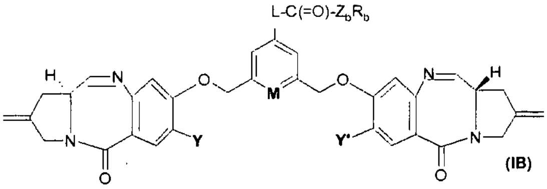
**(IB)**

**4.** Composé selon la revendication 1 à 3 dans lequel Y et Y' représentent un groupe $(C_1-C_4)$alcoxy, plus particulièrement méthoxy.

**5.** Composé selon la revendication 1 à 4 dans lequel L est choisi parmi :

- $ALK\text{-}S\text{-}CH_2C(=O)\text{-}NH\text{-}(CH_2CH_2O)_i\text{-}CH_2CH_2\text{-}$

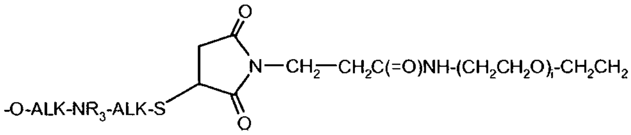

- $O\text{-}ALK\text{-}NR_3\text{-}ALK\text{-}S\text{-}CH_2C(=O)\text{-}NH\text{-}(CH_2CH_2O)_i\text{-}CH_2CH_2$

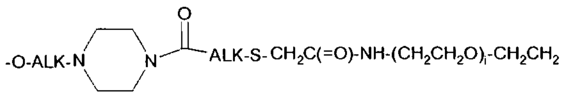 ; 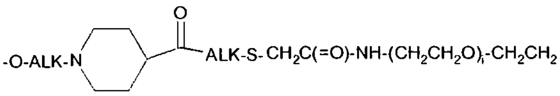 ;

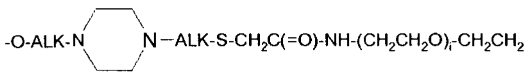 ;

- $O\text{-}ALK\text{-}NR_3\text{-}ALK\text{-}S\text{-}(CH_2CH_2O)_i\text{-}CH_2CH_2\text{-}$

**6.** Composé selon la revendication 1 ou 5 dans lequel L est choisi parmi :

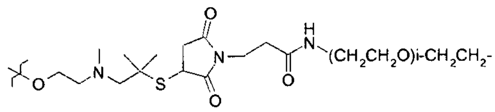 ; 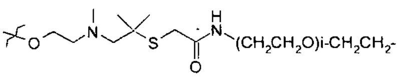

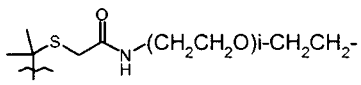 ; 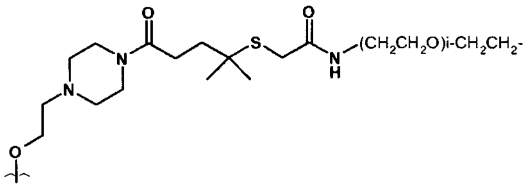 ;

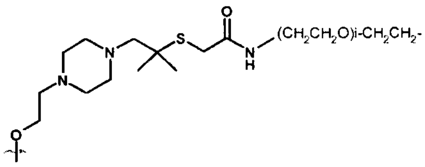 ; 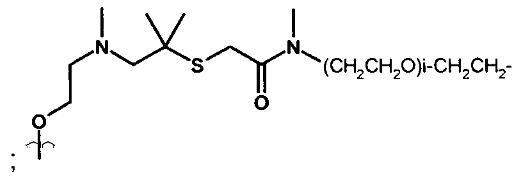 ;

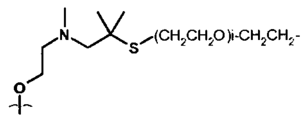 .

**7.** Composé selon la revendication 1 ou 5 dans lequel L est choisi parmi :

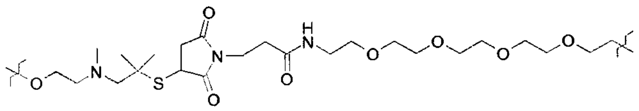

;

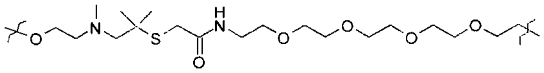

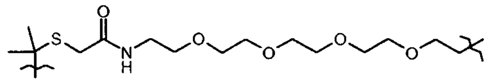

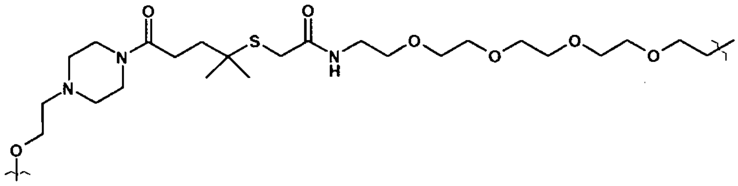

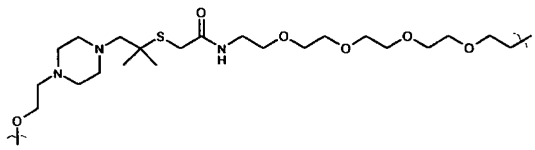

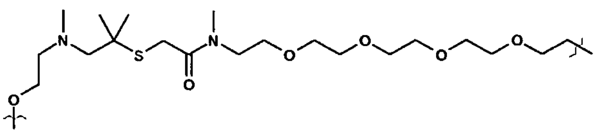

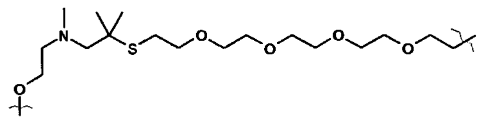

**8.** Composé selon la revendication 1 à 7 dans lequel $-COZ_bR_b$ représente -COOH, $-COO(C_1-C_6)$alkyle, $-COOCH_3$, $-COOCH_2CH=CH_2$,

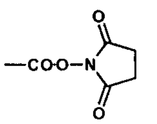

ou

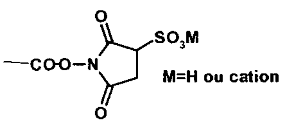


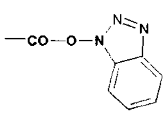

,

ou le groupe

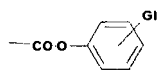

dans lequel **GI** représente au moins un groupe inductif, plus particulièrement

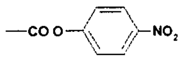 ou 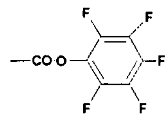

ou

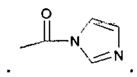

**9.** Composé selon la revendication 1 choisi parmi l'un des suivants :

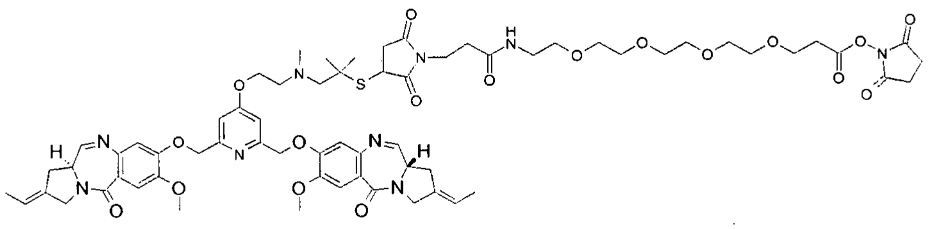

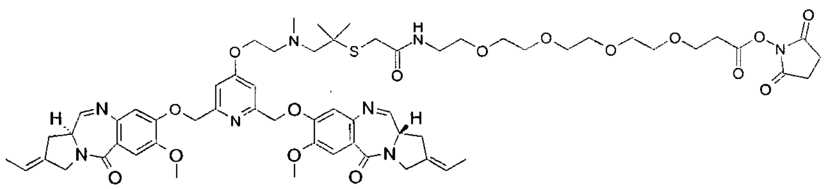

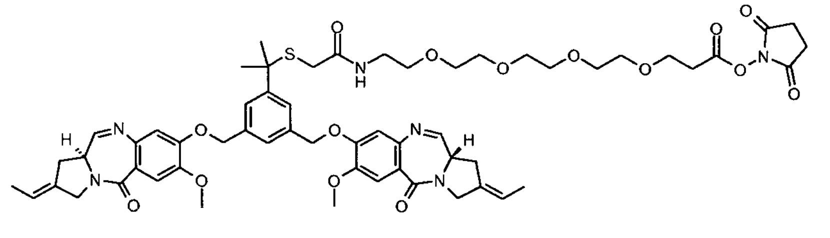

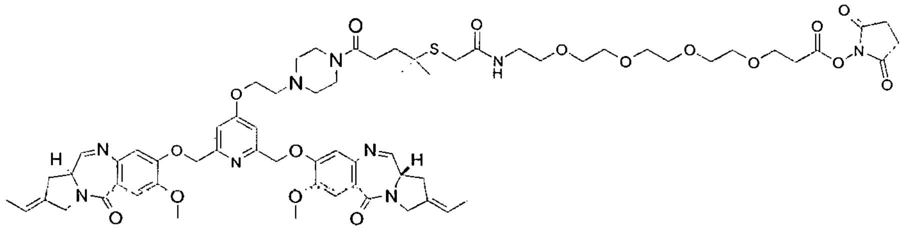

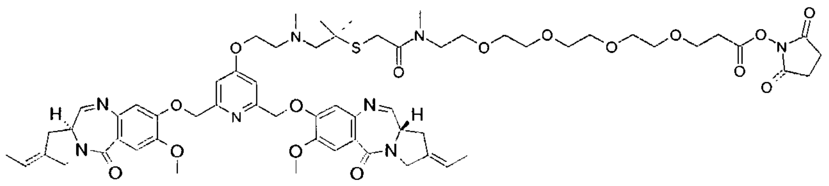

**10.** Procédé de préparation d'un conjugué, consistant à :

(i) mettre en contact et laisser réagir une solution aqueuse d'un agent de ciblage éventuellement tamponnée et une solution d'un composé tel que défini à l'une des revendications 1 à 9 ;
(ii) puis à éventuellement séparer le conjugué formé à l'étape (i) du composé de formule (I) et/ou de l'agent de ciblage n'ayant pas réagi et/ou des agrégats qui se seraient formés.

**11.** Procédé selon la revendication 10 dans lequel le groupe-$C(=O)Z_bR_b$ est réactif vis-à-vis des groupes chimiques présent sur l'agent de ciblage, notamment vis-à-vis des groupes amino présents sur un anticorps, de façon à assurer l'attachement du composé de formule (I) sur l'agent de ciblage par formation d'une liaison covalente.

**12.** Procédé selon la revendication 10 à 11 dans laquelle l'étape (ii) consiste à :

- séparer le conjugué qui s'est formé à l'étape (i) de l'agent de ciblage n'ayant pas réagi et des agrégats éventuellement présents dans la solution ;
ou bien
- à ne séparer le conjugué de l'étape (i) que du composé de formule (I) n'ayant pas réagi et des agrégats qui se seraient formés et à laisser dans la solution l'agent de ciblage qui n'aurait éventuellement pas réagi.

**13.** Procédé selon l'une des revendications 10 à 12 dans lequel l'agent de ciblage est un ligand, une protéine, un anticorps, plus particulièrement monoclonal, un fragment de protéine ou d'anticorps, un peptide, un oligonucléotide ou un oligosaccharide.

**14.** Procédé selon la revendication 10 à 13 dans lequel la réaction a lieu à une température comprise entre 20 et 40°C et/ou la durée de la réaction varie entre 1 et 24 h.

**15.** Procédé selon l'une des revendications 10 à 14 dans lequel après l'étape (i) ou (ii), la solution du conjugué subit une étape (iii) d'ultrafiltration et/ou de diafiltration.

**16.** Conjugué susceptible d'être obtenu par le procédé selon l'une des revendications 10 à 15.

**17.** Conjugué selon la revendication 16 dans lequel l'agent de ciblage est un anticorps, de préférence monoclonal, **caractérisé par** un DAR moyen compris entre 1 et 10, de préférence entre 1,5 et 7.
le DAR étant calculé par l'équation DAR = $c_D/c_A$
avec :

$$c_D = [(e_{A\,LO1} \times A_{LO2}) - (e_{A\,LO2} \times A_{LO1})] / [(e_{D\,LO2} \times e_{A\,LO1}) - (e_{A\,LO2} \times e_{D\,LO1})]$$

$$c_A = [A_{LO1} - (c_D \times e_{D\,LO1})] / e_{A\,LO1}$$

$A_{LO1}$ et $A_{LO2}$ désignant les absorbances d'une solution aqueuse du conjugué aux longueurs d'onde LO1 et LO2, mesurées sur le pic correspondant du spectre SEC.
$e_D$ LO1 et $e_{D\,LO2}$ désignent respectivement les coefficients d'absorption molaires du dimère de pyrrolo[1,4]benzodiazépine avant conjugaison aux deux longueurs d'onde LO1 et L02 ;
$e_{A\,LO1}$ et $e_{A\,LO2}$ désignent respectivement les coefficients d'absorption molaires de l'anticorps nu aux deux longueurs d'onde LO1 et LO2,
LO1= 280 nm et LO2= 320 nm.

**18.** Utilisation d'un composé selon l'une des revendications 1 à 9 pour la préparation d'un agent de ciblage auquel est attaché de façon covalente en position para de M le dimère de formule :

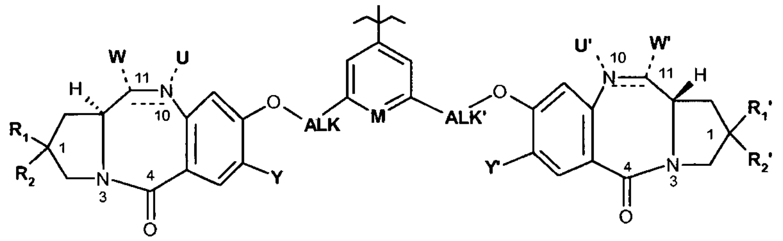

**19.** Utilisation selon la revendication 18 dans laquelle l'agent de ciblage est un anticorps, de préférence monoclonal.

**20.** Utilisation selon la revendication 18 ou 19 dans laquelle le dimère a pour formule :

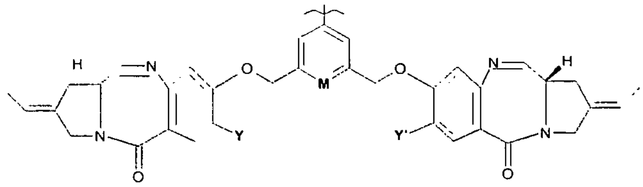

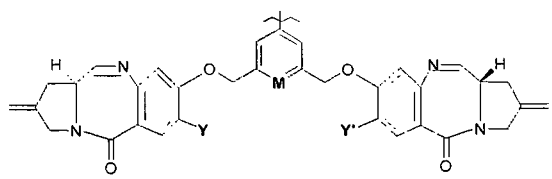

**21.** Utilisation selon la revendication 18 à 20 dans laquelle Y et Y' représentent un groupe ($C_1$-$C_4$)alcoxy, plus particulièrement méthoxy.

**22.** Agent de ciblage auquel a été attaché de façon covalente en position para de M le dimère de formule :

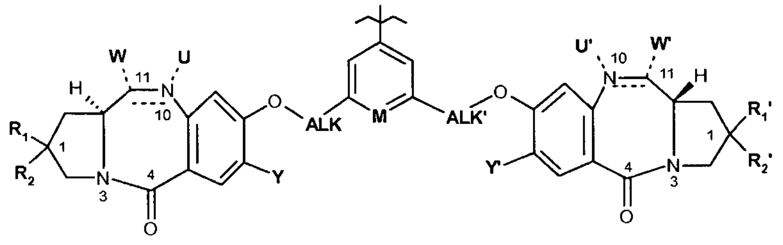

après réaction d'un composé tel que défini l'une des revendications 1 à 9 avec l'agent de ciblage.

**23.** Agent de ciblage selon la revendication 22 dans lequel le dimère est tel que défini à l'une des revendications 20 ou 21.

**24.** Agent de ciblage selon la revendication 23 ayant une affinité pour un antigène ou groupe d'antigènes localisé sur des cellules cancéreuses ou des cellules stromales associées à une tumeur.

**25.** Agent de ciblage selon la revendication 22 à 24 qui est un anticorps, de préférence monoclonal.

**26.** Solution de conjugué susceptible d'être obtenue par le procédé selon l'une des revendications 10 à 15.

**27.** Composé selon l'une des revendications 1 à 9 pour utilisation en tant qu'anticancéreux.

**28.** Conjugué selon la revendication 16 ou 17 pour utilisation en tant qu'anticancéreux.

**29.** Composition pharmaceutique comprenant un composé selon l'une des revendications 1 à 9 ou bien un conjugué selon la revendication 16 ou 17 ainsi qu'au moins un excipient.

**30.** Utilisation d'un composé de formule $\mathbf{P_2}$

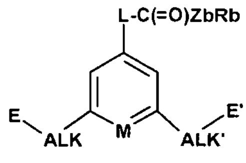
$P_2$

ou bien un composé de formule :

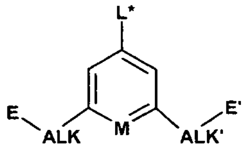

dans laquelle **L*** est choisi parmi :

- ALK-SH ;
- O-ALK-$NR_3$-ALK-SH ;

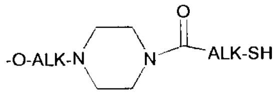 ; 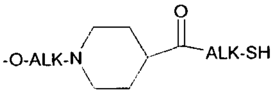 ; 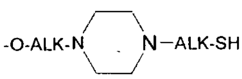

formules dans lesquelles :

■ L, M, ALK, ALK', $R_3$, $Z_b$ et $\mathbf{R_b}$ sont tels que définis à l'une des revendications 1 à 9 ;
■ E et E' représentent indépendamment l'un de l'autre un groupe -OH ou un groupe partant ;

en tant qu'intermédiaire pour la préparation d'un composé de formule (I) tel que défini à l'une des revendications 1 à 9.

**31.** Utilisation selon la revendication 30 dans laquelle le groupe partant est choisi parmi un atome d'halogène, un groupe mésylate, tosylate, nosylate ou $-OPPh_3^+$.

**32.** Utilisation d'un composé choisi parmi l'un des suivants

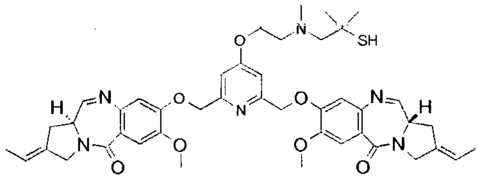
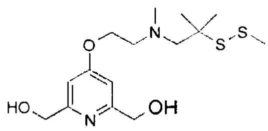
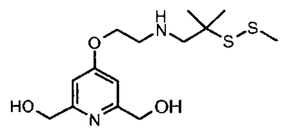

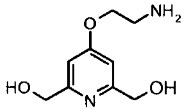
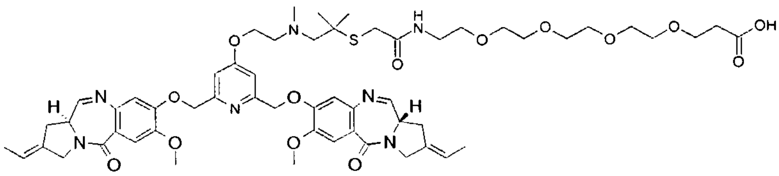

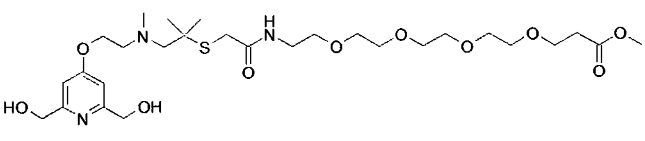
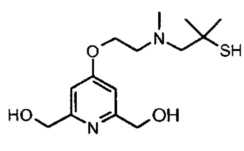

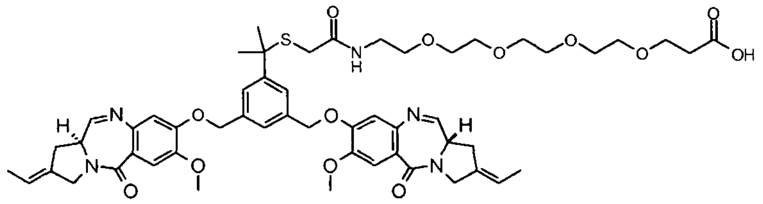

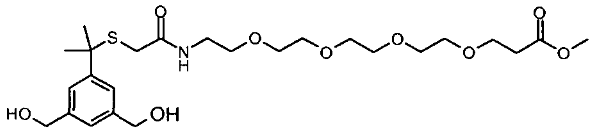

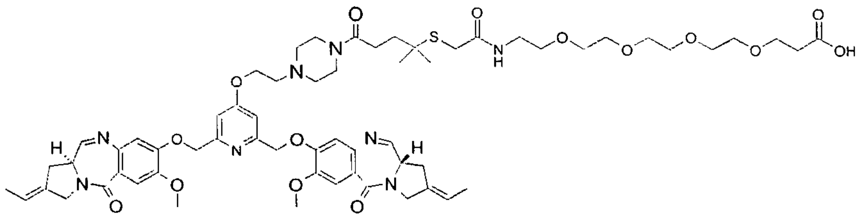

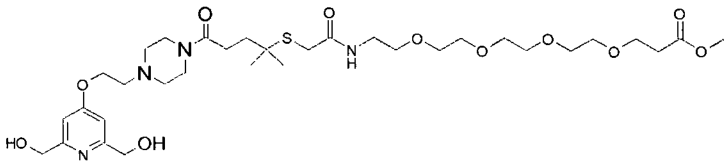

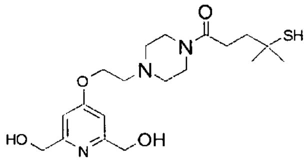

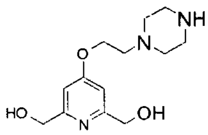

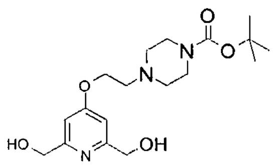

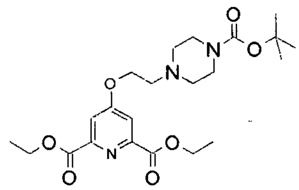

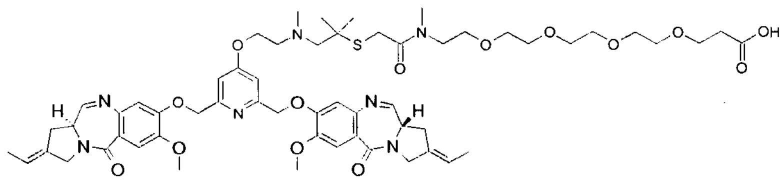

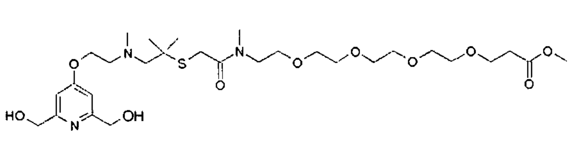

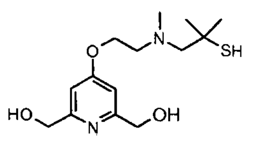

étant entendu que la fonction terminale -COOH peut être remplacée par une fonction terminale -COO($C_1$-$C_6$)alkyle, notamment -COOMe, et que la fonction terminale -SH peut être remplacée par une fonction disulfure, notamment -SSMe,
en tant qu'intermédiaire pour la préparation d'un composé de formule (I) tel que défini à l'une des revendications 1 à 9.

**33.** Utilisation d'un composé selon l'une des revendications 1 à 9 pour la fabrication d'un anticancéreux.

**34.** Utilisation d'un conjugué selon la revendication 16 ou 17 pour la fabrication d'un anticancéreux.

## Patentansprüche

**1.** Verbindung der Formel (I):

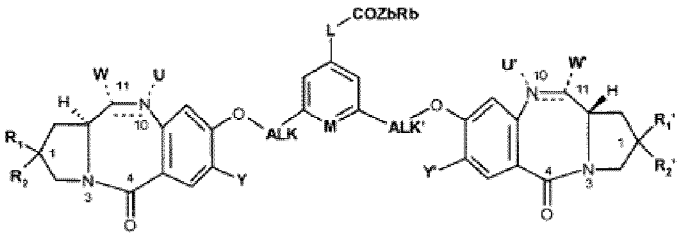

(I)

wobei:

● ---- für eine Einfachbindung oder eine Doppelbindung steht, mit der Maßgabe, dass, wenn ---- für eine Einfachbindung steht, dann:

❖ ---- für eine Einfachbindung steht,
❖ U und/oder U', die gleich oder verschieden sind, unabhängig voneinander für H steht/stehen,
❖ W und/oder W', die gleich oder verschieden sind, unabhängig voneinander für Folgendes steht/stehen:

OH, -OR, -OCOR, -COOR, -OCOOR, -OCONRR', ein zyklisches Carbamat, derart, dass N10 und C11 in einem Ring enthalten sind, -NRCONRR', -OCSNHR, ein zyklisches Thiocarbamat, derart, dass N10 und C11 in einem Ring enthalten sind, -SH, -SR, -SOR, -SOOR, $-SO_3^-$, -NRSOOR', -NRR', ein zyklisches Amin, derart, dass N10 und C11 in einem Ring enthalten sind, -NROR', -NRCOR', $-N_3$, CN, Hal, eine Trialkylphosphonium- oder Triarylphosphonium-Gruppe,

● $R_1$, $R_2$, $R_1$', $R_2$' , die gleich oder verschieden sind, unabhängig voneinander für: H, Hal oder eine $(C_1-C_6)$-Alkyl-Gruppe stehen, die gegebenenfalls mit einem oder mehreren Substituent(en) substituiert ist, ausgewählt aus : Hal, CN, NRR', $CF_3$, OR, einer Aryl- oder Heteroaryl-Gruppe, $S(O)_qR$ mit q = 0, 1 oder 2,
oder
● $R_1$ und $R_2$ und/oder $R_1$' und $R_2$' jeweils zusammen eine Doppelbindung $=CH_2$ oder $=CH-CH_3$ bilden,
● Y und Y', die gleich oder verschieden sind, unabhängig voneinander für H oder OR stehen,
● M für CH oder N steht,
● ALK und ALK', die gleich oder verschieden sind, unabhängig voneinander für eine $(C_1-C_6)$-Alkylen-Gruppe stehen,
● R und R' unabhängig voneinander für H oder eine $(C_1-C_6)$-Alkyl- oder Aryl-Gruppe stehen, die gegebenenfalls mit einem oder mehreren Substituent(en) substituiert ist, ausgewählt aus: Hal, CN, NRR', $CF_3$, OR, einer Aryl- oder Heteroaryl-Gruppe,
● L steht für:

❖ die Gruppe $-L_1-L_2-$, worin $L_1$ an den M umfassenden aromatischen Ring über die Gruppe ALK oder OALK gebunden ist und durch eine der folgenden Gruppen dargestellt wird:

- ALK-S-

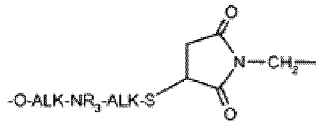

- $O-ALK-NR_3-ALK-S-$

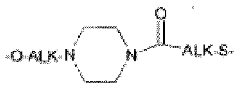

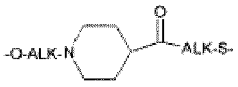

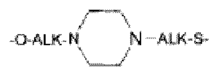

und $L_2$ für die Gruppe $-CH_2C(=O)-NR_3-(CH_2CH_2O)_i-ALK$-steht, die an $L_I$ über $-CH_2C(=O)$-gebunden ist, oder

∵ für die Gruppe $-O-ALK-NR_3-ALK-S-$ $(CH_2CH_2O)_i-ALK-$, die an den M umfassenden aromatischen Ring über die Gruppe OALK gebunden ist,

● $R_3$ für H oder eine $(C_1-C_6)$-Alkyl-Gruppe steht,

● i für eine ganze Zahl von 1 bis 40, besser von 1 bis 20, vorzugsweise von 1 bis 10 steht,

● $Z_b$ für eine Einfachbindung, -0- oder -NH- steht, wobei $R_b$ für H oder eine $(C_1-C_6)$-Alkyl-, $(C_3-C_7)$-Cycloalkyl-, Aryl-, Heteroaryl- oder $(C_3-C_7)$-Heterocycloalkyl-Gruppe steht, oder auch $Z_b$ für eine Einfachbindung und $R_b$ für Hal steht.

**2.** Verbindung nach Anspruch 1 der Formel:

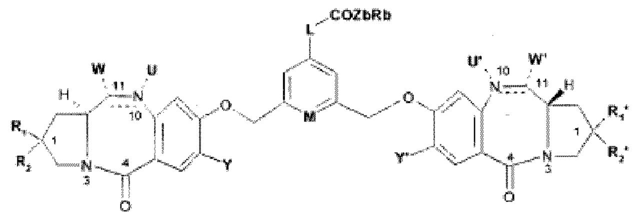

**3.** Verbindung nach Anspruch 1 der Formel (IA) oder (IB) :

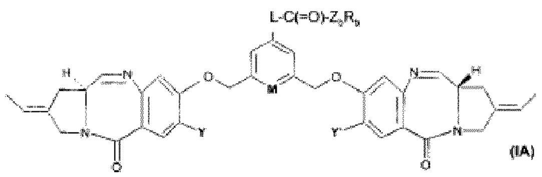

(IA)

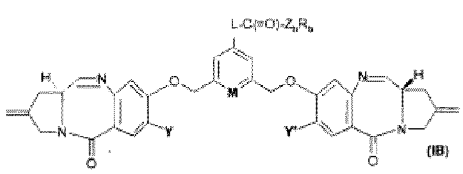

(IB)

**4.** Verbindung nach Anspruch 1 bis 3, wobei Y und Y' für eine $(C_1-C_4)$-Alkoxy-, insbesondere Methoxy-Gruppe stehen.

**5.** Verbindung nach Anspruch 1 bis 4, wobei L aus Folgenden ausgewählt ist: $-Alk-S-CH_2C(=O)-NH-(CH_2CH_2O)_i-CH_2CH_2-$

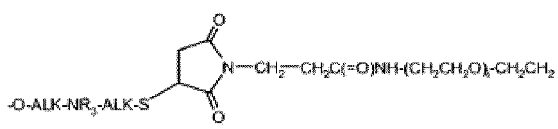

$-O-ALK-NR_3-ALK-S-CH_2C(=0)-NH-(CH_2CH_2O)_i-CH_2CH_2$

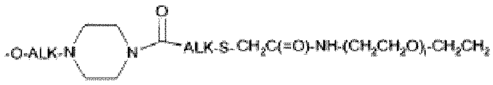
;
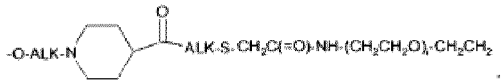
;

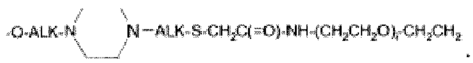
;

-O-Alk-$NR_3$-ALK-S- $(CH_2CH_2O)_i$-$CH_2CH_2$-

**6.** Verbindung nach Anspruch 1 oder 5, wobei L aus Folgenden ausgewählt ist:

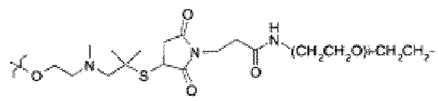
;
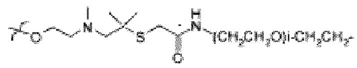

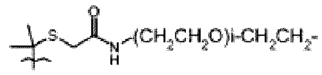
;
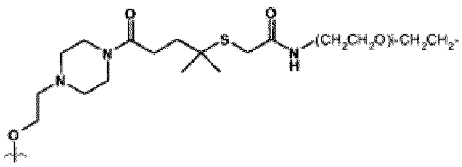
;

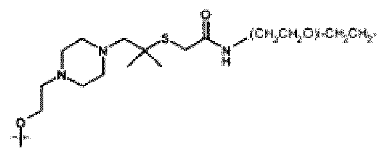
;
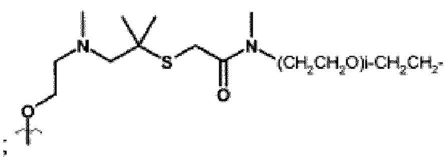
;

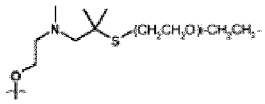
.

**7.** Verbindung nach Anspruch 1 oder 5, wobei L aus Folgenden ausgewählt ist:

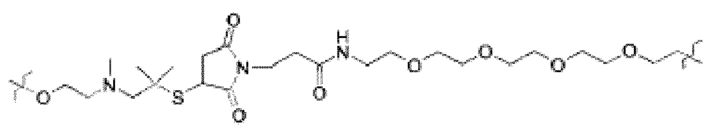

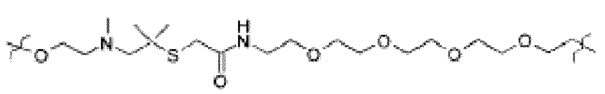

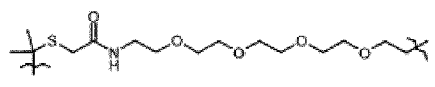

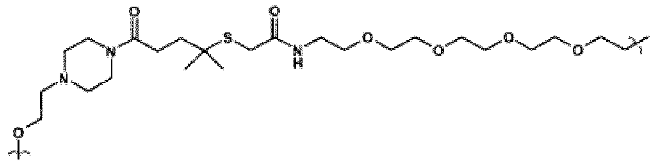

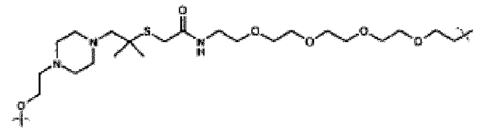

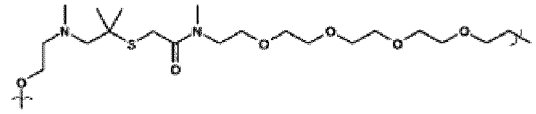

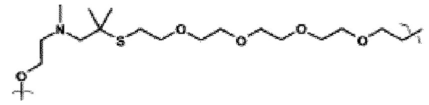

**8.** Verbindung nach Anspruch 1 bis 7, wobei $-COZ_bR_b$ für -COOH, $-COO(C_1-C_6)$ -Alkyl, $-COOCH_3$, $COOCH_2CH=CH_2$,

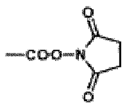

oder

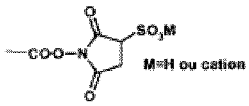

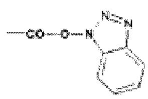

oder die Gruppe

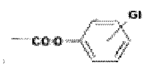

steht, wobei GI für mindestens eine induktive Gruppe, insbesondere

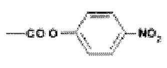

oder

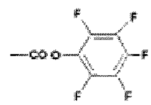,

oder

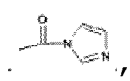,

steht.

**9.** Verbindung nach Anspruch 1, die aus den folgenden ausgewählt ist:

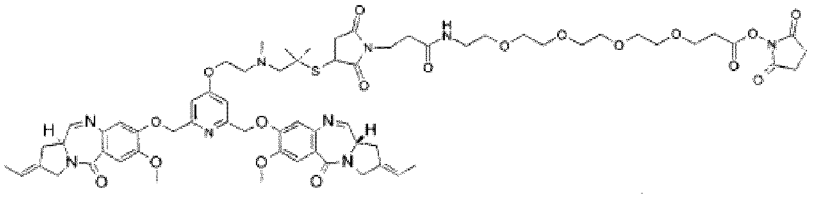

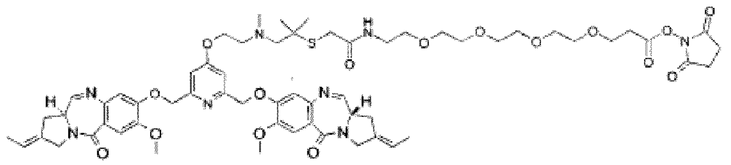

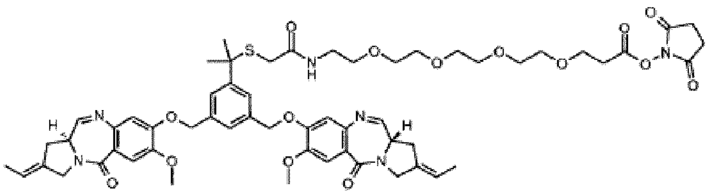

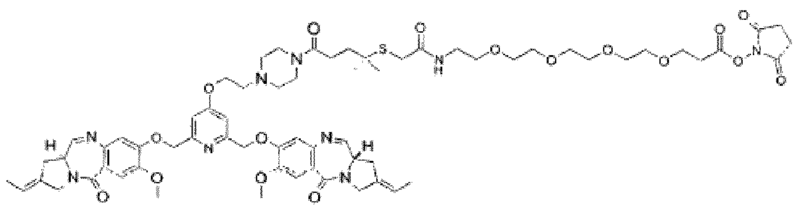

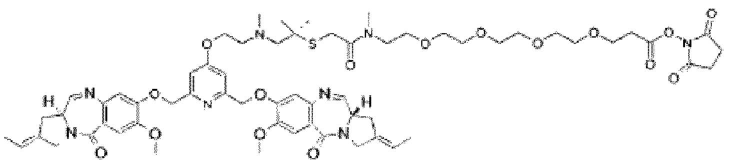

**10.** Verfahren zur Herstellung eines Konjugats, bei dem man:

(i) eine gegebenenfalls gepufferte, wässrige Lösung eines Zielsteuerungsmittels und eine Lösung einer Verbindung nach einem der Ansprüche 1 bis 9 in Kontakt bringt und umsetzt,
(ii) anschließend gegebenenfalls das in Schritt (i) gebildete Konjugat von der Verbindung der Formel (I) und/oder dem nicht umgesetzten Zielsteuerungsmittel und/oder von Aggregaten, die sich möglicherweise gebildet haben, abtrennt.

**11.** Verfahren nach Anspruch 10, wobei die Gruppe -C(=O)$Z_bR_b$ mit auf dem Zielsteuerungsmittel vorhandenen chemischen Gruppen, insbesondere mit auf einem Antikörper vorhandenen Aminogruppen, derart reaktiv ist, dass die Bindung der Verbindung der Formel (I) an das Zielsteuerungsmittel durch Bildung einer kovalenten Bindung gewährleistet ist.

**12.** Verfahren nach Anspruch 10 bis 11, wobei man in Schritt (ii):

- das im Schritt (i) gebildete Konjugat von dem nicht umgesetzten Zielsteuerungsmittel und gegebenenfalls in der Lösung vorhandenen Aggregaten abtrennt
oder
- nur das Konjugat von Schritt (i) von der nicht umgesetzten Verbindung der Formel (I) und den Aggregaten, die sich gebildet haben können, abtrennt und in der Lösung das Zielsteuerungsmittel belässt, das gegebenenfalls

nicht umgesetzt worden ist.

**13.** Verfahren nach einem der Ansprüche 10 bis 12, wobei das Zielsteuerungsmittel ein Ligand, ein Protein, ein Antikörper, insbesondere ein monoklonaler Antikörper, ein Fragment eines Proteins oder Antikörpers, ein Peptid, ein Oligonukleotid oder ein Oligosaccharid ist.

**14.** Verfahren nach Anspruch 10 bis 13, wobei die Reaktion bei einer Temperatur zwischen 20 und 40 °C erfolgt und/oder die Dauer der Reaktion zwischen 1 und 24 Std. variiert.

**15.** Verfahren nach einem der Ansprüche 10 bis 14, wobei nach dem Schritt (i) oder (ii) die Lösung des Konjugats einem Ultrafiltrations- und/oder Diafiltrationsschritt (iii) unterzogen wird.

**16.** Konjugat, das durch das Verfahren nach einem der Ansprüche 10 bis 15 erhältlich ist.

**17.** Konjugat nach Anspruch 16, wobei das Zielsteuerungsmittel ein Antikörper, vorzugsweise ein monoklonaler Antikörper, ist, **gekennzeichnet durch** ein mittleres DAR zwischen 1 und 10, vorzugsweise zwischen 1,5 und 7, wobei das DAR **durch** die Gleichung DAR $_{=}$ $c_D/c_A$ berechnet wird
mit:

$$c_D = [(e_{A\,LO1} \times A_{LO2}) - (e_{A\,LO2} \times A_{LO1})] / [(e_{D\,LO2} \times e_{A\,LO1}) - (e_{A\,LO2} \times e_{D\,LO1})]$$

$$c_A = [A_{LO1} - (c_D \times e_{D\,LO1})] / e_{A\,LO1}$$

wobei $A_{LO1}$ und $A_{LO2}$ für die Absorptionen einer wässrigen Lösung des Konjugats bei den Wellenlängen LO1 und L02 stehen, gemessen am entsprechenden Peak des SEC-Spektrums,
$e_{D\,LO1}$ bzw. $e_{D\,LO2}$ für die molaren Absorptionskoeffizienten des Dimers Pyrrolo[1,4]benzodiazepin vor der Konjugation bei den zwei Wellenlängen LO1 und L02 stehen,
$e_{A\,LO1}$ bzw. $e_{A\,LO2}$ für die molaren Absorptionskoeffizienten des nackten Antikörpers bei den zwei Wellenlängen LO1 und L02 stehen,
LO1 = 280 nm und L02 = 320 nm.

**18.** Verwendung einer Verbindung nach einem der Ansprüche 1 bis 9 zur Herstellung eines Zielsteuerungsmittels, an das kovalent an der para-Position von M das Dimer der folgenden Formel gebunden ist:

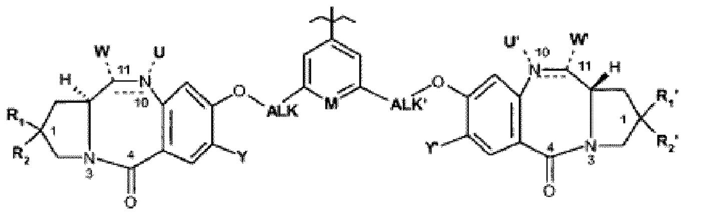

**19.** Verwendung nach Anspruch 18, wobei das Zielsteuerungsmittel ein Antikörper, vorzugsweise ein monoklonaler Antikörper, ist.

**20.** Verwendung nach Anspruch 18 oder 19, wobei das Dimer folgende Formel hat:

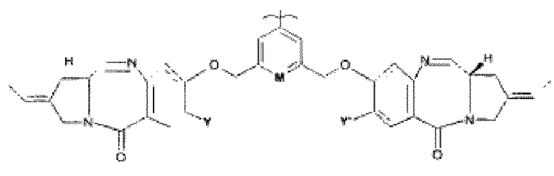 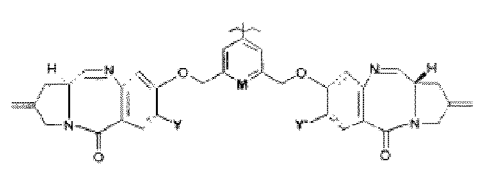

**21.** Verwendung nach Anspruch 18 bis 20, wobei Y und Y' für eine ($C_1$-$C_4$)-Alkoxy-, insbesondere eine Methoxy-Gruppe stehen.

**22.** Zielsteuerungsmittel, an das kovalent an der para-Position von M das Dimer der folgenden Formel:

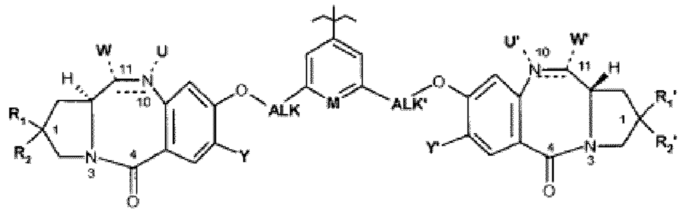

nach der Umsetzung einer Verbindung nach einem der Ansprüche 1 bis 9 mit dem Zielsteuerungsmittel gebunden ist.

**23.** Zielsteuerungsmittel nach Anspruch 22, wobei das Dimer wie in einem der Ansprüche 20 oder 21 definiert ist.

**24.** Zielsteuerungsmittel nach Anspruch 23 mit einer Affinität für ein Antigen oder eine Gruppe von Antigenen auf Krebszellen oder mit einem Tumor assoziierten Stromazellen.

**25.** Zielsteuerungsmittel nach Anspruch 22 bis 24, bei dem es sich um einen Antikörper, vorzugsweise einen monoklonalen Antikörper handelt.

**26.** Konjugat-Lösung, die durch das Verfahren nach einem der Ansprüche 10 bis 15 erhältlich ist.

**27.** Verbindung nach einem der Ansprüche 1 bis 9 für die Verwendung als Antikrebsmittel.

**28.** Konjugat nach Anspruch 16 oder 17 für die Verwendung als Antikrebsmittel.

**29.** Pharmazeutische Zusammensetzung, umfassend eine Verbindung nach einem der Ansprüche 1 bis 9 oder ein Konjugat nach Anspruch 16 oder 17 sowie mindestens einen Excipienten.

**30.** Verwendung einer Verbindung der Formel $P_2$

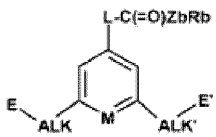

$P_2$

oder einer Verbindung der Formel:

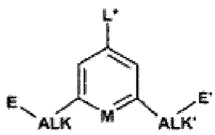

,

wobei L* ausgewählt ist aus:

- ALK-SH,
- O-ALK-$NR_3$-ALK-SH,

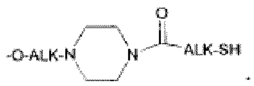 ; 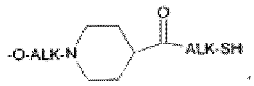 ; 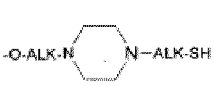 ,

wobei in den Formeln:

- L, M, ALK, ALK', $R_3$, $Z_b$ und $R_b$ wie in einem der Ansprüche 1 bis 9 definiert sind,
- E und E' unabhängig voneinander für eine Gruppe -OH oder eine Abgangsgruppe stehen,

als Zwischenprodukt für die Herstellung einer Verbindung der Formel (I) nach einem der Ansprüche 1 bis 9.

**31.** Verwendung nach Anspruch 30, wobei die Abgangsgruppe ausgewählt ist aus einem Halogenatom, einer Mesylat-, Tosylat-, Nosylat-Gruppe oder $-OPPh_3^+$.

**32.** Verwendung einer aus einer der folgenden ausgewählten Verbindung:

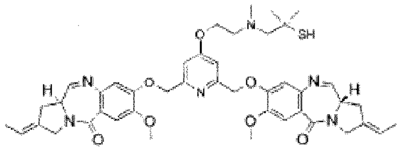

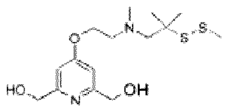

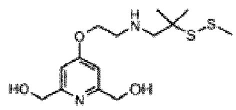

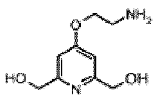

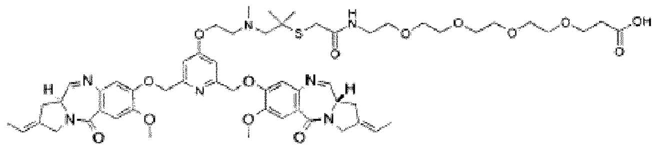

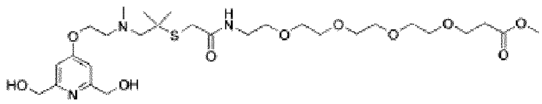

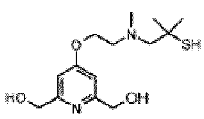

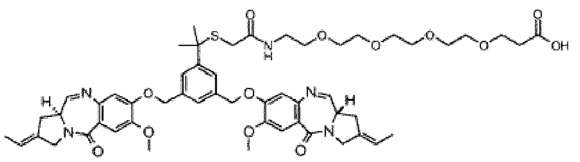

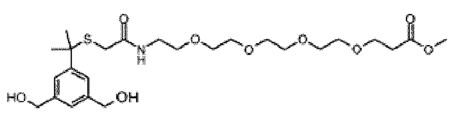

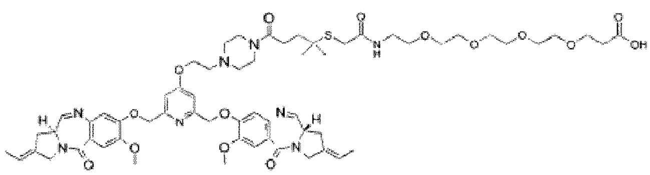

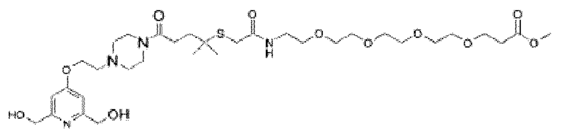
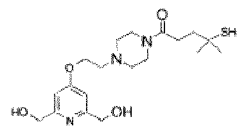

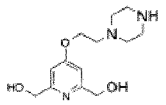
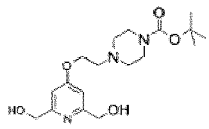
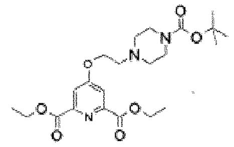

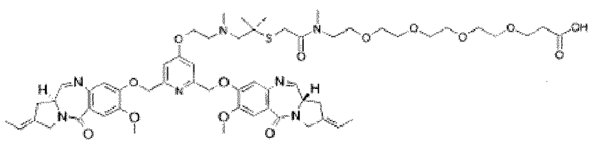

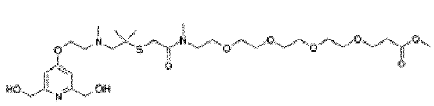
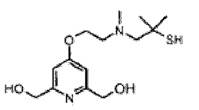

,

wobei selbstverständlich sein sollte, dass die endständige Funktion -COOH durch eine endständige Funktion COO($C_1$-$C_6$)-Alkyl, insbesondere -COOMe, ersetzt werden kann und dass die endständige Funktion -SH durch eine Disulfid-Funktion insbesondere -SSMe, ersetzt werden kann,
als Zwischenprodukt zur Herstellung einer Verbindung der Formel (I) nach einem der Ansprüche 1 bis 9.

**33.** Verwendung einer Verbindung nach einem der Ansprüche 1 bis 9 zur Herstellung eines Antikrebsmittels.

**34.** Verwendung eines Konjugats nach Anspruch 16 oder 17 zur Herstellung eines Antikrebsmittels.

## Claims

**1.** Compound of formula (I):

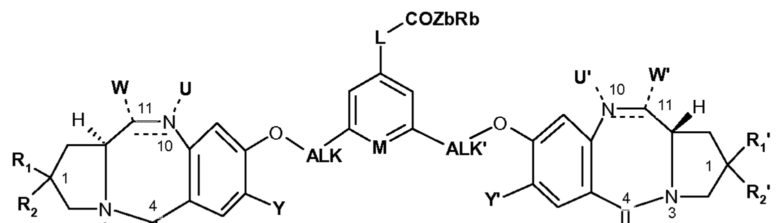

(I)

in which:

• represents a single bond or a double bond with the condition that, if represents a single bond, then:

- ❖---- represents a single bond;
- ❖ U and/or **U',** which are identical or different, represent(s), independently of one another, H;
- ❖ **W** and/or **W',** which are identical or different, represent(s), independently of one another: OH, -OR, -OCOR, -COOR, -OCOOR, -OCONRR', a cyclic carbamate such that N10 and C11 are included in a ring, -NRCONRR', -OCSNHR, a cyclic thiocarbamate such that N10 and C11 are included in a ring, - SH, -SR,

-SOR, -SOOR, $-SO_3^-$, -NRSOOR', -NRR', a cyclic amine such that N10 and C11 are included in a ring, -NROR', -NRCOR', $-N_3$, -CN, Hal or a trialkylphosphonium or triarylphosphonium group;

• $\mathbf{R_1}$, $\mathbf{R_2}$, $\mathbf{R_1}$**'** and $\mathbf{R_2}$**'** , which are identical or different, represent, independently of one another: H, Hal or a (C1-C6)alkyl group optionally substituted by one or more substituent(s) chosen from: Hal, CN, NRR', $CF_3$, OR, an aryl or heteroaryl group, or $S(O)_qR$ with q = 0, 1 or 2;
or else
• $\mathbf{R_1}$ and $\mathbf{R_2}$ and/or $\mathbf{R_1}$**'** and $\mathbf{R_2}$**'** together form respectively a double bond $=CH_2$ or $=CH-CH_3$;
• **Y** and **Y',** which are identical or different, represent, independently of one another, H or OR;
• **M** represents CH or N;
• **ALK** and **ALK',** which are identical or different, represent, independently of one another, a $(C_1-C_6)$alkylene group;
• **R** and **R'** represent, independently of one another, H or a $(C_1-C_6)$alkyl or aryl group optionally substituted by one or more substituent(s) chosen from: Hal, CN, NRR', $CF_3$, OR or an aryl or heteroaryl group;
• **L** represents:

❖ the $\mathbf{-L_1-L_2-}$ group in which $\mathbf{L_1}$ is attached to the aromatic ring comprising **M** via the ALK or OALK group and represents one of the following groups:
-ALK-S-

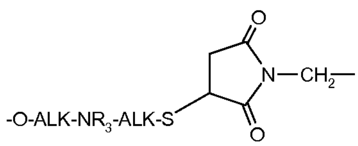

$-O-ALK-NR_3-ALK-S-$

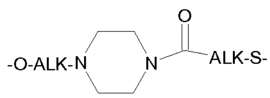

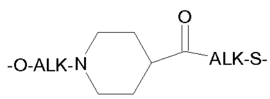

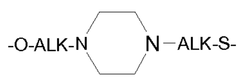

and $\mathbf{L_2}$ represents the $-CH_2C(=O)-NR_3-(CH_2CH_2O)_i$-ALK-group attached to $\mathbf{L_1}$ via $-CH_2C(=O)-$;
or else
❖ the $-O-ALK-NR_3-ALK-S-(CH_2CH_2O)_i$-ALK- group attached to the aromatic ring comprising **M** via the OALK group;

• $\mathbf{R_3}$ represents H or a $(C_1-C_6)$alkyl group;
• **i** represents an integer ranging from 1 to 40, rather from 1 to 20, preferably from 1 to 10;
• $\mathbf{Z_b}$ represents a single bond, -0- or -NH- and $\mathbf{R_b}$ represents H or a $(C_1-C_6)$alkyl, $(C_3-C_7)$cycloalkyl, aryl, heteroaryl or $(C_3-C_7)$heterocycloalkyl group or else $\mathbf{Z_b}$ represents a single bond and $\mathbf{R_b}$ represents Hal.

**2.** Compound according to Claim 1 of formula:

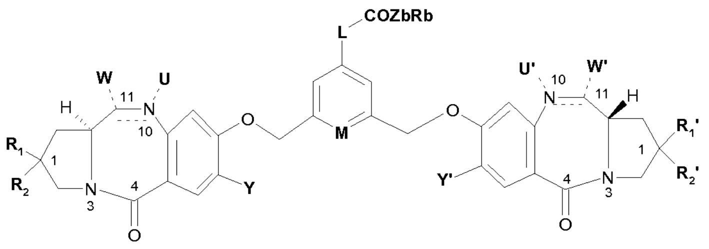

3. Compound according to Claim 1 of formula **(IA)** or **(IB)** :

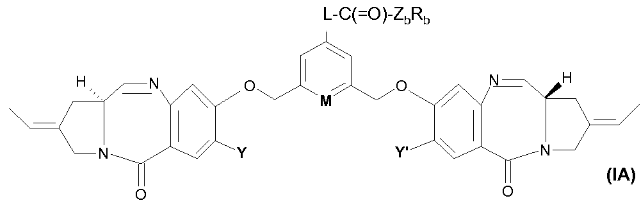

(IA)

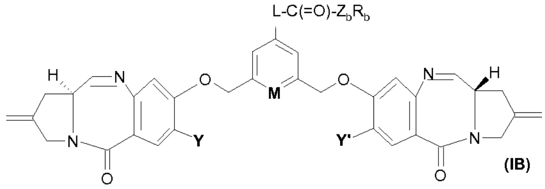

(IB)

4. Compound according to Claim 1 to Claim 3, in which **Y** and **Y'** represent a ($C_1$-$C_4$)alkoxy group, more particularly a methoxy group.

5. Compound according to Claim 1 to Claim 4, in which **L** is chosen from: $-ALK-S-CH_2C(=O)-NH-(CH_2CH_2O)_i-CH_2CH_2-$

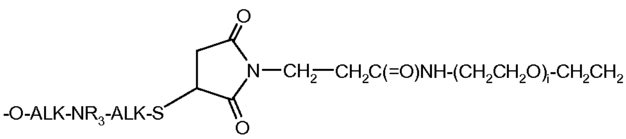

$-O-ALK-NR_3-ALK-S-CH_2C (=0) -NH- (CH_2CH_2O)_i-CH2_CH2$

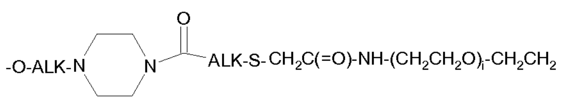

;

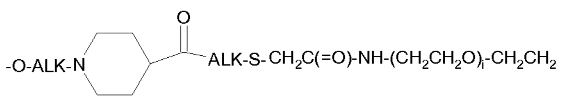

;

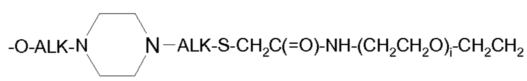

;

$-O-ALK-NR_3-ALK-S-(CH_2CH_2O)_i-CH_2CH_2-$

**6.** Compound according to Claim 1 or Claim 5, in which **L** is chosen from:

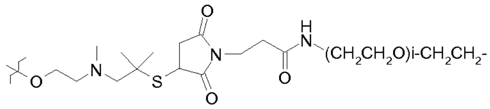

;

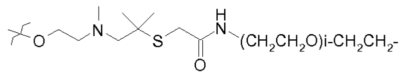

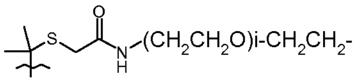

;

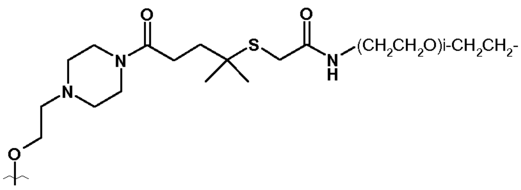

;

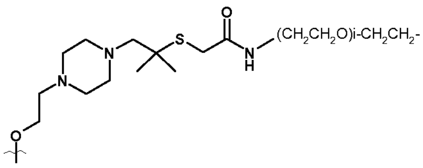

;

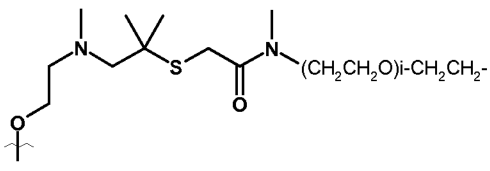

;

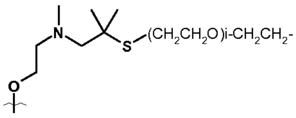

.

**7.** Compound according to Claim 1 or Claim 5, in which **L** is chosen from:

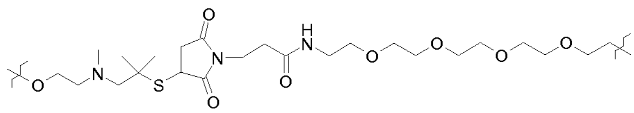

;

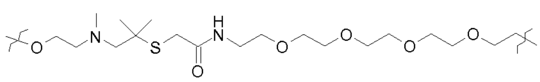

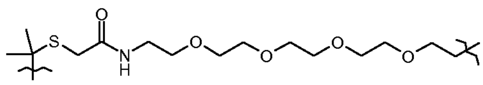

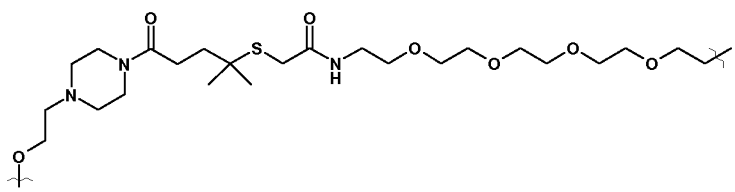

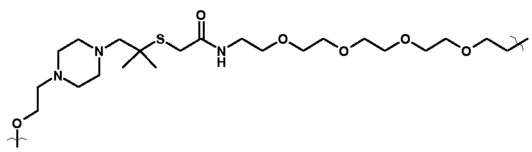

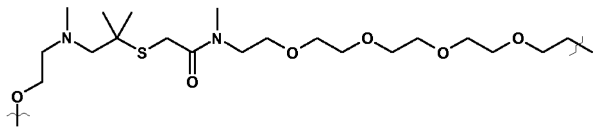

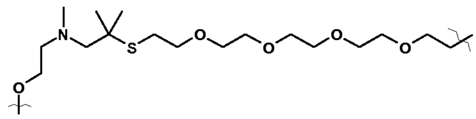

**8.** Compound according to Claim 1 to Claim 7, in which $-COZ_bR_b$ represents -COOH,- $COO(C_1-C_6)$alkyl, $-COOCH_3$, $-COOCH_2CH=CH_2$,

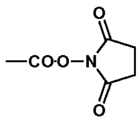

, 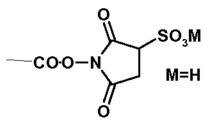 M=H or cation , 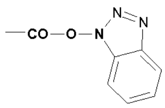 ,

the

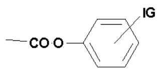

group, in which **IG** represents at least one inductive group, more particularly

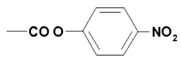 or 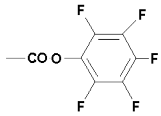 ,

or

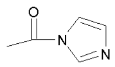 .

**9.** Compound according to Claim 1, chosen from one of the following:

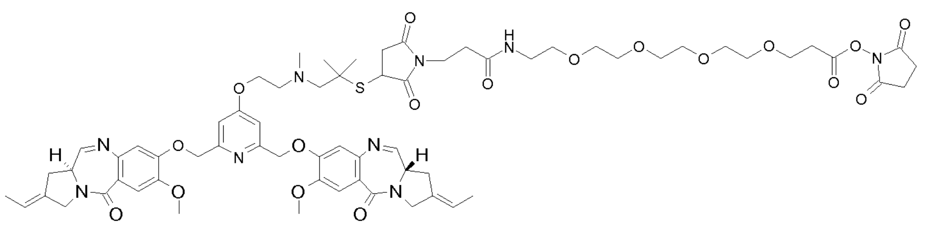

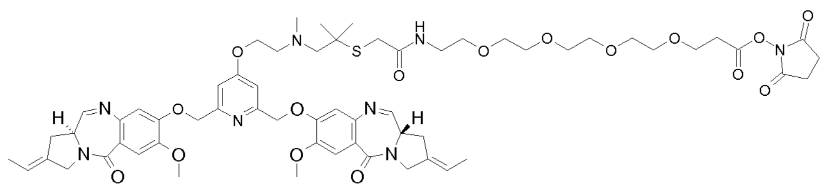

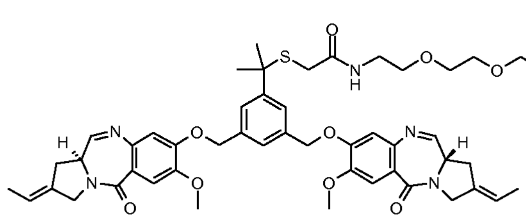

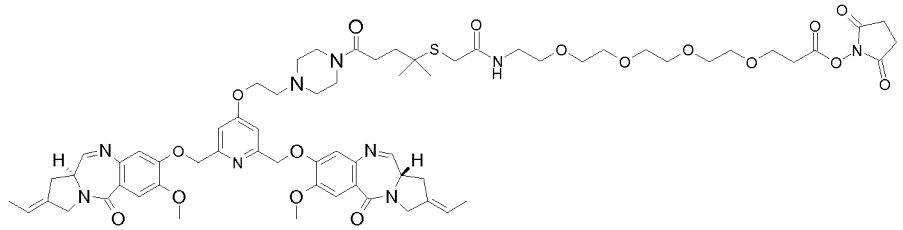

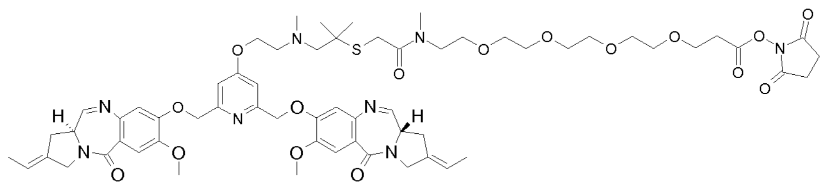

**10.** Process for the preparation of a conjugate, consisting in:

(i) bringing into contact and allowing to react an optionally buffered aqueous solution of a binding agent and a solution of a compound as defined in one of Claims 1 to 9;
(ii) and then optionally separating the conjugate formed in stage (i) from the compound of formula (I) and/or from the binding agent not having reacted and/or from the aggregates which might be formed.

**11.** Process according to Claim 10, in which the - $C(=O)Z_bR_b$ group is reactive with regard to the chemical groups present on the binding agent, in particular with regard to the amino groups present on an antibody, so as to ensure the attachment of the compound of formula (I) to the binding agent by formation of a covalent bond.

**12.** Process according to Claim 10 or Claim 11, in which stage (ii) consists in:

- separating the conjugate which has formed in stage (i) from the unreacted binding agent and from the aggregates possibly present in the solution;
or else
- separating the conjugate of stage (i) only from the unreacted compound of formula (I) and from the aggregates which might be formed and leaving in the solution the binding agent which might not have reacted.

**13.** Process according to one of Claims 10 to 12, in which the binding agent is a ligand, a protein, an antibody, more particularly a monoclonal antibody, a protein or antibody fragment, a peptide, an oligonucleotide or an oligosaccharide.

**14.** Process according to Claim 10 to Claim 13, in which the reaction takes place at a temperature of between 20 and

40°C and/or the duration of the reaction varies between 1 and 24 h.

**15.** Process according to one of Claims 10 to 14, in which, after stage (i) or (ii), the solution of the conjugate is subjected to a stage (iii) of ultrafiltration and/or of diafiltration.

**16.** Conjugate capable of being obtained by the process according to one of Claims 10 to 15.

**17.** Conjugate according to Claim 16, in which the binding agent is an antibody, preferably a monoclonal antibody, **characterized by** a mean DAR of between 1 and 10, preferably between 1.5 and 7, the DAR being calculated by the equation DAR = $c_D/c_A$
with:

$$c_D = [(e_{A\ LO1} \times A_{LO2}) - (e_{A\ LO2} \times A_{LO1})] / [(e_{D\ LO2} \times e_{A\ LO1}) - (e_{A\ LO2} \times e_{D\ LO1})]$$

$$c_A = [A_{LO1} - (c_D \times e_{D\ LO1})] / e_{A\ LO1}$$

$A_{LO1}$ and $A_{LO2}$ denoting the absorbances of an aqueous solution of the conjugate at the wavelengths LO1 and L02, measured on the corresponding peak of the SEC spectrum,
$e_{D\ LO1}$ and $e_{D\ LO2}$ respectively denoting the molar absorption coefficients of the pyrrolo[1,4]benzodiazepine dimer before conjugation at the two wavelengths LO1 and L02;
$e_{A\ LO1}$ and $e_{A\ LO2}$ respectively denoting the molar absorption coefficients of the naked antibody at the two wavelengths LO1 and L02,
LO1 = 280 nm and L02 = 320 nm.

**18.** Use of a compound according to one of Claims 1 to 9 in the preparation of a binding agent to which is covalently attached, in the position para to M, the dimer of formula:

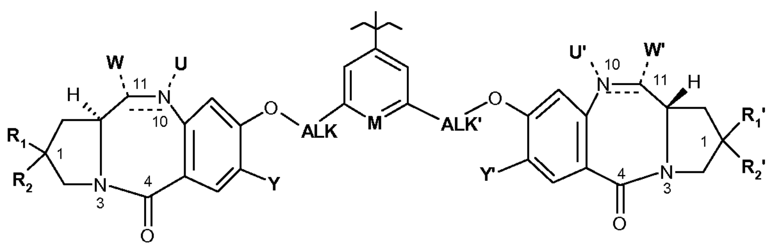

**19.** Use according to Claim 18, in which the binding agent is an antibody, preferably a monoclonal antibody.

**20.** Use according to Claim 18 or Claim 19, in which the dimer has the formula:

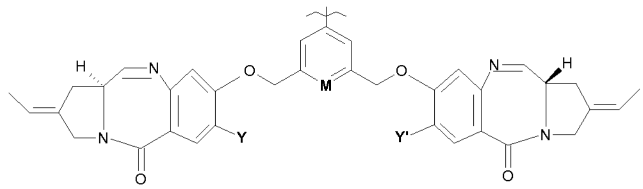

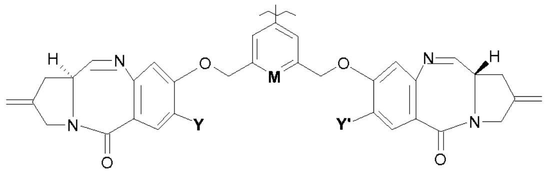

**21.** Use according to Claim 18 to Claim 20, in which **Y** and **Y'** represent a ($C_1$-$C_4$)alkoxy group, more particularly a methoxy group.

**22.** Binding agent to which was covalently attached, in the position para to M, the dimer of formula:

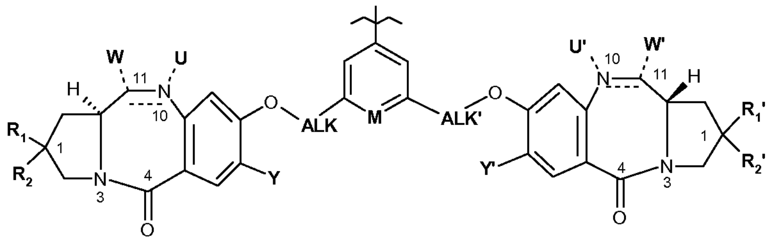

after reaction of a compound as defined in one of Claims 1 to 9 with the binding agent.

**23.** Binding agent according to Claim 22, in which the dimer is as defined in either of Claims 20 and 21.

**24.** Binding agent according to Claim 23, having affinity for an antigen or group of antigens located on cancer cells or stromal cells associated with a tumour.

**25.** Binding agent according to Claim 22 to Claim 24, which is an antibody, preferably a monoclonal antibody.

**26.** Solution of conjugate capable of being obtained by the process according to one of Claims 10 to 15.

**27.** Compound according to one of Claims 1 to 9 for use as anticancer agent.

**28.** Conjugate according to Claim 16 or Claim 17 for use as anticancer agent.

**29.** Pharmaceutical composition comprising a compound according to one of Claims 1 to 9 or else a conjugate according to Claim 16 or Claim 17 and at least one excipient.

**30.** Use of a compound of formula $\mathbf{P_2}$

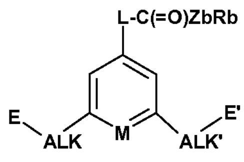

$\mathbf{P_2}$

or else a compound of formula:

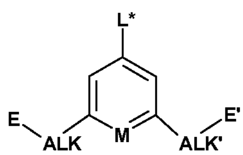

in which **L*** is chosen from:

-ALK-SH ;
-O-ALK-$NR_3$-ALK-SH ;

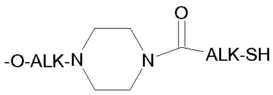 ; 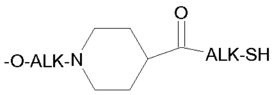 ; 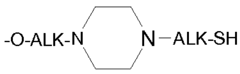

in which formulae:

- ■ L, M, ALK, ALK' , $R_3$, $Z_b$ and $R_b$ are as defined in one of Claims 1 to 9;
- ■ E and E' represent, independently of one another, an -OH group or a leaving group;

as intermediate in the preparation of a compound of formula (I) as defined in one of Claims 1 to 9.

**31.** Use according to Claim 30, in which the leaving group is chosen from a halogen atom or a mesylate, tosylate, nosylate or $-OPPh_3^+$ group.

**32.** Use of a compound chosen from one of the following:

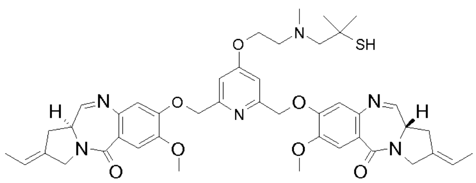
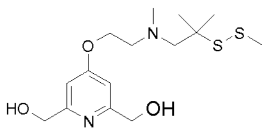
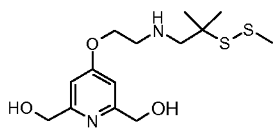

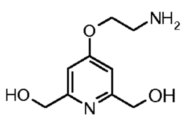
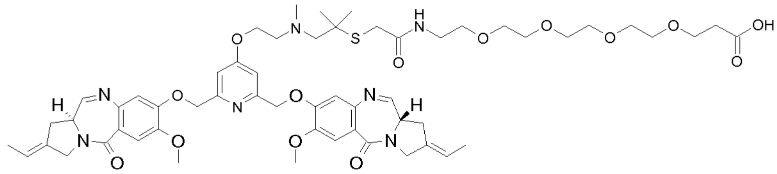

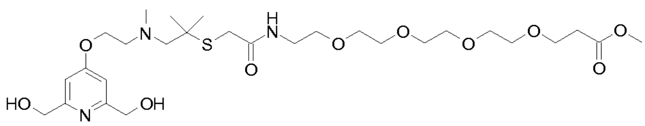
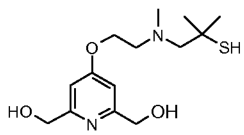

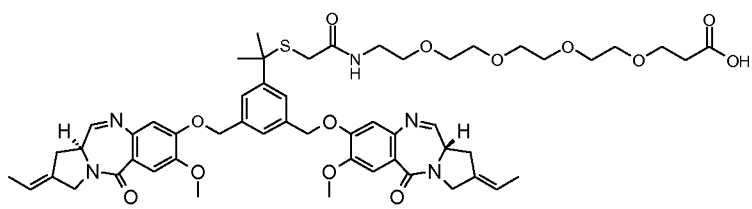

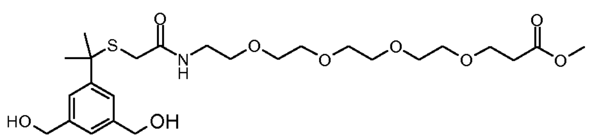

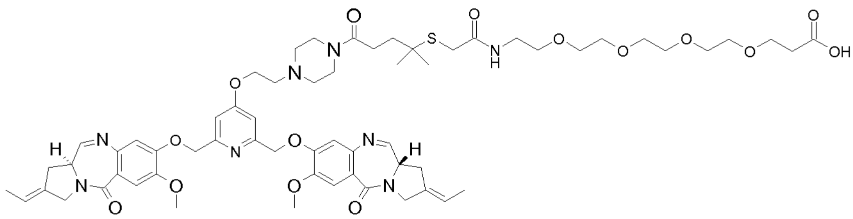

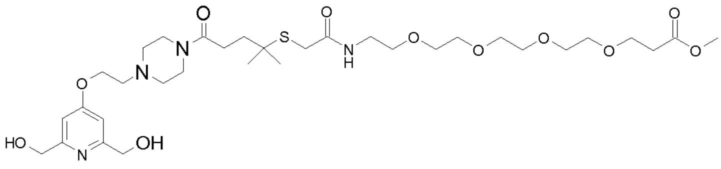

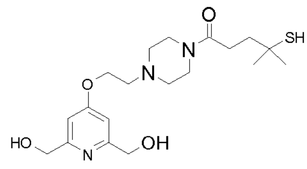

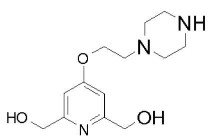

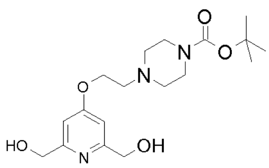

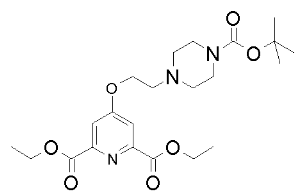

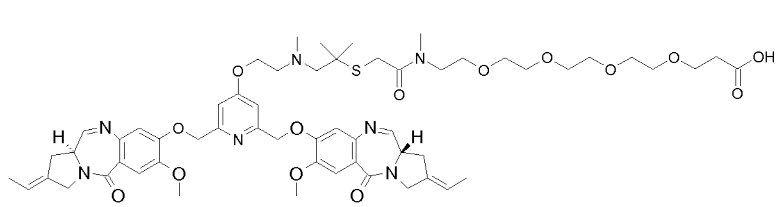

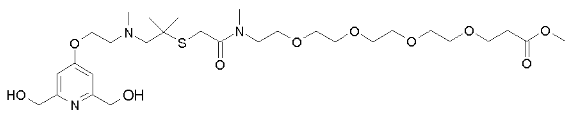

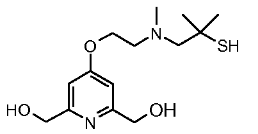

it being understood that the -COOH end functional group can be replaced by a -COO($C_1$-$C_6$)alkyl end functional group, in particular a -COOMe end functional group, and that the -SH end functional group can be replaced by a disulphide functional group, in particular an -SSMe functional group,
as intermediate in the preparation of a compound of formula (I) as defined in one of Claims 1 to 9.

**33.** Use of a compound according to one of Claims 1 to 9 in the manufacture of an anticancer agent.

**34.** Use of a conjugate according to Claim 16 or Claim 17 in the manufacture of an anticancer agent.

**Fig.1** : Spectre de masse haute résolution (HRMS) déconvolué du conjugué de l'ex.1 après déglycosylation

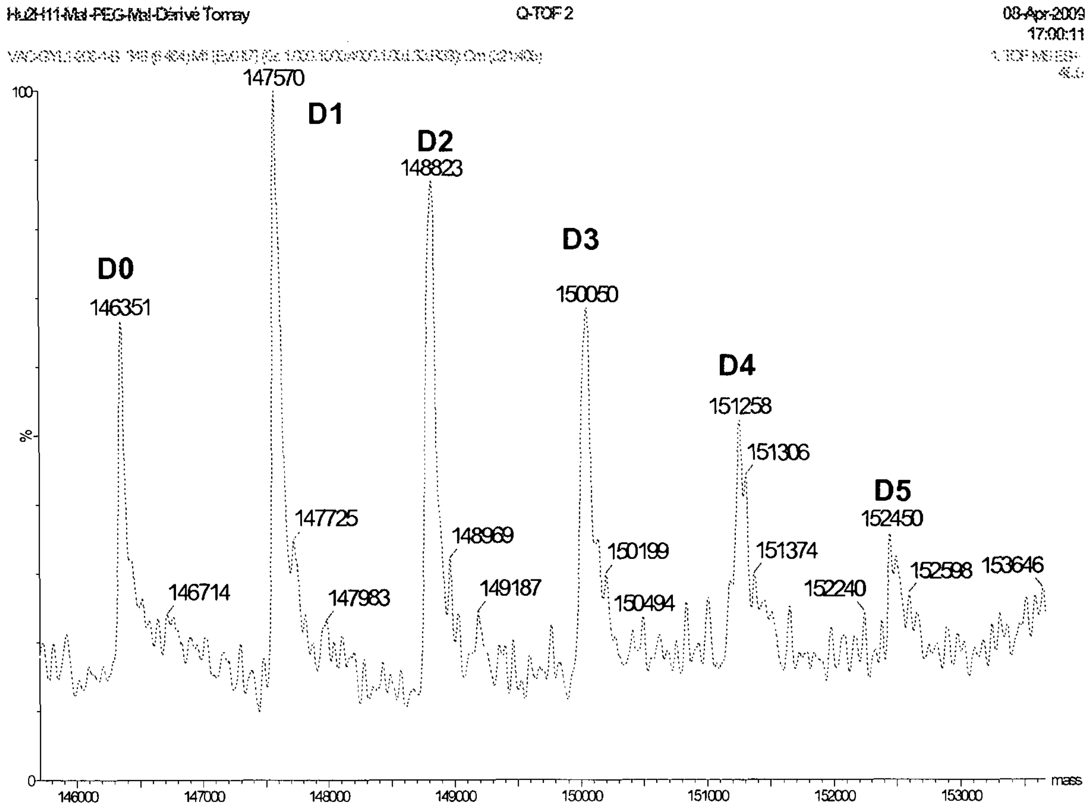

**Fig.2** : Spectre de masse haute résolution (HRMS) déconvolué du conjugué de l'ex.2 après déglycosylation

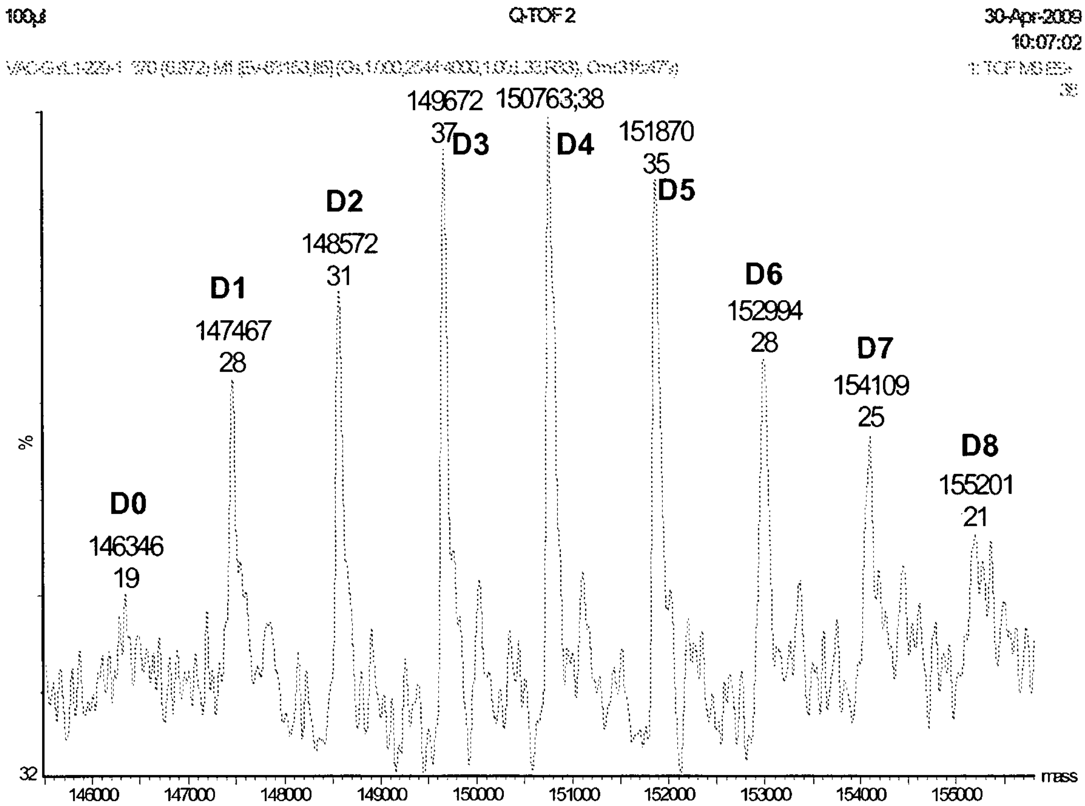

**Fig.3** : Spectre de masse haute résolution (HRMS) déconvolué du conjugué de l'ex.3 après déglycosylation

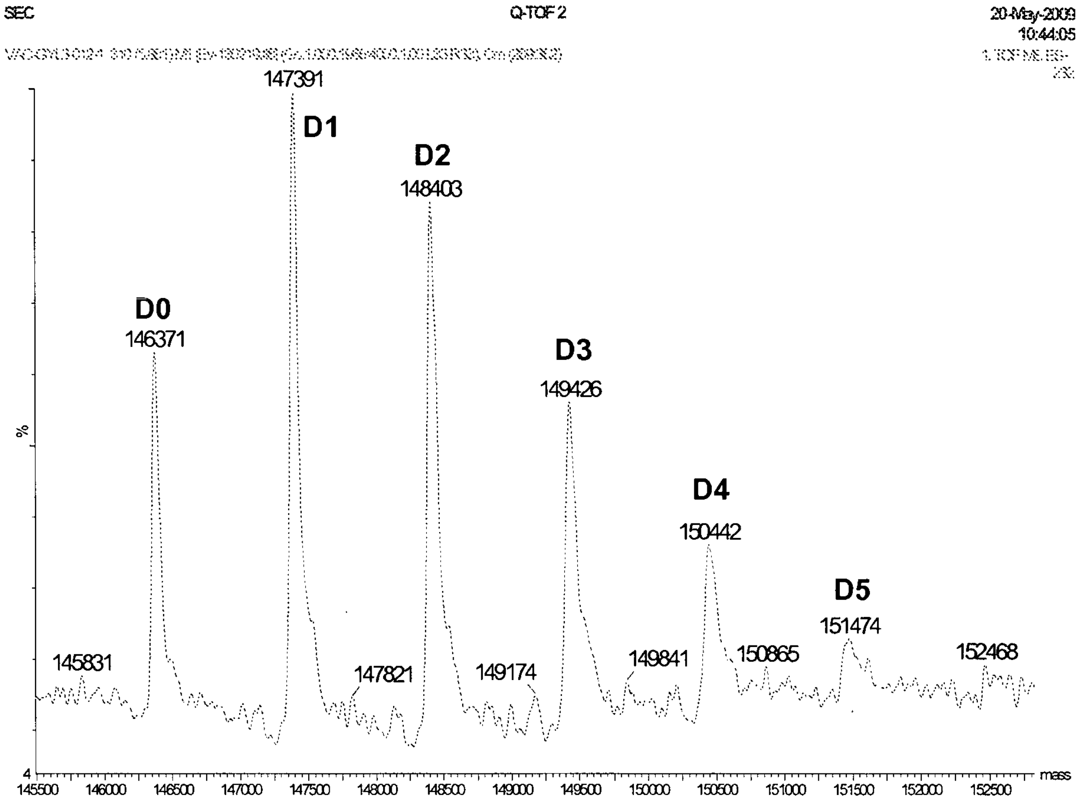


**Fig.4** : Spectre de masse haute résolution (HRMS) déconvolué du conjugué de l'ex.4 après déglycosylation

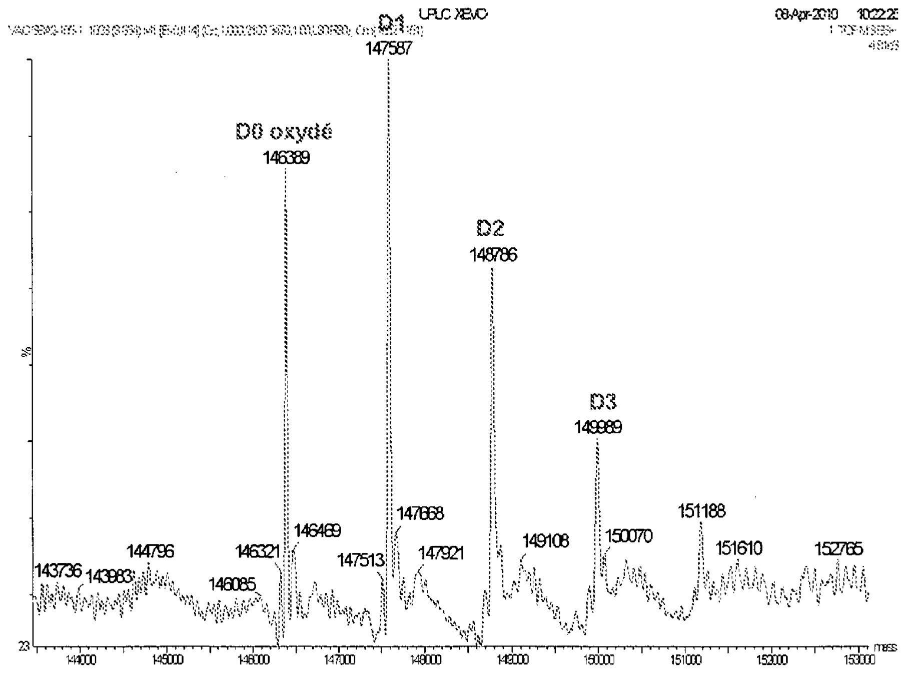

**Fig.5** : Spectre de masse haute résolution (HRMS) déconvolué du conjugué non déglycosylé de l'ex.6

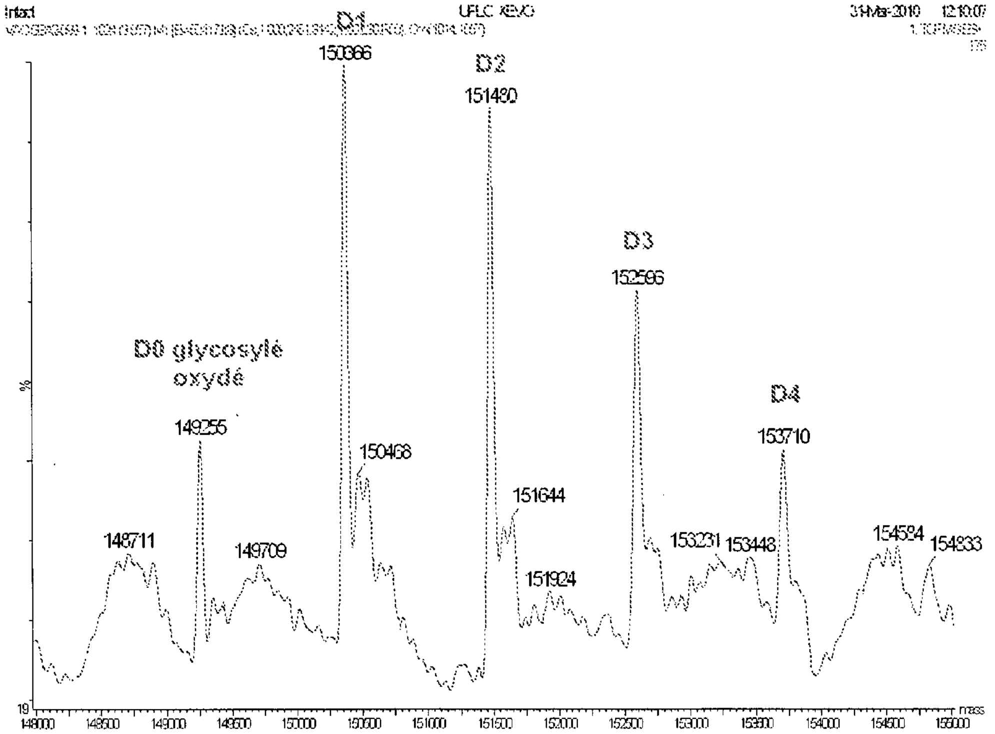

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0012508 A
- WO 2005085260 A
- WO 04103272 A
- WO 06061258 A
- WO 07144709 A
- WO 2007102069 A
- WO 07085930 A
- WO 2009016516 A
- WO 09026274 A
- WO 2006069246 A
- WO 0012507 A
- WO 2005040170 A
- FR 1516743
- WO 2007127440 A
- US 7230101 B
- WO 2007085930 A
- WO 03068144 A
- WO 04043344 A
- WO 08010101 A
- WO 08047242 A
- WO 05009369 A
- WO 2008010101 A
- WO 09016516 A


### Littérature non-brevet citée dans la description


- *Eur.J.Med.Chem.,* 2005, vol. 40, 641-654
- *Tetrahedron Letters,* 1988, vol. 29 (40), 5105-51108
- **MONNERET C. et al.** *Bulletin du Cancer,* 2000, vol. 87 (11), 829-38
- **RICART A.D et al.** *Nature Clinical Practice Oncology,* 2007, vol. 4, 245-255
- **SINGH R. ; RICKSON H.K.** *Therapeutic Antibodies : Methods and Protocols,* 2009, vol. 525, 445-467
- **GARNETT M.C. et al.** *Advanced Drug Delivery Reviews,* 2001, vol. 53, 171-216
- **JUNUTULA J.R. et al.** *Nature Biotechnology,* 2008, vol. 26, 925-932
- **DE GRAAF A.J. et al.** *Bioconjugate Chem,* 03 Février 2009
- **CHIN J.W. et al.** *JACS,* 2002, vol. 124, 9026-9027
- *JACS,* 1958, vol. 80, 4423
- *JACS,* 1960, vol. 82, 4596
- **MORI M. et al.** *Tetrahedron,* 1986, vol. 42, 3793-3806
- **Z.TOZUKA.** Studies on tomaymycin. Total syntheses of the antitumor antibiotics E- and Z- tomaymycins. *J. Antibiotics,* 1983, vol. XXXVI (3), 276-282
- Liebigs Annalen der Chemie. 1991, vol. 10, 987-988
- *Tetrahedron,* 2005, vol. 61 (7), 1755-1763
- **WESSJOHANN, L. et al.** *Synthesis,* 1989, vol. 5, 359-63
- **KITAGAWA T. et al.** *JACS,* 2006, vol. 128 (45), 14448-14449
- **GREG T. HERMANSON.** Bioconjugate Techniques. Elsevier Inc, 721
- **RICHARD A. et al.** *Chem. Eur. J.,* 2005, vol. 11, 7315-7321
- **SAKELLARIOU E.G. et al.** *Tetrahedron,* 2003, vol. 59, 9083-9090
- *J.Membrane Sci.,* 2008, vol. 318, 311-316
- Protein Aggregation and Bioprocessing. *AAPS Journal,* 2006, vol. 8 (3), E572-E579
- *Analytical Biochemistry,* 1993, vol. 212 (2), 469-480
- Therapeutic Antibodies and Protocols. Springer Science, 2009, vol. 525
- **CARTER P.J. et al.** Antibody-drug conjugates for cancer therapy. *Cancer J.,* 2008, vol. 14, 154-169
- **CHARI R.** Targeted cancer therapy: conferring specificity to cytotoxic drugs. *Acc. Chem. Res.,* 2008, vol. 41, 98-107
- **G.T.CRISP ; P.D.TURNER.** *Tetrahedron,* 2000, vol. 56 (42), 8335
- **SCRIMIN, P. ; TECILLA, P.; ; TONELLATO, U ; VENDRAME, T.** *J. Org. Chem.,* 1989, vol. 54, 5988
- Human tumor xenograft models. **PLOWMAN J ; DYKES DJ ; HOLLINGSHEAD M ; SIMPSON-HERREN L ; ALLEY MC.** NCI drug development : Feibig HH BA,. 1999, 101-125